(19) Europäisches Patentamt
European Patent Office
Office européen des brevets

(11) EP 4 424 675 A1

(12) **EUROPEAN PATENT APPLICATION**
published in accordance with Art. 153(4) EPC

(43) Date of publication:
**04.09.2024 Bulletin 2024/36**

(21) Application number: **22887724.7**

(22) Date of filing: **28.10.2022**

(51) International Patent Classification (IPC):
***C07D 239/74*** (2006.01) ***A61K 31/517*** (2006.01)
***A61P 35/00*** (2006.01) ***A61P 3/04*** (2006.01)
***A61P 25/00*** (2006.01) ***A61P 9/10*** (2006.01)
***A61P 9/12*** (2006.01) ***C07D 401/12*** (2006.01)
***C07D 487/10*** (2006.01) ***C07D 403/12*** (2006.01)

(52) Cooperative Patent Classification (CPC):
**A61K 31/517; A61P 3/04; A61P 9/10; A61P 9/12; A61P 25/00; A61P 35/00; C07D 239/74; C07D 401/12; C07D 403/12; C07D 487/10**

(86) International application number:
**PCT/KR2022/016766**

(87) International publication number:
**WO 2023/075529 (04.05.2023 Gazette 2023/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.10.2021 KR 20210146983**

(71) Applicant: **Voronoi Inc.**
**Incheon 21984 (KR)**

(72) Inventors:
- **JUNG, Myungho**
  **Incheon 21984 (KR)**
- **JEON, Hyeonho**
  **Incheon 21984 (KR)**
- **SON, Jungbeom**
  **Incheon 21984 (KR)**
- **KIM, Namdoo**
  **Incheon 21984 (KR)**
- **KIM, Sunghwan**
  **Incheon 21984 (KR)**

(74) Representative: **Viering, Jentschura & Partner mbB**
**Patent- und Rechtsanwälte**
**Am Brauhaus 8**
**01099 Dresden (DE)**

(54) **QUINAZOLINE DERIVATIVE COMPOUND AND USE THEREOF**

(57) The present disclosure relates to quinazoline derivative compounds and medicinal uses thereof. Specifically, the present disclosure provides compounds capable of treating or preventing c-KIT or PDGFR-related diseases by inhibiting c-KIT or PDGFR.

EP 4 424 675 A1

## Description

[Technical field]

The present disclosure relates to quinazoline derivative compounds and medicinal uses thereof. Specifically, the present disclosure relates to compounds capable of treating or preventing c-KIT or PDGFR-related diseases by inhibiting c-KIT or PDGFR.

[Background Art]

c-KIT is a protein encoding the human KIT gene. The ligand of the c-KIT receptor is a stem cell factor (SCF) and when SCF acts on the c-KIT ligand binding domain, the SCF/c-KIT interaction occurs. As a result, the c-KIT protein forms a dimer and becomes active by autophosphorylation. The activated c-KIT then regulates survival such as growth, differentiation, proliferation, and natural death of cells through various signal transduction processes such as the intracellular PI3K/Akt system, Ras/MAP kinase, and JAK/STAT. Various cancers associated with c-KIT are related to activation of c-KIT. When c-KIT can be activated, it can be caused by overexpression or mutation in addition to expression by interaction with ligand. Overexpression of c-KIT is achieved according to the autocrine mechanism, and breast cancer, rectal cancer, small cell lung cancer, and neuroblastoma may occur according to this loop. In addition, when gain-of-function mutations of c-KIT occur, c-KIT becomes resistant to natural death due to excessive activation such as continuous activation of c-KIT regardless of the SCF ligand and signal transmission system below it. Many mutations encoding the c-KIT gene are found in various human cancers, including gastrointestinal stromal tumors (GIST) caused by mutations in the juxtamembrane region of c-KIT, as well as neuroblastoma, acute myelogenous leukemia (AML), and systemic mastocytosis (SM) caused by c-KIT mutations. In particular, the c-KIT D816V mutation (c-KIT D816V), in which the aspartate residue at residue 816 of the c-KIT activation loop is substituted with valine, appears predominantly in mast cell-related diseases, especially it occurs in more than 90% of cases that meet the World Health Organization's diagnostic criteria for systemic mastocytosis. Currently, there are imatinib (Gleevec) and sunitinib (Sutent) that have been approved by the FDA for their c-KIT inhibitory effect and are used for the treatment of GIST patients. It is known to be effective against c-KIT's wild type (c-KIT WT) and c-KIT V560G mutation, but it was confirmed that there is no effect of inhibiting c-KIT D816V.

Meanwhile, platelet-derived growth factor receptors (PDGFR) are tyrosine kinase receptors belonging to the PDGFR family. PDGFR is composed of PDGF-$\alpha$ and PDGF-$\beta$ subunits, and forms homo- or hetero-dimers according to the binding of growth factors such as PDGF to play an important role in cell proliferation, cell differentiation, and cell growth and development through intracellular signal transduction. Signaling dysfunction due to PDGFR overactivity is found in a variety of pathological conditions, such as fibrosis, cancer, neurological disease, and atherosclerosis (literature [Papadopoulos, Natalia, and Johan Lennartsson. "The PDGF/PDGFR pathway as a drug target." Molecular aspects of medicine 62 (2018): 75-88.]). Systemic sclerosis, one of the fibrosis diseases, is a disease that causes multi-organ disorders and is characterized by fibrosis of the skin and blood vessels and fibrosis of internal organs such as the gastrointestinal tract, lungs, heart, and kidneys. Pulmonary fibrosis, one of the main symptoms of systemic sclerosis, is speculated to be caused by induction of excessive extracellular matrix (ECM) protein deposition in the intercellular space due to uncontrolled proliferation of fibroblast present in the lungs and differentiation into myofibroblast.

As such, unmet demand for novel compounds that can be usefully utilized for the treatment of c-KIT or PDGFR related diseases by regulating c-KIT or PDGFR activity is increasing.

[Disclosure]

[Technical Problem]

An object of the present disclosure is to provide a quinazoline derivative compound, a stereoisomer thereof, or a pharmaceutically acceptable salt thereof.

Another object of the present disclosure is to provide a method for preparing the compound.

Still another object of the present disclosure is to provide a pharmaceutical use of the compound, specifically, a pharmaceutical composition for treating or preventing c-KIT or PDGFR-related diseases comprising the compound as an active ingredient, or to provide a method for treating or preventing a c-KIT or PDGFR-related disease, including a method of administering the compound.

[Technical Solution]

In order to achieve the above-described objects, the present inventors made efforts to study, and as a result,

found that the following quinazoline derivative compounds represented by Formula 1 inhibited c-KIT or PDGFR activity, and completed the present disclosure.

Quinazoline derivative compounds

One embodiment of the present disclosure is a compound represented by Chemical Formula 1 below, a stereoisomer thereof, or a pharmaceutically acceptable salt thereof.

[Chemical Formula 1]

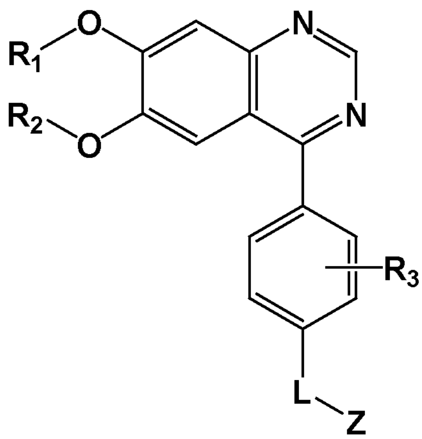

in the Chemical Formula 1,

$R_1$ and $R_2$ are each independently -H, -(C1-C6)alkyl, $-(CH_2)_m-R_X$, or $-(C{=}O)-R_X$ {wherein, $R_1$ and $R_2$ cannot be methyl at the same time};
$R_x$ is -(C1-C6)alkoxy, $-NR^AR^B$, -halo, cycloalkyl, heterocycloalkyl, aryl, heteroaryl, or heterohydroaryl {wherein, at least one H in the cycloalkyl or heterocycloalkyl ring may be substituted with -(C1-C6)alkyl, - (C1-C6) hydroxyalkyl, -(C1-C6)alkyl-O-(C1-C6)alkyl, -(C=O)-(C1-C6)alkyl, -(C=O)-O-(C1-C6)alkyl, $-NR^CR^D$, -(=O)-, cycloalkyl, or benzyl [wherein, at least one H in the benzyl ring may be substituted with -(C1-C6)alkyl, -(C1-C6)alkoxy, -(C1-C6)haloalkyl, or halo]; at least one H in the aryl, heteroaryl, or heterohydroaryl ring may be substituted with -(C1-C6)alkyl, -(C1-C6)haloalkyl, or halo};
$R^A$ and $R^B$ are each independently -H, -(C1-C6)alkyl, cycloalkyl, heterocycloalkyl, aryl, or heteroaryl {wherein, at least one H in the cycloalkyl, heterocycloalkyl, aryl, or heteroaryl ring may be substituted with -(C1-C6)alkyl};
$R^C$ and $R^D$ are each independently -H or -(C1-C6)alkyl;
m is 0, 1, 2, 3, or 4;
$R_3$ is -H, -(C1-C6)alkyl, -(C1-C6)alkoxy, or -halo;
L is $-NH(C{=}O)-L_1-(CH_2)_n-L_2-$, $-NH-S({=}O)_2-L_1-(CH_2)_n-L_2-$, - $(C{=}O)NH-L_1-(CH_2)_n-L_2-$, $-L_1-(CH_2)_n-L_2-(C{=}O)NH-$, or -NH(C=O)NH-;
$L_1$ and $L_2$ are each independently $-CR^ER^F-$, -C(=O)NH-, or null;
$R^E$ and $R^F$ are each independently -H, -(C1-C6)alkyl, $-NH_2$, - NH(C=O)O-(C1-C6)alkyl, -OH, or -halo, or $R^E$ and $R^F$ are combined with carbon to form -(C3-C6)cycloalkyl;
n is 0, 1, 2, 3, or 4;
Z is aryl, heteroaryl, heterohydroaryl, cycloalkyl, heterocycloalkyl, or -(C2-C6)alkenyl {wherein, at least one H in the aryl, heteroaryl, or heterohydroaryl ring may be substituted with -(C1-C6)alkyl, -(C1-C6)haloalkyl, -(C1-C6)hydroxyalkyl, - (C1-C6)cyanoalkyl, -CN, $-NR^GR^H$, -(C1-C6)alkoxy, or -halo; at least one H in the cycloalkyl or heterocycloalkyl ring may be substituted with -(C1-C6)alkyl};
$R^G$ and $R^H$ are each independently H or -(C1-C6)alkyl.

According to an embodiment of the present disclosure, the compound represented by Chemical Formula 1 above, the stereoisomer thereof, or the pharmaceutically acceptable salt thereof may be included in the following scope:

$R_1$ and $R_2$ are each independently -H, -(C1-C6)alkyl, $-(CH_2)_m-R_x$, or $-(C{=}O)-R_X$ {wherein, $R_1$ and $R_2$ cannot be methyl at the same time};

$R_X$ is -(C1-C6)alkoxy, $-NR^AR^B$, -halo, heterocycloalkyl, aryl, heteroaryl, or heterohydroaryl {wherein, at least one H in the heterocycloalkyl ring may be substituted with -(C1-C6)alkyl, - (C1-C6)hydroxyalkyl, -(C1-C6)alkyl-O-(C1-C6)alkyl, -(C=O)-(C1-C6)alkyl, -(C=O)-O-(C1-C6)alkyl, $-NR^CR^D$, -(=O)-, cycloalkyl, or benzyl [wherein, at least one H in the benzyl ring may be substituted with -(C1-C6)alkyl, -(C1-C6)alkoxy, -(C1-C6)haloalkyl, or halo]; at least one H in the aryl, heteroaryl, or heterohydroaryl ring may be substituted with -(C1-C6)haloalkyl};

$R^A$ and $R^B$ are each independently -H, -(C1-C6)alkyl, cycloalkyl, or heteroaryl {wherein, at least one H in the cycloalkyl or heteroaryl ring may be substituted with -(C1-C6)alkyl};

$R^C$ and $R^D$ are each independently -H or -(C1-C6)alkyl;

m is 0, 1, 2, 3, or 4;

$R_3$ is -H, -(C1-C6)alkyl, or -halo;

L is $-NH(C=O)-L_1-(CH_2)_n-L_2-$, $-NH-S(=O)_2-L_1-(CH_2)_n-L_2-$, $-(C=O)NH-L_1-(CH_2)_n-L_2-$, or -NH(C=O)NH-;

$L_1$ and $L_2$ are each independently $-CR^ER^F-$ or null;

$R^E$ and $R^F$ are each independently -H, -(C1-C6)alkyl, or $-NH_2$, or $R^E$ and $R^F$ are combined with carbon to form -(C3-C6)cycloalkyl;

n is 0, 1, 2, 3, or 4;

Z is phenyl, 5-10 membered heteroaryl, 5-10 membered heterohydroaryl, 5-10 membered heterocycloalkyl, or -(C2-C6)alkenyl {wherein, at least one H in the phenyl, 5-10 membered heteroaryl, or 5-10 membered heterohydroaryl ring may be substituted with -(C1-C6)alkyl, -(C1-C6)haloalkyl, -CN, -(C1-C6)alkoxy, or -halo; at least one H in the 5-10 membered heterocycloalkyl ring may be substituted with -(C1-C6)alkyl}.

According to an embodiment of the present disclosure, the compound represented by Chemical Formula 1 above, the stereoisomer thereof, or the pharmaceutically acceptable salt thereof may be included in the following scope:

$R_1$ and $R_2$ are each independently -H, -(C1-C6)alkyl, $-(CH_2)_m-R_X$, or $-(C=O)-R_X$ {wherein, $R_1$ and $R_2$ cannot be methyl at the same time};

$R_x$ is -(C1-C6)alkoxy, $-NR^AR^B$, -halo, heterocycloalkyl, aryl, heteroaryl, or heterohydroaryl {wherein, the heterocycloalkyl is a monocyclic ring, a spiro ring, a bridged ring, or a fused ring, at least one H in the heterocycloalkyl ring may be substituted with -(C1-C6)alkyl, -(C1-C6)hydroxyalkyl, -(C1-C6)alkyl-O-(C1-C6)alkyl, -(C=O)-(C1-C6)alkyl, -(C=O)-O-(C1-C6)alkyl, $-NR^CR^D$, -(=O)-, cycloalkyl, or benzyl [wherein, at least one H in the benzyl ring may be substituted with -(C1-C6)alkyl, -(C1-C6)alkoxy, -(C1-C6)haloalkyl, or halo]; at least one H in the aryl, heteroaryl, or heterohydroaryl ring may be substituted with -(C1-C6)haloalkyl};

$R^A$ and $R^B$ are each independently -H, -(C1-C6)alkyl, cycloalkyl, or heteroaryl {wherein, at least one H in the cycloalkyl or heteroaryl ring may be substituted with -(C1-C6)alkyl};

$R^C$ and $R^D$ are each independently -H or -(C1-C6)alkyl;

m is 0, 1, 2, or 3.

According to an embodiment of the present disclosure, the compound represented by Chemical Formula 1 above, the stereoisomer thereof, or the pharmaceutically acceptable salt thereof may be included in the following scope: The compound represented by Chemical Formula 1 is represented by Chemical Formula 2 below;

[Chemical Formula 2]

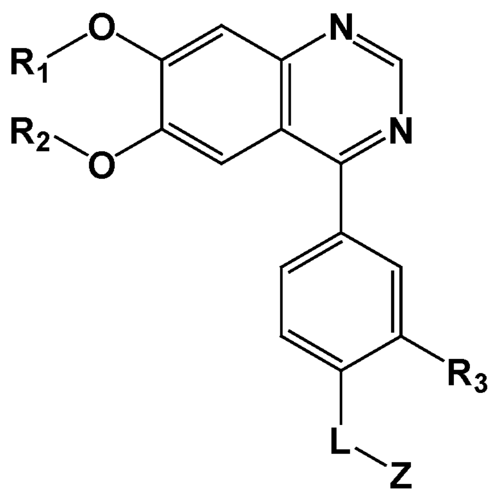

in the Chemical Formula 2,
$R_3$ is -H, -(C1-C6)alkyl, or -halo.

According to an embodiment of the present disclosure, the compound represented by Chemical Formula 1 above, the stereoisomer thereof, or the pharmaceutically acceptable salt thereof may be included in the following scope:

L is -NH (C=O) $-L_1-(CH_2)_n-L_2-$, $-NH-S(=O)_2-L_1-(CH_2)_n-L_2-$, - (C=O)NH-$L_1-(CH_2)_n-L_2-$, or -NH(C=O)NH-;
$L_1$ and $L_2$ are each independently $-CR^ER^F-$ or null;
$R^E$ and $R^F$ are each independently -H, -(C1-C6)alkyl, or $-NH_2$, or $R^E$ and $R^F$ are combined with carbon to form -(C3-C6)cycloalkyl;
n is 0, 1, 2, or 3.

According to an embodiment of the present disclosure, the compound represented by Chemical Formula 1 above, the stereoisomer thereof, or the pharmaceutically acceptable salt thereof may be included in the following scope: Z is phenyl, 5-10 membered heteroaryl, 5-10 membered heterohydroaryl, 5-10 membered heterocycloalkyl, or -(C2-C6)alkenyl {wherein, at least one H in the phenyl, 5-10 membered heteroaryl, or 5-10 membered heterohydroaryl ring may be substituted with -(C1-C6)alkyl, -(C1-C6)haloalkyl, -CN, -(C1-C6)alkoxy, or -halo; at least one H in the 5-10 membered heterocycloalkyl ring may be substituted with -(C1-C6)alkyl}.

In addition, according to a specific embodiment of the present disclosure, the compound represented by Chemical Formula 1 may be selected from the group consisting of the compounds of Examples 1 to 169 listed in Tables 1 to 5 described below.

An embodiment of the present disclosure is a pharmaceutical composition comprising the compound represented by Chemical Formula 1, the stereoisomer thereof, or the pharmaceutically acceptable salt thereof as an active ingredient.

In the present disclosure, unless otherwise specified, "alkyl" may refer to acyclic which is a straight or branched chain, cyclic, or saturated hydrocarbon of which they are bonded. For example, "$C_{1-6}$alkyl" may indicate an alkyl containing 1 to 6 carbon atoms. The acyclic alkyl may include, for example, methyl, ethyl, n-propyl, n-butyl, isopropyl, sec-butyl, isobutyl, *tert*-butyl, and the like, but is not limited to. The cyclic alkyl may be used interchangeably with "cycloalkyl" herein, and as an example, may include cyclopropyl, cyclobutyl, cyclopentyl, cyclohexyl, cycloheptyl, cyclooctyl, and the like, but is not limited to.

In the present disclosure, "alkoxy" may indicate -(O-alkyl) as an alkyl ether group, wherein alkyl is same as defined above. For example, "$C_{1-6}$alkoxy" may mean alkoxy containing $C_{1-6}$alkyl, that is, -(O-$C_{1-6}$alkyl), and as an example, alkoxy may include methoxy, ethoxy, n-propoxy, isopropoxy, n-butoxy, isobutoxy, *sec*-butoxy, tert-butoxy, and the like, but is not limited thereto.

In the present disclosure, "halo" may be F, Cl, Br, or I.

In the present disclosure, "haloalkyl" may mean a straight-chain or branched-chain alkyl(hydrocarbon) having one or more halo-substituted carbon atoms as defined herein. Examples of the haloalkyl may include methyl, ethyl, propyl, isopropyl, isobutyl, or n-butyl independently substituted with one or more halogens, such as F, Cl, Br, or I, but are not limited thereto.

In the present disclosure, "hydroxyalkyl" may mean straight-chain or branched-chain alkyl(hydrocarbon) having a carbon atom substituted with hydroxy (OH).

In the present disclosure, "aminoalkyl" may mean straight-chain or branched-chain alkyl(hydrocarbon) having a cabon atom substituted with amino (NR'R"). Here, R' and R" may each independently be selected from the group consisting of hydrogen and $C_{1-6}$alkyl, and the selected R' and R" may each independently be substituted or unsubstituted.

In the present disclosure, "cyanoalkyl" may mean straight-chain or branched-chain alkyl(hydrocarbon) having a carbon atom substituted with cyano (CN).

In the present disclosure, "heterocycloalkyl" may mean a ring containing at least one selected from N, O, P, P(=O), and S in the ring, and may be saturated or partially unsaturated. Here, when unsaturated, it may be referred to as a heterocycloalkene. Unless otherwise stated, a heterocycloalkyl can be a monocyclic ring, or a multiple ring, such as a spiro ring, a bridged ring, or a fused ring. In addition, "3- to 12-membered heterocycloalkyl" may mean a heterocycloalkyl containing 3 to 12 atoms forming a ring, and as an example, heterocycloalkyl may include pyrrolidine, piperidine, imidazolidine, pyrazolidine, butyrolactam, valerolactam, imidazolidinone, hydantoin, dioxolane, phthalimide, piperidine, pyrimidine-2,4(1H,3H)-dione, 1,4-dioxane, morpholine, thiomorpholine, thiomorpholine-S-oxide, thiomorpholine-S,S-oxide, piperazine, pyran, pyridone, 3-pyrroline, thiopyran, pyron, tetrahydrofuran, tetrahydrothiophene, quinuclidine, tropane, 2-azaspiro[3.3]heptane, (1*R*,5*S*)-3-azabicyclo[3.2.1]octane, (1*s*,4*s*)-2-azabicyclo[2.2.2]octane, (1*R*,4*R*)-2-oxa-5-azabicyclo[2.2.2]octane, and the like, but is not limited thereto.

In the present disclosure, "arene" may mean an aromatic hydrocarbon ring. The arene may be a monocyclic arene or a polycyclic arene. The number of ring-forming carbon atoms of the arene may be 5 or more and 30 or less, 5 or more and 20 or less, or 5 or more and 15 or less. Examples of the arene may include benzene, naphthalene, fluorene,

anthracene, phenanthrene, bibenzene, terbenzene, quarterbenzene, quinquebenzene, sexibenzene, triphenylene, pyrene, benzofluoranthene, chrysene, and the like, but are not limited thereto. In the present specification, a residue obtained by removing one hydrogen atom from the above "arene" is referred to as "aryl".

In the present disclosure, the "heteroarene" may be a ring including one or more of O, N, P, Si, and S as a heterogeneous element. The number of ring-forming carbon atoms of heteroarene may be 2 or more and 30 or less, or 2 or more and 20 or less. The heteroarene may be a monocyclic heteroarene or a polycyclic heteroarene. The polycyclic heteroarenes may have, for example, a bicyclic or tricyclic structure. Examples of the heteroaren may include thiophene, purine, pyrrole, pyrazole, imidazole, thiazole, oxazole, isothiazole, oxadiazole, triazole, pyridine, bipyridyl, triazine, acridyl, pyridazine , pyrazine, quinoline, quinazoline, quinoxaline, phenoxazine, phthalazine, pyrimidine, pyrido pyrimidine, pyrido pyrazine, pyrazino pyrazine, isoquinoline, indole, carbazole, imidazopyridazine, imidazopyridine, imidazopyrimidine, pyrazolopyrimidine, imidazopyrazine, pyrazolopyridine, *N*-arylcarbazole, *N*-heteroarylcarbazole, *N*-alkylcarbazole, benzoxazole, benzoimidazole, benzothiazole, benzocarbazole, benzothiophene, dibenzothiophene, thienothiophene, benzofuran, phenanthroline, isoxazole, oxadiazole, thiadiazole, benzothiazole, tetrazole, phenothiazine, dibenzosilol, dibenzofuran, and the like, but are not limited thereto. In one embodiment of the present disclosure, the heteroarene may also include a bicyclic heterocyclo-arene containing an arene ring fused to a heterocycloalkyl ring or a heteroarene fused to a cycloalkyl ring. In the present specification, a residue obtained by removing one hydrogen atom from the "heteroarene" is referred to as "heteroaryl".

In the present disclosure, "hydroarene" or "hydroaryl" means that one or more double bonds are saturated in an aromatic hydrocarbon ring.

In the present disclosure, "heterohydroarene" or "heterohydroaryl" is means that one or more double bonds are saturated in a "heteroarene" or "heteroaryl" ring. In the present disclosure, the "ring" may be a monocyclic ring or a polycyclic ring. The polycyclic ring may be a spiro ring, a bridged ring, or a fused ring.

In the present disclosure, the term "stereoisomer" means a compound of the present disclosure or a salt thereof having the same chemical formula or molecular formula but sterically different. Each of these stereoisomers and mixtures thereof is also included within the scope of the present disclosure. Unless otherwise stated, a solid-line bond (-) connected to an asymmetric carbon atom may include a wedge-shaped solid-line bond 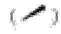 or a wedge-shaped dotted-line bond 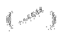 representing an absolute arrangement of the stereocentric.

The compound of Chemcial Formula 1 of the present disclosure may exist in the form of a "pharmaceutically acceptable salt". As the salt, an acid addition salt formed by a pharmaceutically acceptable free acid is useful. The term "pharmaceutically acceptable salt" refers to any organic acid or inorganic acid addition salt of the compound whose side effects do not reduce the beneficial efficacy of the compound represented by Chemical Formula 1 at concentrations having an effective action that is relatively non-toxic and harmless to a patient.

The acid addition salt is prepared by conventional methods, for example, by dissolving the compound in an excess aqueous acid solution and precipitating the salt using a water-miscible organic solvent, such as methanol, ethanol, acetone, or acetonitrile. Equimolar amounts of the compound and acid or alcohol in water may be heated, and then the mixture may be evaporated to dryness, or the precipitated salt may be filtered off with suction.

Here, and organic acid and inorganic acid can be used as the free acid, wherein the inorganic acid may be hydrochloric acid, phosphoric acid, sulfuric acid, nitric acid, or the like, and the organic acid may be methanesulfonic acid, p-toluenesulfonic acid, acetic acid, trifluoroacetic acid, maleic acid, succinic acid, oxalic acid, benzoic acid, tartaric acid, fumaric acid, manderic acid, propionic acid, citric acid, lactic acid, glycollic acid, gluconic acid, galacturonic acid, glutamic acid, glutaric acid, glucuronic acid, aspartic acid, ascorbic acid, carbonic acid, vanillic acid, hydroiodic acid, or the like. However, it is not limited thereto.

In addition, a pharmaceutically acceptable metal salt may be prepared using a base. The alkali metal salt or the alkaline earth metal salt is obtained, for example, by dissolving the compound in an excess alkali metal hydroxide or alkaline earth metal hydroxide solution, filtering the undissolved compound salt, and then evaporating and drying the filtrate. Here, as the metal salt, it is particularly suitable for preparing a sodium, potassium, or calcium salt, but is not limited thereto. In addition, the corresponding silver salt may be obtained by reacting an alkali metal or an alkaline earth metal salt with a suitable silver salt (e.g., silver nitrate).

Unless otherwise indicated, the pharmaceutically acceptable salt of the present disclosure includes salts of acidic or basic groups that may be present in the compound of Chemical Formula 1. For example, the pharmaceutically acceptable salt may include sodium, calcium and potassium salts of a hydroxyl group, and other pharmaceutically acceptable salts of an amino group may include hydrobromides, sulfates, hydrogen sulfates, phosphates, hydrogen phosphate, dihydrogen phosphate, acetate, succinate, citrate, tartrate, lactate, mandelate, methanesulfonate (mesylate), and p-toluenesulfonate (tosylate) salts, and the like; and may be prepared through a method for preparing a salt known to the art.

**Use of Quinazoline Derivative Compounds**

One embodiment of the present disclosure is a composition for inhibiting c-KIT or PDGFR comprising a compound represented by Chemical Formula 1 below, a stereoisomer thereof, or a pharmaceutically acceptable salt thereof as an active ingredient. One embodiment of the present disclosure is also a pharmaceutical composition for treating or preventing c-KIT or PDGFR-related diseases comprising the compound.

[Chemical Formula 1]

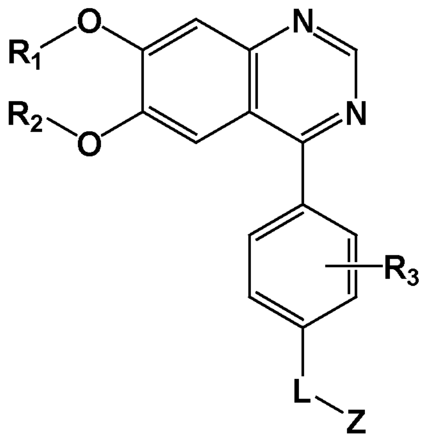

The Chemical Formula 1 is as defined above.

According to one embodiment of the present disclosure, the quinazoline derivatives represented by Chemical Formula 1 exhibits excellent inhibitory activity against c-KIT or PDGFR, and thus may be useful for treating or preventing c-KIT or PDGFR-related diseases.

In the present disclosure, the c-KIT-related diseases may be any one or more selected from cancer or mast cell-related diseases but is not limited thereto.

In the present disclosure, among c-KIT-related diseases, the cancer may be any one or more selected from the group consisting of leukemia, skin cancer, breast cancer, rectal cancer, lung cancer, neuroblastoma, thyroid cancer, germ cell tumor, prostate cancer, and gastrointestinal stromal tumor, but is not limited thereto.

In the present disclosure, among the c-KIT-related diseases, the mast cell-related diseases may be mastocytosis, acne, fibrodysplasia ossificans progressiv, and cystic fibrosis; acute nephritic syndrome, glomerulonephritis, renal amyloidosis, renal interstitial fibrosis, and inflammatory myopathy; HIV, type II diabetes, cerebral ischemia, CNS disorders, bacterial infections, interstitial cystitis, and inflammatory bowel disease; tumor angiogenesis; autoimmune diseases and allergic disorders, but is not limited thereto.

Meanwhile, in the present disclosure, the PDGFR-related disease may be any one or more selected from fibrosis, cancer, neurological disease, atherosclerosis, and pulmonary hypertension, but is not limited thereto.

In the present disclosure, among PDGFR-related diseases, the fibrosis includes all fibrosis that can exhibit preventive or therapeutic efficacy due to inhibition of PDGFR activity, and specifically it may be any one or more selected from systemic sclerosis, pulmonary fibrosis, hepatic fibrosis, cirrhosis, renal fibrosis, myelofibrosis, myocardial fibrosis, sarcoidosis, keloid, burn-induced hypertrophic scar, proliferative retinopathy, glaucoma, cataract, posterior capsular opacification, angioplasty, vascular restenosis after vascular surgery or vascular injury, cystic fibrosis, and Marfan syndrome, but is not limited thereto.

In the present disclosure, among PDGFR-related diseases, the cancer includes all cancers that can exhibit preventive or therapeutic efficacy due to inhibition of PDGFR activity, and it may be solid cancer or blood cancer. For example, the cancer may be any one or more selected from the group consisting of squamous cell carcinoma, small cell lung cancer, non-small cell lung cancer, adenocarcinoma of lung, squamous cell carcinoma of lung, peritoneal cancer, skin cancer, skin or ocular melanoma, rectal cancer, perianal gland cancer, esophageal cancer, small bowel cancer, endocrine adenocarcinoma, parathyroid cancer, adrenal cancer, soft tissue sarcoma, urethral cancer, chronic or acute leukemia, lymphocytic lymphoma, hepatocellular carcinoma, gastrointestinal cancer, stomach cancer, pancreatic cancer, glioblastoma, cervical cancer, ovarian cancer, liver cancer, bladder cancer, liver tumor, breast cancer, colon cancer, colorectal cancer, endometrial cancer or uterine cancer, salivary gland cancer, kidney cancer, prostate cancer, vulvar cancer, thyroid cancer, head and neck cancer, brain cancer, osteosarcoma, neuroblastoma, germ cell tumor, and gastrointestinal stromal tumor, but is not limited thereto. The cancer includes not only primary cancer but also metastatic

cancer.

In the present disclosure, among PDGFR-related diseases, the neurological diseases include all neurological diseases that can exhibit preventive or therapeutic effects due to inhibition of PDGFR activity, and specifically it may be any one or more selected from central nervous system diseases, neurodegenerative diseases, Alzheimer's disease, Parkinson's disease, multiple sclerosis, Huntington's disease, senile dementia, epilepsy, amyotrophic lateral sclerosis, stroke, and brain or spinal cord injury followed by nerve damage and axonal degeneration-related disorders, but is not limited thereto.

In the present disclosure, among PDGFR-related diseases, the atherosclerosis includes all atherosclerosis that can exhibit preventive or therapeutic efficacy due to inhibition of PDGFR activity.

In the present disclosure, among PDGFR-related diseases, the pulmonary hypertension includes all pulmonary hypertension that can exhibit preventive or therapeutic efficacy due to inhibition of PDGFR activity.

The pharmaceutical composition of the present disclosure may further include one or more pharmaceutically acceptable carrier in addition to the compound represented by Chemical Formula 1, the stereoisomer thereof, or the pharmaceutically acceptable salt thereof for administration. The pharmaceutically acceptable carrier may be a mixture of saline, sterile water, Ringer's solution, buffered saline, dextrose solution, malto dextrin solution, glycerol, ethanol, and one or more of these components, and other conventional additives such as antioxidants, buffers, and Bacteriostatic agent can be added as necessary. In addition, diluents, dispersants, surfactants, binders, and lubricants may be additionally added to prepare formulations for injections such as aqueous solutions, suspensions, and emulsions, pills, capsules, granules, or tablets. Accordingly, the composition of the present disclosure may be a patch, liquid, pill, capsule, granule, tablet, suppository or the like. These formulations may be prepared by a conventional method used for formulation in the art or a method disclosed in the literature [Remington's Pharmaceutical Science, Mack Publishing Company, Easton PA], and may be formulated into various formulations depending on each disease or ingredient.

The pharmaceutical composition of the present disclosure may further include one or more active ingredients exhibiting the same or similar efficacy in addition to the compound represented by Chemical Formula 1, the stereoisomer thereof, or the pharmaceutically acceptable salt thereof. The pharmaceutical composition of the present disclosure can be used for clinical administration and can be prepared to be administered in various oral and parenteral dosage forms.

The present disclosure provides a method for preventing or treating c-KIT or PDGFR-related diseases, comprising: administering to a subject in need thereof a therapeutically effective amount of the compound represented by Chemical Formula 1, the stereoisomer thereof, or the pharmaceutically acceptable salt thereof. The subject may be a mammal including a human.

The term "therapeutically effective amount" used in the present disclosure refers to the amount of compound represented by Chemical Formula 1 effective for preventing or treating c-KIT or PDGFR-related diseases. Specifically, "therapeutically effective amount" means an amount sufficient to treat a disease with a reasonable benefit/risk ratio applicable to medical treatment, and the effective dose level may be determined by factors including individual type and severity, age, sex, disease type, drug activity, drug sensitivity, administration time, administration route and discharge rate, treatment period, factors including drugs used concurrently and other factors well-known in the medical field. The pharmaceutical composition of the present disclosure may be administered as an individual therapeutic agent or in combination with other therapeutic agents, and may be administered sequentially or simultaneously with a commercially available therapeutic agent. In addition, the pharmaceutical composition of the present disclosure can be single or multiple administrations. It is important to administer the minimum amount capable of obtaining the maximum effect without side effects in consideration of all the above factors, and may be easily determined by those skilled in the art. The dosage of the pharmaceutical composition of the present disclosure may be determined by an expert according to various factors such as the patient's condition, age, sex, complications, and the like. Since the active ingredient of the pharmaceutical composition of the present disclosure has excellent safety, it may be used more than the determined dose.

In addition, the present disclosure can inhibit c-KIT or PDGFR by administering the compound represented by Chemical Formula 1, the stereoisomer thereof, or the pharmaceutically acceptable salt thereof to mammals including humans.

Further, according to one embodiment of the present disclosure, the present disclosure provides use of the compound represented by Chemical Formula 1, the stereoisomer thereof, or the pharmaceutically acceptable salt thereof for the preparation of a medicament for treating c-KIT or PDGFR-related diseases. The compound represented by Chemical Formula 1 for preparing a drug may be mixed with an acceptable adjuvant, diluent, carrier, and the like, and may be prepared as a combined preparation with other active agents to have a synergistic action of the active ingredients.

One embodiment of the present disclosure provides a method for treating or preventing any one or more diseases selected from the group consisting of cancer, mast cell-related diseases, fibrosis diseases, neurological diseases, atherosclerosis, and pulmonary hypertension, comprising administering to a subject in need thereof a therapeutically effective amounts of the compound represented by Chemical Formula 1, the stereoisomer thereof, or the pharmaceutically acceptable salts thereof.

One embodiment of the present disclosure provides a method for inhibiting c-KIT or PDGFR, comprising administering to a subject in need thereof a therapeutically effective amount of the compound represented by Chemical Formula 1, the stereoisomer thereof, or the pharmaceutically acceptable salt thereof.

One embodiment of the present disclosure provides use of the compound represented by Chemical Formula 1, the stereoisomer thereof, or the pharmaceutically acceptable salt thereof, for use in the preparation of a medicament for treating or preventing any one or more disease selected from the group consisting of cancer, mast cell-related disease, fibrosis disease, neurological disease, atherosclerosis, and pulmonary hypertension.

One embodiment of the present disclosure provides use of the compound represented by Chemical Formula 1, the stereoisomer thereof, or the pharmaceutically acceptable salt thereof, for use in the preparation of a medicament for treating or preventing c-KIT or PDGFR-related diseases.

The matters mentioned in the use, composition, treatment method, and the like of the present disclosure are equally applied unless contradictory to each other.

[Advantageous Effects]

The novel compounds of the present disclosure exhibit an action effect of inhibiting c-KIT or PDGFR activity. Accordingly, the compound of the present disclosure may be usefully used for treating or preventing c-KIT or PDGFR-related diseases.

[Best Mode]

Hereinafter, the components and effects the present disclosure will be described in more detail through Examples. However, the following Examples are merely presented to illustrate the present disclosure, and the scope of the present disclosure is not limited thereto.

The commercially available reagents used were used as they were purchased without further purification. In the present disclosure, room temperature refers to a temperature of 20 to 25°C, and concentration or solvent distillation removal under reduced pressure was performed using a rotary evaporator.

**<Methods and conditions for analysis and purification>**

In the present disclosure, a series of compounds synthesized from examples were synthesized, purified by the following method, and their structures were analyzed.

1. Analytical Conditions for Compounds

Condition of High Performance Liquid Chromatography (HPLC) for analysis

The compound was analyzed using equipment equipped with a Mass QDa detector in Waters' UPLC system (ACQUITY UPLC PDA Detector). The column used for compound purification was Waters' ACQUITY UPLC BEH C18 (1.7 um, 2.1×50 mm), and the column temperature was set at 30°C. Water containing 0.1% formic acid was used as mobile phase A, and acetonitrile containing 0.1% formic acid was used as mobile phase B. The gradient conditions were 10-100% B for 3 minutes and the flow rate was 0.6 mL/min.

In the present specification, HPLC and UPLC are used as interchangeable terms.

2. Purification Conditions for Compounds

(1) Medium Pressure Liquid Chromatography (MPLC) for purification

Medium pressure liquid chromatography was performed using TELEDYNE ISCO's CombiFlash Rf+UV (usage time 60 minutes).

(2) Prep-HPLC

1) Prep-LCMS (Preparative-Liquid chromatography mass spectrometry) for purification

The compound was purified using equipment equipped with a Mass QDa detector in Waters' Autopurification HPLC system (2767 sample manager, 2545 binary gradient module, 2998 Photodiode Array Detector). The column used was Waters' SunFire Prep C18 OBDTM (5 um, 19×50 mm), and the column temperature was maintained at room temperature. Water containing 0.035% trifluoroacetic acid was used as mobile phase A, and methanol containing 0.035%

trifluoroacetic acid was used as mobile phase B. The gradient condition was 15-100% B for 10 minutes and the flow rate was 25 mL/min.

2) Prep-150 LC system (Preparative-Liquid chromatography UV spectrometry) for purification

The compound was purified using Waters' Prep 150 LC system (2545 Quaternary gradient module, 2998 Photodiode Array Detector, Fraction Collector III). The column used was Waters' XTerra Prep RP18 OBDTM (10 um, 30×300 mm), and the column temperature was maintained at room temperature.

3) ACCQPrep HP150 system for purification

The compound was purified using Teledyne Isco's ACCQPrep HP150 system. The column used was Waters' XTerra Prep RP18 OBDTM (10 um, 30×300 mm), and the column temperature was maintained at room temperature.

3. NMR analysis

The NMR spectrum was recorded and analyzed using Bruker's AVANCE III 400 or AVANCE III 400 HD, and the acquired data was expressed in ppm (parts per million, δ).

Preparation examples are shown below. Although a salt (eg, TFA, HCl) was used in the actual preparation method, it may be excluded from the reaction scheme of each preparation examples.

**<Example 1> Preparation of *N*-(4-(7-(2-(diethylamino)ethoxy)-6-methoxyquinazoline-4-yl)phenyl)-2-(4-(trifluoromethyl)phenyl)acetamide**

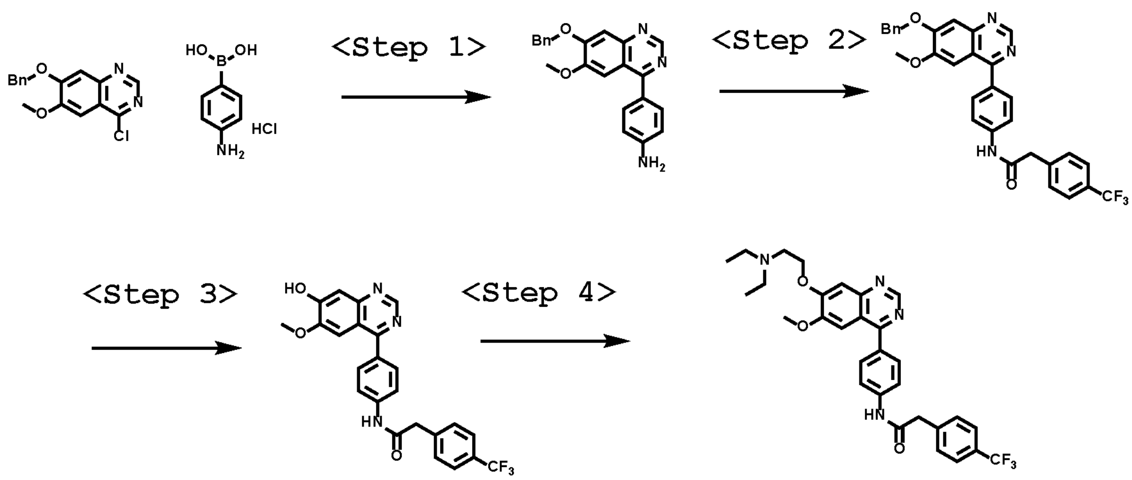


Step 1: After dissolving 4-aminophenyl boronic acid, pinacol ester, hydrochloride (0.50g, 2.88mmol), 7-(benzyloxy)-4-chloro-6-methoxyquinazoline (0.87g, 2.88mmol), tetrakis(triphenylphosphine)palladium(0) (0.30g, 0.87mmol), and sodium carbonate (0.61g, 5.77mmol) in 1,4-dioxane (11.1ml) and distilled water (3.3ml) under nitrogen, oxygen removal was performed for 10 minutes. After the reaction mixture was reacted at 80°C for 3 hours, distilled water was added to the reaction mixture, and then the organic matter was extracted with ethyl acetate (x3). After washing the collected organic layer with brine, the remaining water was removed using sodium sulfate, and concentrated under reduced pressure. The concentrated organic layer was crystallized using dichloromethane and then filtered to obtain the desired compound 4-(7-(benzyloxy)-6-methoxyquinazoline-4-yl)aniline (0.80 g, 78%).
MS(m/z): 358.3 $[M+H]^+$; UPLC r.t.(min): 1.37

Step 2: After dissolving 2-(4-(trifluoromethyl)phenyl)acetic acid (1.62g, 7.93mmol), 1-[bis(dimethylamino)methylene]-1H-1,2,3-triazolo[4,5,-b]pyridinium 3-oxide hexafluorophosphate (HATU) (4.02g, 10.58mmol) in dimethylformamide (13.2ml), *N,N*-diisopropylethylamine (2.73g, 21.15 mmol) was added dropwise. 4-(7-(benzyloxy)-6-methoxyquinazoline-4-yl)aniline (2.70 g, 5.29 mmol) obtained in the step 1 was added dropwise to the reaction mixture, and then stirred at room temperature for 3 hours. After adding distilled water to the reactant, the organic matter was extracted with ethyl acetate (x3). After washing the collected organic layer with brine, the remaining water was removed using

sodium sulfate, and concentrated under reduced pressure. The concentrated mixture was purified through MPLC (EtOAc:Hex= 10-100%) to obtain a solid desired compound *N*-(4-(7-(benzyloxy)-6-methoxyquinazoline-4-yl)phenyl)-2-(4-(trifluoromethyl)phenyl)acetamide (2.30 g, 80%).
MS(m/z): 544.3 $[M+H]^+$; UPLC r.t.(min): 1.83

Step 3: After dissolving *N*-(4-(7-(benzyloxy)-6-methoxyquinazoline-4-yl)phenyl)-2-(4-(trifluoromethyl)phenyl)acetamide (1.20g, 2.21mmol) obtained in the step 2 in methanol (11.0ml), Pd/C (0.20g, 0.44mmol) was added. The reaction mixture was stirred at 25°C for 1 hour under hydrogen. The reactant was filtered by a celite filter, washed with methanol, and then concentrated under reduced pressure. After concentration, it was purified through MPLC (DCM:MeOH= 0-10%) to obtain the desired compound *N*-(4-(7-hydroxy-6-methoxyquinazoline-4-yl)phenyl)-2-(4-(trifluoromethyl)phenyl)acetamide (0.78 g, 78%).
MS(m/z): 454.1 $[M+H]^+$ UPLC r.t.(min): 1.47

Step 4: After dissolving *N*-(4-(7-hydroxy-6-methoxyquinazoline-4-yl)phenyl)-2-(4-(trifluoromethyl)phenyl)acetamide (0.09g, 0.20mmol) obtained in the step 3, 2-chloro-*N,N*-diethylethane-1-amine hydrochloride (0.04g, 0.24mmol), and potassium carbonate (0.05g, 0.40mmol) in dimethylformamide (2.0ml), *N,N*-diisopropylethylamine (0.05g, 0.40mmol) was added dropwise, followed by stirring at 90°C for 6 hours. After confirming the completion of the reaction and adding distilled water, the organic matter was extracted with ethyl acetate (x3). After washing the collected organic layer with brine, the remaining water was removed using sodium sulfate, and concentrated under reduced pressure. After concentration, it was purified through MPLC (DCM:MeOH=0-10%) to obtain the desired compound N-(4-(7-(2-(diethylamino)ethoxy)-6-methoxyquinazoline-4-yl)phenyl)-2-(4-(trifluoromethyl)phenyl)acetamide (0.05g, 45.6 %) .
MS(m/z): 553.4 $[M+H]^+$ UPLC r.t.(min): 1.35

**<Example 2> Preparation of *N*-(4-(6-methoxy-7-((1-methylpiperidin-4-yl)oxy)quinazoline-4-yl)phenyl)-2-(4-(trifluoromethyl)phenyl)acetamide**

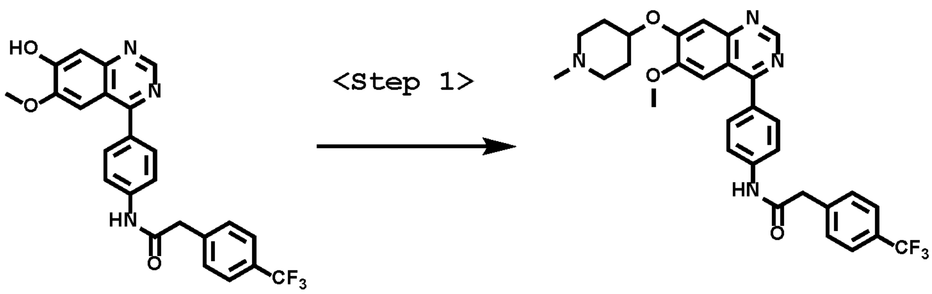


Step 1: After dissolving *N*-(4-(7-hydroxy-6-methoxyquinazoline-4-yl)phenyl)-2-(4-(trifluoromethyl)phenyl)acetamide (0.05g, 0.11mmol) obtained in the step 3 of Example 1, 1-methylpiperidin-4-ol (0.02g, 0.17mmol), and triphenylphosphine (0.09g, 0.33mmol) in dichloromethane (1.1ml), diisopropyl azodicarboxylate (0.07g, 0.33mmol) was added dropwise. After stirring at room temperature overnight, when the reaction was completed, it was concentrated under reduced pressure and purified through MPLC (DCM:MeOH=0-10%) to obtain the desired compound *N*-(4-(6-methoxy-7-((1-methylpiperidin-4-yl)oxy)quinazoline-4-yl)phenyl)-2-(4-(trifluoromethyl)phenyl)acetamide (0.02g, 33 %).
MS(m/z): 551.3 $[M+H]^+$ UPLC r.t. (min): 1.32

**<Example 3> Preparation of *N*-(4-(6-methoxy-7-(2-(4-methylpiperazin-1-yl)ethoxy)quinazoline-4-yl)phenyl)-2-(4-(trifluoromethyl)phenyl)acetamide**

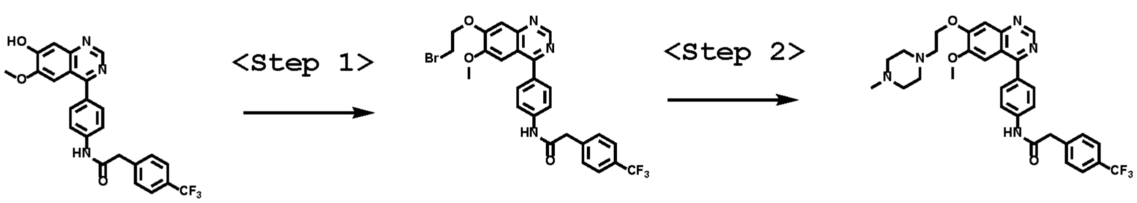

Step 1: After dissolving *N*-(4-(7-hydroxy-6-methoxyquinazolin-4-yl)phenyl)-2-(4-(trifluoromethyl)phenyl)acetamide (80.0mg, 0.18mmol) obtained in the step 3 of Example 1, 2-bromoethanol (35.3mg, 0.28mmol), and triphenylphosphine (64.8mg, 0.25mmol) in dichloromethane (1.7ml), diisopropyl azodicarboxylate (42.8mg, 0.21mmol)was added dropwise, followed by stirring at room temperature overnight. When the reaction was completed, it was concentrated under reduced pressure and purified through MPLC (EtOAc:Hex=30-100%) to obtain the desired compound *N*-(4-(7-(2-bromoethoxy)-6-methoxyquinazolin-4-yl)phenyl)-2-(4-(trifluoromethyl)phenyl)acetamide (80.0mg, 81 %).
MS(m/z): 562.1 $[M+H]^+$ UPLC r.t.(min): 1.71

Step 2: After dissolving *N*-(4-(7-(2-bromoethoxy)-6-methoxyquinazolin-4-yl)phenyl)-2-(4-(trifluoromethyl)phenyl)acetamide (0.05g, 0.09mmol) obtained in the step 1 and 1-methylpiperazine (0.04g, 0.36mmol) in dimethylformamide (0.9ml), *N,N*-diisopropylethylamine (0.02g, 0.18mmol) was added dropwise and then stirred overnight at room temperature. After the completion of the reaction, distilled water was added and the organic matter was extracted with ethyl acetate(x3). After washing the collected organic layer with brine, the remaining water was removed using sodium sulfate, and concentrated under reduced pressure. The concentrated mixture was purified through MPLC (EtOAc:Hex=30-100%), and the desired compound *N*-(4-(6-methoxy-7-(2-(4-methylpiperazin-1-yl)ethoxy)quinazolin-4-yl)phenyl)-2-(4-(trifluoromethyl)phenyl)acetamide (11.0mg, 65.5 %) was obtained.
MS(m/z): 580.4 $[M+H]^+$ UPLC r.t.(min): 1.26

**<Example 4> Preparation of *N*-(4-(7-((1-ethylpiperidin-4-yl)methoxy)-6-methoxyquinazolin-4-yl)phenyl)-2-(4-(trifluoromethyl)phenyl)acetamide**

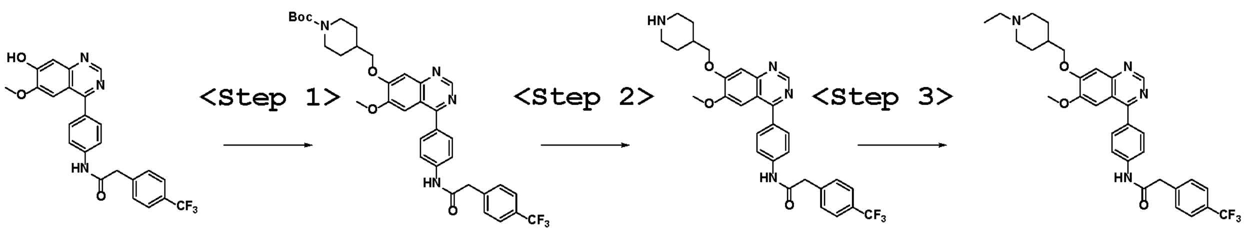


Step 1: After dissolving *N*-(4-(7-hydroxy-6-methoxyquinazolin-4-yl)phenyl)-2-(4-(trifluoromethyl)phenyl)acetamide (0.10g, 0.22mmol) obatained in the step 3 of Example 1 and *tert*-butyl 4-((tosyloxy)methyl)piperidin-1-carboxylate (0.12g, 0.33mmol) in dimethylformamide (1.1ml), potassium carbonate (0.06g, 0.44mmol) was added dropwise. After stirring the reaction mixture at 70°C overnight, when the reaction was complete, the desired compound tert-butyl 4-(((6-methoxy-4-(4-(2-(4-(trifluoromethyl)phenyl)acetamido)phenyl)quinazolin-7-yl)oxy)methyl)piperidin-1-carboxylate (0.08g, 55.7 %) was obtained by purification through MPLC (DMC:MeOH= 0-10%).
MS(m/z): 651.4 $[M+H]^+$ UPLC r.t.(min): 1.99

Step 2: After dissolving *tert*-butyl 4-(((6-methoxy-4-(4-(2-(4-(trifluoromethyl)phenyl)acetamido)phenyl)quinazolin-7-yl)oxy)methyl)piperidin-1-carboxylate (0.08g, 0.12mmol) obtained in the step 1 in dichloromethane (1.2ml), trifluoroacetic acid (0.14g, 1.229mmol) was added slowly dropwise at 0°C. After stirring the reaction mixture at room temperature overnight, it was concentrated and purified using Prep-HPLC to obtain the desired compound N-(4-(6-methoxy-7-(piperidin-4-ylmethoxy)quinazolin-4-yl)phenyl)-2-(4-(trifluoromethyl)phenyl)acetamide 2,2,2-trifluoroacetate (7.5mg, 92 %).
MS(m/z): 551.3 $[M+H]^+$ UPLC r.t.(min): 1.40

Step 3: After dissolving N-(4-(6-methoxy-7-(piperidin-4-ylmethoxy)quinazolin-4-yl)phenyl)-2-(4-(trifluoromethyl)phenyl)acetamide 2,2,2-trifluoroacetate (0.03g, 0.05mmol) obtained in the step 2, sodium triacetoxyhydroborate (0.02g, 0.09mmol), acetaldehyde (2.98mg, 0.07mmol) in 1,4-dioxane (0.5ml), *N,N*-diisopropylethylamine (0.03g, 0.23mmol) was added dropwise. After stirring the reaction mixture at room temperature overnight, when the reaction was completed, it was purified through prep-HPLC to obtain the desired compound *N*-(4-(7-((1-ethylpiperidin-4-yl)methoxy)-6-methoxyquinazolin-4-yl)phenyl)-2-(4-(trifluoromethyl)phenyl)acetamide (0.01g, 38 %).
MS(m/z): 595.4 $[M+H]^+$ UPLC r.t.(min): 1.50

**Examples 1 to 54**

Examples 5 to 54 were prepared in a similar manner to Examples 1 to 4, and the compound names, NMR, mass, and UPLC analysis results of Examples 1 to 54 are summarized in Table 1 below.

In the above preparation example, even if the compound is prepared in the form of a salt, it is listed in the table in a Free Form. Therefore, the salt practically used in the preparation method may be added to the compound in which

no salt is added, or other salts may be added.

[Table 1]

| Example | Structure | Compound Name | $^1$H NMR; MS(m/z) | UPLC r.t. (min) |
|---|---|---|---|---|
| 1 | 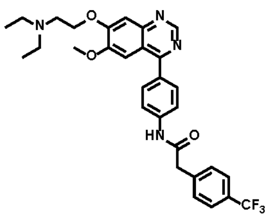 | *N*-(4-(7-(2-(diethylamino) ethoxy )-6-methoxyquinazolin-4-yl)phenyl)-2-(4-(trifluoromethyl)phe nyl) acetamide | $^1$H NMR (400 MHz, MeOD-$d_4$): δ 9.02 (s, 1H), 7.86 (d, *J* = 8.5 Hz, 2H), 7.77 (d, *J* = 8.7 Hz, 2H), 7.66 (d, *J* = 8.1 Hz, 2H), 7.58 (d, *J* = 8.1 Hz, 2H), 7.40 - 7.35 (m, 2H), 4.36 (t, *J* = 5.5 Hz, 2H), 3.93 - 3.82 (m, 5H), 3.12 (t, *J* = 5.4 Hz, 2H), 2.79 (q, *J* = 7.2 Hz, 4H), 1.15 (t, *J* = 7.1 Hz, 6H) 553.3 [M+H]$^+$ | 1.35 |
| 2 | 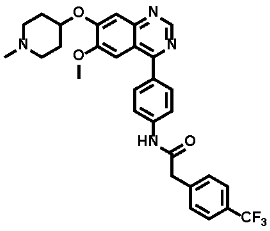 | *N*-(4-(6-methoxy-7-((1-methylpiperidin-4-yl)oxy) quinazolin-4-yl)phenyl)-2-(4-(trifluoromethyl)phe nyl) acetamide | 551.3 [M+H]$^+$ | 1.32 |
| 3 | 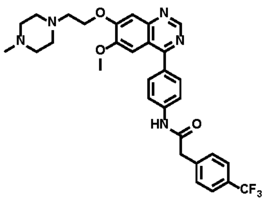 | *N*-(4-(6-methoxy-7-(2-(4-methylpiperazin-1-yl)ethoxy) quinazolin -4-yl)phenyl)-2-(4-(trifluoromethyl)phe nyl) acetamide | 580.4 [M+H]$^+$ | 1.26 |
| 4 | 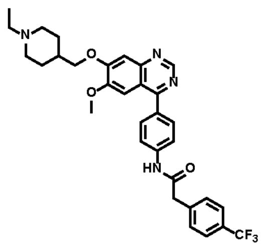 | *N*-(4-(7-((1-ethylpiperidin-4-yl) methoxy)-6-methoxyquinazolin-4-yl) phenyl)-2-(4-(trifluoromethyl) phe nyl)acetamide | 579.3 [M+H]$^+$ | 1.51 |
| 5 | 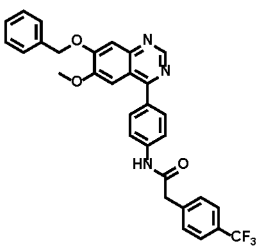 | *N*-(4-(7-(benzyloxy)-6-methoxyquinazolin-4-yl) phenyl)-2-(4-(trifluoromethyl) phe nyl)acetamide | 544.2 [M+H]$^+$ | 1.81 |

(continued)

| Example | Structure | Compound Name | $^1H$ NMR; MS(m/z) | UPLC r.t. (min) |
|---|---|---|---|---|
| 6 | 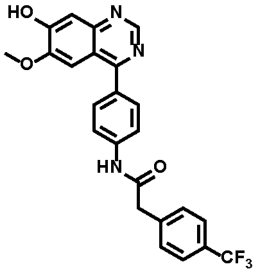 | *N*-(4-(7-hydroxy-6-methoxyquinazolin-4-yl) phenyl)-2-(4-(trifluoromethyl) phe nyl)acetamide | 454.1 $[M+H]^+$ | 1.45 |
| 7 | 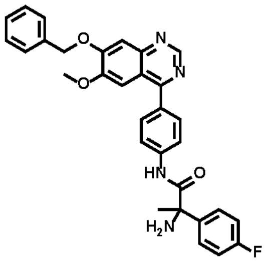 | 2-amino-*N*-(4-(7-(benzyloxy)-6-methoxyquinazolin-4-yl) phenyl)-2-(4-fluorophenyl) propana mide | 523.3 $[M+H]^+$ | 1.33 |
| 8 | 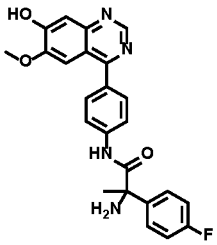 | 2-amino-2-(4-fluorophenyl)-*N*-(4-(7-hydroxy-6-methoxyquinazolin-4-yl)phenyl)propanamid e | 433.2 $[M+H]^+$ | 0.96 |
| 9 | 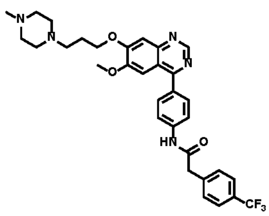 | *N*-(4-(6-methoxy-7-(3-(4-methylpiperazin-1-yl)propoxy) quinazoli n-4-yl)phenyl)-2-(4-(trifluoromethyl)phe nyl) acetamide | $^1H$ NMR (400 MHz, $CDCl_3$) δ 9.15 (s, 1H), 7.75 (d, *J* = 8.8 Hz, 2 H), 7.67 (d, *J* = 8.8 Hz, 4 H), 7.51 (d, *J* = 8 Hz, 2 H), 7.35 (s, 1H), 7.30-7.29 (m, 2H), 4.35 (t, *J* = 6, 6.4 Hz, 2H), 3.86-3.84 (m, 5H), 2.96 (t, *J* = 6 Hz, 2H), 2.67 (brs, 4H), 2.48 (brs, 4H), 2.30 (s, 1H)<br>594.4 $[M+H]^+$ | 1.22 |
| 10 | 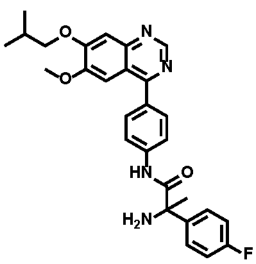 | 2-amino-2-(4-fluorophenyl)-*N*-(4-(7-isobutoxy-6-methoxyquinazolin-4-yl) phenyl)propanamid e | 498.2 $[M+H]^+$ | 1.34 |

(continued)

| Example | Structure | Compound Name | $^1$H NMR; MS(m/z) | UPLC r.t. (min) |
|---|---|---|---|---|
| 11 | 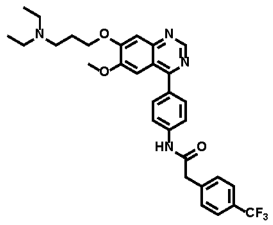 | *N*-(4-(7-(3-(diethylamino) propox y)-6-methoxyquinazolin-4-yl) phenyl)-2-(4-(trifluoromethyl) phe nyl) acetamide | $^1$H NMR (400 MHz, MeOD-$d_4$): δ δ 9.00 (s, 1H), 7.86 (d, *J* = 8.4 Hz, 2H), 7.76 (d, *J* = 8.4 Hz, 2H), 7.66 (d, *J* = 8.1 Hz, 2H), 7.58 (d, *J* = 8.1 Hz, 2H), 7.39 - 7.31 (m, 2H), 4.26 (t, *J* = 6.0 Hz, 2H), 3.90 - 3.81 (m, 5H), 2.86 - 2.78 (m, 2H), 2.69 (q, *J* = 7.2 Hz, 4H), 2.10 (dd, *J* = 9.5, 5.8 Hz, 2H), 1.12 (t, *J* = 7.2 Hz, 6H)<br>567.3 [M+H]$^+$ | 1.36 |
| 12 | 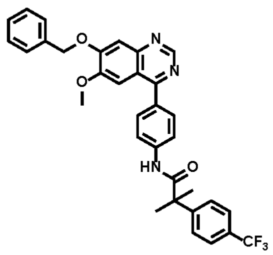 | *N*-(4-(7-(benzyloxy)-6-methoxyquinazolin-4-yl) phenyl)-2-methyl-2-(4-(trifluoromethyl)phe nyl) propanamide | 572.4 [M+H]$^+$ | 1.99 |
| 13 | 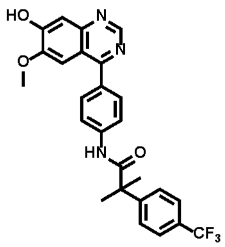 | *N*-(4-(7-hydroxy-6-methoxyquinazolin-4-yl) phenyl)-2-methyl-2-(4-(trifluoromethyl)phe nyl) propanamide | 482.2 [M+H]$^+$ | 1.63 |
| 14 | 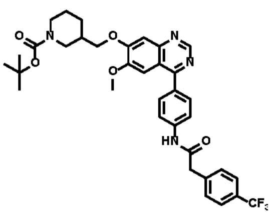 | *tert*-butyl 3-(((6-methoxy-4-(4-(2-(4-(trifluoromethyl)phe nyl)acetamido)phenyl ) quinazolin-7-yl)oxy)methyl) piperi din-1-carboxylate | 651.4 [M+H]$^+$ | 2.00 |
| 15 | 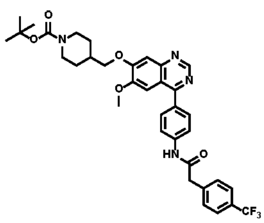 | *tert*-butyl 4-(((6-methoxy-4-(4-(2-(4-(trifluoromethyl)phe nyl)acetamido)phenyl ) quinazolin-7-yl)oxy)methyl) piperi din-1-carboxylate | 651.4 [M+H]$^+$ | 2.00 |

(continued)

| Example | Structure | Compound Name | $^1$H NMR; MS(m/z) | UPLC r.t. (min) |
|---|---|---|---|---|
| 16 | 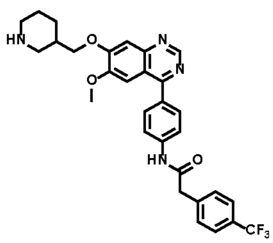 | *N*-(4-(6-methoxy-7-(piperidin-3-ylmethoxy)quinazolin -4-yl) phenyl)-2-(4-(trifluoromethyl) phe nyl)acetamide | 551.3 $[M+H]^+$ | 1.41 |
| 17 | 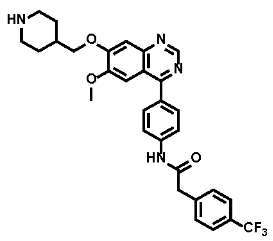 | *N*-(4-(6-methoxy-7-(piperidin-4-ylmethoxy)quinazolin -4-yl) phenyl)-2-(4-(trifluoromethyl) phe nyl)acetamide | 551.2 $[M+H]^+$ | 1.40 |
| 18 | 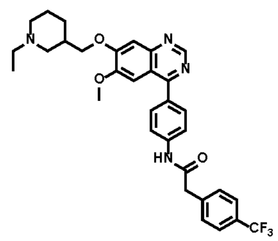 | *N*-(4-(7-((1-ethylpiperidin-3-yl) methoxy)-6-methoxyquinazolin-4-yl) phenyl)-2-(4-(trifluoromethyl) phe nyl)acetamide | $^1$H NMR (400 MHz, MeOD-$d_4$): δ 8.99 (s, 1H), 7.86 (d, *J* = 8.5 Hz, 2H), 7.75 (d, *J* = 8.3 Hz, 2H), 7.66 (d, *J* = 8.1 Hz, 2H), 7.58 (d, *J* = 8.1 Hz, 2H), 7.35 - 7.28 (m, 2H), 4.20 (dd, *J* = 9.7, 5.0 Hz, 1H), 4.11 (dd, *J* = 9.7, 6.7 Hz, 1H), 3.90 - 3.82 (m, 5H), 3.71 - 3.63 (m, 1H), 3.56 - 3.46 (m, 1H), 3.14 (qd, *J* = 7.3, 2.4 Hz, 2H), 2.87 - 2.76 (m, 2H), 2.54 - 2.41 (m, 1H), 2.08 - 1.98 (m, 2H), 1.92 - 1.85 (m, 1H), 1.56 - 1.44 (m, 1H), 1.34 (t, *J* = 7.3 Hz, 3H)<br>579.3 $[M+H]^+$ | 1.51 |
| 19 | 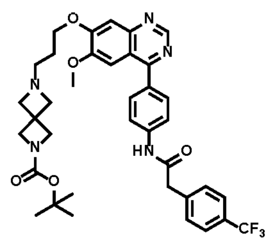 | *tert*-butyl 6-(3-((6-methoxy-4-(4-(2-(4-(trifluoromethyl)phe nyl)acetamido)phenyl ) quinazolin-7-yl)oxy)propoxy)-2,6-diazaspiro[3.3]hepta ne-2-carboxylate | 692.5 $[M+H]^+$ | 1.58 |
| 20 | 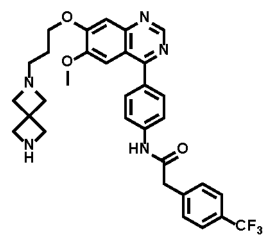 | *N*-(4-(7-(3-(2,6-diazaspiro[3.3] hepta ne-2-yl)propoxy)-6-methoxyquinazolin-4-yl) phenyl)-2-(4-(trifluoromethyl) phe nyl)acetamide | 592.3 $[M+H]^+$ | 1.23 |

(continued)

| Example | Structure | Compound Name | $^1$H NMR; MS(m/z) | UPLC r.t. (min) |
|---|---|---|---|---|
| 21 | 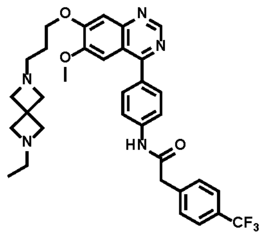 | *N*-(4-(7-(3-(6-ethyl-2,6-diazaspiro[3.3]hepta ne-2-yl) propoxy)-6-methoxyquinazolin-4-yl) phenyl)-2-(4-(trifluoromethyl) phe nyl)acetamide | 620.4 [M+H]$^+$ | 1.33 |
| 22 | 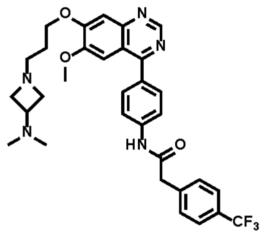 | *N*-(4-(7-(3-(3-(dimethylamino) azeti dine-1-yl)propoxy)-6-methoxyquinazolin-4-yl) phenyl)-2-(4-(trifluoromethyl) phe nyl)acetamide | 594.3 [M+H]$^+$ | 1.27 |
| 23 | 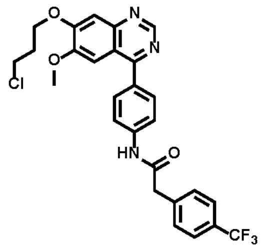 | *N*-(4-(7-(3-chloropropoxy)-6-methoxyquinazolin-4-yl) phenyl)-2-(4-(trifluoromethyl) phe nyl)acetamide | 530.2 [M+H]$^+$ | 1.77 |
| 24 | 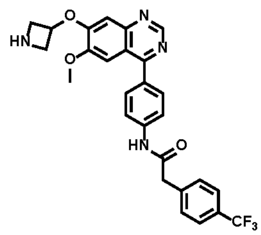 | *N*-(4-(7-(azetidine-3-yloxy)-6-methoxyquinazolin-4-yl) phenyl)-2-(4-(trifluoromethyl) phe nyl)acetamide | 509.3 [M+H]$^+$ | 1.63 |
| 25 | 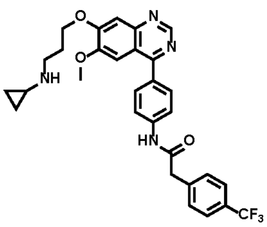 | *N*-(4-(7-(3-(cyclopropylamino) pr opoxy)-6-methoxyquinazolin-4-yl) phenyl)-2-(4-(trifluoromethyl) phe nyl)acetamide | 551.3 [M+H]$^+$ | 1.68 |
| 26 | 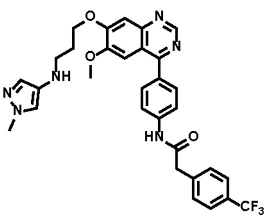 | *N*-(4-(6-methoxy-7-(3-((1-methyl-1*H*-pyrazol-4-yl) amino)propoxy)qui nazolin-4-yl)phenyl)-2-(4-(trifluoromethyl)phe nyl) acetamide | 591.3 [M+H]$^+$ | 1.68 |

(continued)

| Example | Structure | Compound Name | $^1$H NMR; MS(m/z) | UPLC r.t. (min) |
|---|---|---|---|---|
| 27 | 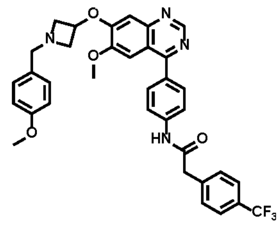 | *N*-(4-(6-methoxy-7-((1-(4-methoxybenzyl)azetid in-3-yl) oxy)quinazolin-4-yl)phenyl)-2-(4-(trifluoromethyl)phe nyl) acetamide | 629.4 [M+H]$^+$ | 1.45 |
| 28 | 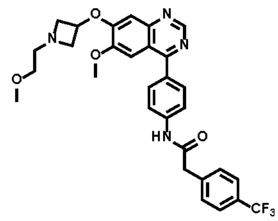 | *N*-(4-(6-methoxy-7-((1-(2-methoxyethyl)azetidi n-3-yl) oxy)quinazolin-4-yl)phenyl)-2-(4-(trifluoromethyl)phe nyl) acetamide | 571.3 [M+H]$^+$ | 1.36 |
| 29 | 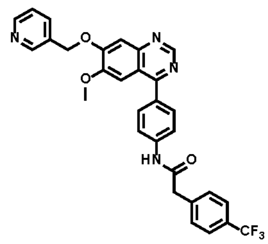 | *N*-(4-(6-methoxy-7-(pyridin-3-ylmethoxy)quinazolin -4-yl) phenyl)-2-(4-(trifluoromethyl) phe nyl)acetamide | 545.3 [M+H]$^+$ | 1.45 |
| 30 | 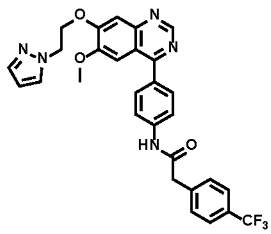 | *N*-(4-(7-(2-(1*H*-pyrazol-1-yl) ethoxy)-6-methoxyquinazolin-4-yl)phenyl)-2-(4-(trifluoromethyl)phe nyl) acetamide | $^1$H NMR (400 MHz, MeOD-$d_4$): δ 8.99 (s, 1H), 7.84 (d, *J* = 8.7 Hz, 2H), 7.79 (d, *J* = 2.3 Hz, 1H), 7.77 - 7.72 (m, 2H), 7.66 (d, *J* = 8.2 Hz, 2H), 7.58 (d, *J* = 8.1 Hz, 2H), 7.51 (d, *J* = 1.9 Hz, 1H), 7.36 - 7.30 (m, 2H), 6.29 (t, *J* = 2.1 Hz, 1H), 4.67 (t, *J* = 5.0 Hz, 2H), 4.55 (t, *J* = 5.0 Hz, 2H), 3.90 - 3.78 (m, 5H) 548.3 [M+H]$^+$ | 1.60 |
| 31 | 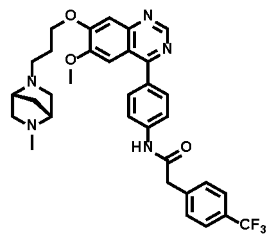 | *N*-(4-(6-methoxy-7-(3-((1*R*, 4*R*)-5-methyl-2,5-diazabicyclo [2.2.1]h eptane-2-yl)propoxy) quinazoli n-4-yl)phenyl)-2-(4-(trifluoromethyl)phe nyl) acetamide | 606.3 [M+H]$^+$ | 1.21 |
| 32 | 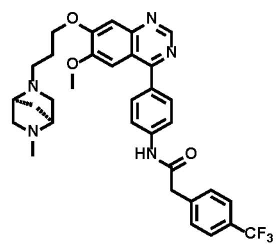 | *N*-(4-(6-methoxy-7-(3-((1*s*,4*s*)-5-methyl-2,5-diazabicyclo [2.2.1]h eptane-2-yl)propoxy) quinazoli n-4-yl)phenyl)-2-(4-(trifluoromethyl)phe nyl) acetamide | 606.4 [M+H]$^+$ | 1.29 |

(continued)

| Example | Structure | Compound Name | $^1$H NMR; MS(m/z) | UPLC r.t. (min) |
|---|---|---|---|---|
| 33 | 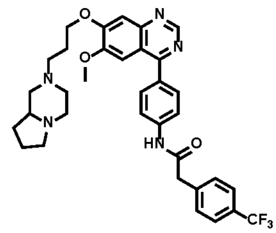 | *N*-(4-(7-(3-(hexahydropyrrolo [1, 2-*a*]pyrazin-2(1*H*)-yl) propoxy)-6-methoxyquinazolin-4-yl) phenyl)-2-(4-(trifluoromethyl) phe nyl)acetamide | 620.4 [M+H]$^+$ | 1.29 |
| 34 | 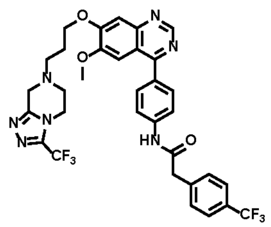 | *N*-(4-(6-methoxy-7-(3-(3-(trifluoromethyl)-5,6-dihydro-[1,2,4]triazole[4,3-a] pyrazin-7(8*H*)-yl)propoxy) quinazoli n-4-yl)phenyl)-2-(4-(trifluoromethyl)phe nyl) acetamide | 686.3 [M+H]$^+$ | 1.60 |
| 35 | 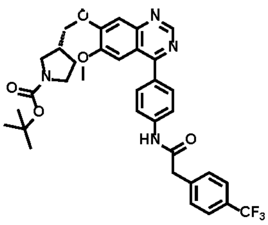 | *tert*-butyl (*R*)-3-(((6-methoxy-4-(4-(2-(4-(trifluoromethyl)phe nyl)acetamido)phenyl ) quinazolin-7-yl)oxy)methyl) pyrrol idin-1-carboxylate | 637.4 [M+H]$^+$ | 1.86 |
| 36 | 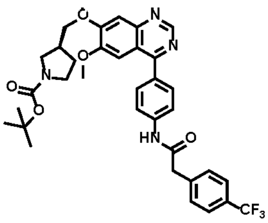 | *tert*-butyl (*S*)-3-(((6-methoxy-4-(4-(2-(4-(trifluoromethyl)phe nyl)acetamido)phenyl ) quinazolin-7-yl)oxy)methyl) pyrrol idin-1-carboxylate | $^1$H NMR (400 MHz, MeOD-$d_4$): δ 9.01 (s, 1H), 7.86 (d, *J* = 8.0 Hz, 2H), 7.77 (d, *J* = 8.0 Hz, 2H), 7.66 (d, *J* = 8.1 Hz, 2H), 7.58 (d, *J* = 8.1 Hz, 2H), 7.36 (s, 2H), 4.29 - 4.22 (m, 1H), 4.21 - 4.14 (m, 1H), 3.92 - 3.84 (m, 5H), 3.67 - 3.60 (m, 1H), 3.57 - 3.48 (m, 1H), 3.43 - 3.35 (m, 1H), 3.29 - 3.24 (m, 1H), 2.86 - 2.76 (m, 1H), 2.21 - 2.11 (m, 1H), 1.97 - 1.82 (m, 1H), 1.47 (s, 9H) 637.4 [M+H]$^+$ | 1.86 |
| 37 | 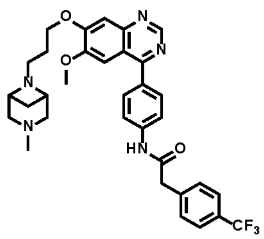 | *N*-(4-(6-methoxy-7-(3-(3-methyl-3,6-diazabicyclo[3.1.1] h eptane-6-yl)propoxy) quinazoli n-4-yl)phenyl)-2-(4-(trifluoromethyl)phe nyl) acetamide | 606.3 [M+H]$^+$ | 1.23 |

(continued)

| Example | Structure | Compound Name | $^1$H NMR; MS(m/z) | UPLC r.t. (min) |
|---|---|---|---|---|
| 38 | 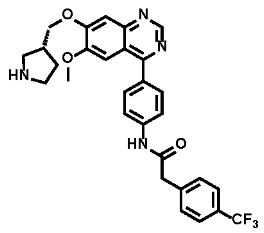 | (*R*)-*N*-(4-(6-methoxy-7-(pyrrolidin-3-ylmethoxy) quinazolin -4-yl)phenyl)-2-(4-(trifluoromethyl)phe nyl) acetamide | 537.3 $[M+H]^+$ | 1.30 |
| 39 | 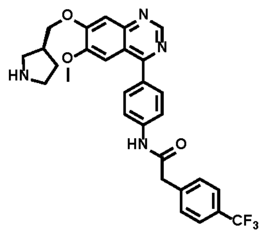 | (*S*)-*N*-(4-(6-methoxy-7-(pyrrolidin-3-ylmethoxy) quinazolin -4-yl)phenyl)-2-(4-(trifluoromethyl)phe nyl) acetamide | 537.3 $[M+H]^+$ | 1.30 |
| 40 | 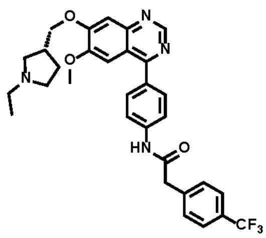 | (*R*)-*N*-(4-(7-((1-ethylpyrrolidin-3-yl)methoxy)-6-methoxyquinazolin-4-yl) phenyl)-2-(4-(trifluoromethyl) phe nyl)acetamide | 565.4 $[M+H]^+$ | 1.34 |
| 41 | 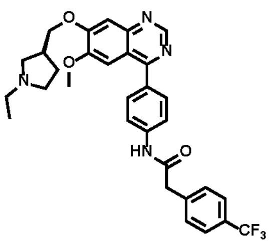 | (*S*)-*N*-(4-(7-((1-ethylpyrrolidin-3-yl)methoxy)-6-methoxyquinazolin-4-yl) phenyl)-2-(4-(trifluoromethyl) phe nyl)acetamide | 565.4 $[M+H]^+$ | 1.34 |
| 42 | 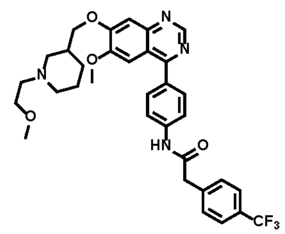 | *N*-(4-(6-methoxy-7-((1-(2-methoxyethyl)piperid in-3-yl) methoxy)quinazoli n-4-yl) phenyl)-2-(4-(trifluoromethyl) phe nyl)acetamide | 609.3 $[M+H]^+$ | 1.36 |
| 43 | 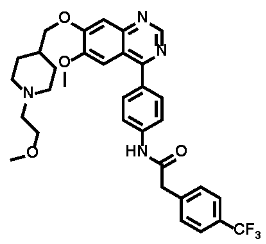 | *N*-(4-(6-methoxy-7-((1-(2-methoxyethyl)piperid in-4-yl) methoxy)quinazoli n-4-yl) phenyl)-2-(4-(trifluoromethyl) phe nyl)acetamide | 609.3 $[M+H]^+$ | 1.35 |

(continued)

| Example | Structure | Compound Name | $^1$H NMR; MS(m/z) | UPLC r.t. (min) |
|---|---|---|---|---|
| 44 | 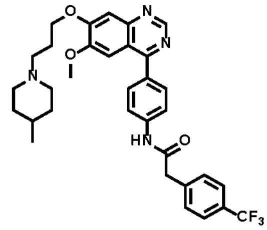 | *N*-(4-(6-methoxy-7-(3-(4-methylpiperidin-1-yl)propoxy) quinazoli n-4-yl)phenyl)-2-(4-(trifluoromethyl)phe nyl) acetamide | 593.3 [M+H]$^+$ | 1.39 |
| 45 | 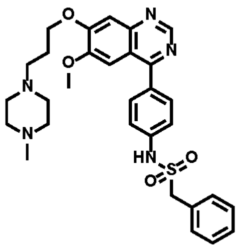 | *N*-(4-(6-methoxy-7-(3-(4-methylpiperazin-1-yl)propoxy) quinazoli n-4-yl)phenyl)-1-phenylmethanesulfona mide | 561.2 [M+H]$^+$ | |
| 46 | 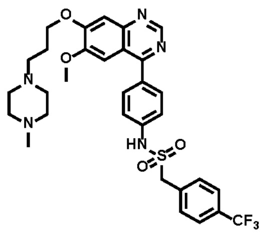 | *N*-(4-(6-methoxy-7-(3-(4-methylpiperazin-1-yl)propoxy) quinazoli n-4-yl)phenyl)-1-(4-(trifluoromethyl)phe nyl) methanesulfonami de | 629.2 [M+H]$^+$ | |
| 47 | 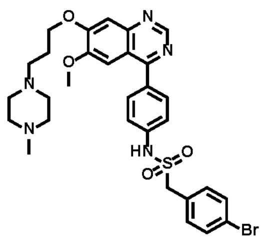 | 1-(4-bromophenyl)-*N*-(4-(6-methoxy-7-(3-(4-methylpiperazin-1-yl)propoxy) quinazoli n-4-yl)phenyl) methanesul fonamide | 639.1 [M+H]$^+$ | |
| 48 | 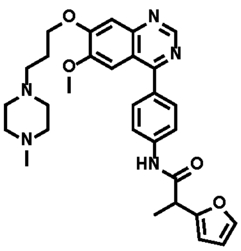 | 2-(furan-2-yl)-*N*-(4-(6-methoxy-7-(3-(4-methylpiperazin-1-yl)propoxy) quinazoli n-4-yl)phenyl) propanamid e | $^1$H NMR (400 MHz, DMSO-$d_6$): δ 10.34 (d, *J* = 4.4 Hz, 1H), 9.08 (s, 1H), 8.45 (d, *J* = 4.5 Hz, 1H), 8.27 (d, *J* = 8.4 Hz, 1H), 7.83 (q, *J* = 8.6 Hz, 4H), 7.60 (d, *J* = 3.1 Hz, 1H), 7.40 (s, 1H), 7.36 (s, 1H), 7.27 (dd, *J* = 8.3, 4.3 Hz, 1H), 4.26 (t, *J* = 6.4 Hz, 2H), 3.85 (s, 3H), 3.78 (q, *J* = 7.0 Hz, 2H), 2.62-2.53 (m, 8H), 2.30 (s, 3H), 1.98 (t, *J* = 6.8 Hz, 2H), 1.41 (d, *J* = 7.0 Hz, 3H).<br>530.1 [M+H]$^+$ | 1.28 |

(continued)

| Example | Structure | Compound Name | $^1$H NMR; MS(m/z) | UPLC r.t. (min) |
|---|---|---|---|---|
| 49 | 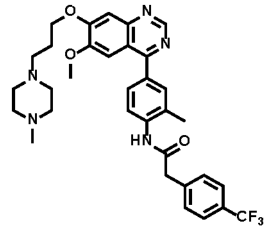 | *N*-(4-(6-methoxy-7-(3-(4-methylpiperazin-1-yl)propoxy) quinazoli n-4-yl)-2-methylphenyl)-2-(4-(trifluoromethyl)phe nyl) acetamide | $^1$H NMR (400 MHz, $CDCl_3$) δ 9.16 (s, 1H), 7.95 (d, *J* = 8.2 Hz, 1H), 7.83 (s, 1H), 7.65 (d, *J* = 7.9 Hz, 2H), 7.57-7.48 (m, 4H), 7.37 (s, 1H), 7.28 (s, 1H), 4.28 (t, *J* = 6.6 Hz, 2H), 3.88 (s, 2H), 3.86 (s, 3H), 2.88-2.33 (m, 10H), 2.30 (s, 3H), 2.14 (d, *J* = 6.7 Hz, 5H). 608.2 [M+H]$^+$ | 1.36 |
| 50 | 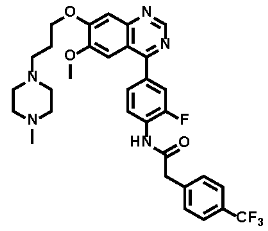 | *N*-(2-fluoro-4-(6-methoxy-7-(3-(4-methylpiperazin-1-yl) propoxy)quinazoli n-4-yl) phenyl)-2-(4-(trifluoromethyl) phe nyl) acetamide | $^1$H NMR (400 MHz, $CDCl_3$) δ 9.15 (s, 1H), 8.51 (t, *J* = 8.3 Hz, 1H), 7.82 (d, *J* = 3.3 Hz, 1H), 7.67 (d, *J* = 8.0 Hz, 2H), 7.60-7.49 (m, 4H), 7.39 (s, 1H), 7.28 (s, 1H), 4.29 (t, *J* = 6.6 Hz, 2H), 3.89 (s, 2H), 3.88 (s, 3H), 2.86-2.32 (m, 10H), 2.31 (s, 3H), 2.13 (p, *J* = 6.8 Hz, 2H) . 612.2 [M+H]$^+$ | 1.44 |
| 51 | 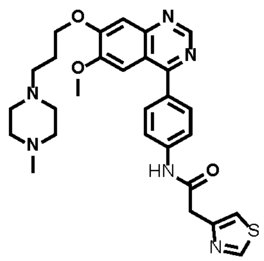 | *N*-(4-(6-methoxy-7-(3-(4-methylpiperazin-1-yl)propoxy) quinazoli n-4-yl)phenyl)-2-(thiazole-4-yl) acetamide | $^1$H NMR (400 MHz, DMSO-*d*$_6$): δ 10.57 (s, 1H), 9.09 (s, 1H), 9.07 (d, *J* = 2.0 Hz, 1H), 7.87 (d, *J* = 8.8 Hz, 2H), 7.82 (d, *J* = 8.7 Hz, 2H), 7.58 (d, *J* = 2.0 Hz, 1H), 7.40 (s, 1H), 7.36 (s, 1H), 4.25 (t, *J* = 6.4 Hz, 2H), 3.94 (s, 2H), 3.85 (s, 3H), 2.47 (t, *J* = 7.0 Hz, 2H), 2.44-2.37 (m, 8H), 2.20 (s, 3H), 1.98 (q, *J* = 6.7 Hz, 2H) . 533.1 [M+H]$^+$ | 1.10 |
| 52 | 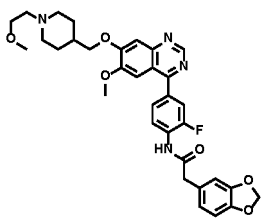 | 2-(benzo[*d*][1,3]dioxol -5-yl)-*N*-(2-fluoro-4-(6-methoxy-7-((1-(2-methoxyethyl)piperid in-4-yl)methoxy)quinazoli n-4-yl)phenyl)acetamide | $^1$H NMR (400 MHz, $CDCl_3$): δ 9.15 (s, 1H), 8.56 (t, *J* = 8.2 Hz, 1H), 7.63 - 7.51 (m, 3H), 7.27 (d, *J* = 5.5 Hz, 2H), 6.90 - 6.79 (m, 3H), 6.01 (s, 2H), 4.07 (d, *J* = 6.5 Hz, 2H), 3.88 (s, 3H), 3.74 (s, 2H), 3.57 (s, 2H), 3.36 (s, 3H), 3.10 (s, 1H), 2.66 (s, 2H), 2.09 (d, *J* = 40.1 Hz, 4H), 1.92 (d, *J* = 13.1 Hz, 2H), 1.59 (d, *J* = 10.8 Hz, 2H) 603.2 [M+H]$^+$ | |

(continued)

| Example | Structure | Compound Name | $^1$H NMR; MS(m/z) | UPLC r.t. (min) |
|---|---|---|---|---|
| 53 | 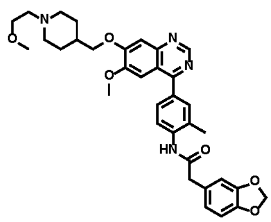 | 2-(benzo[*d*][1,3]dioxol -5-yl)-*N*-(4-(6-methoxy-7-((1-(2-methoxyethyl)piperid in-4-yl) methoxy)quinazolin-4-yl)-2-methylphenyl)acetami de | $^1$H NMR (400 MHz, $CDCl_3$): δ 9.14 (s, 1H), 8.24 (d, *J* = 8.7 Hz, 1H), 7.61 (d, *J* = 6.9 Hz, 2H), 7.32 (s, 2H), 7.20 (s, 1H), 6.93 - 6.82 (m, 3H), 6.02 (s, 2H), 4.06 (d, *J* = 6.6 Hz, 2H), 3.87 (s, 3H), 3.75 (s, 2H), 3.53 (t, *J* = 5.6 Hz, 2H), 3.36 (s, 3H), 3.03 (d, *J* = 11.1 Hz, 2H), 2.60 (d, *J* = 7.0 Hz, 2H), 2.07 (s, 3H), 2.03 (d, *J* = 11.7 Hz, 2H), 1.90 (d, *J* = 13.0 Hz, 2H), 1.58 - 1.45 (m, 3H)<br>599.2 [M+H]$^+$ | |
| 54 | 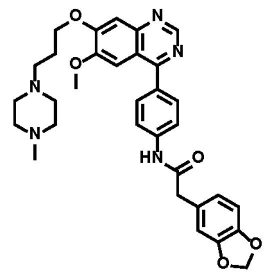 | 2-(benzo[*d*][1,3]dioxol -5-yl)-*N*-(4-(6-methoxy-7-(3-(4-methylpiperazin-1-yl)propoxy) quinazoli n-4-yl)phenyl) acetamide | $^1$H NMR (400 MHz, DMSO-$d_6$): δ 10.40 (s, 1H), 9.09 (s, 1H), 7.83 (d, *J* = 1.8 Hz, 3H), 7.40 (s, 1H), 7.36 (s, 1H), 6.95 (d, *J* = 1.6 Hz, 1H), 6.89 (d, *J* = 7.9 Hz, 1H), 6.82 (dd, *J* = 8.0, 1.7 Hz, 1H), 6.00 (s, 1H), 4.25 (t, *J* = 6.4 Hz, 2H), 3.85 (s, 3H), 3.61 (s, 2H), 2.53 (d, *J* = 1.8 Hz, 4H), 2.45 (t, *J* = 7.1 Hz, 4H), 2.15 (s, 4H), 1.97 (t, *J* = 6.9 Hz, 2H), 1.24 (s, 3H).<br>570.2 [M+H]$^+$ | 1.30 |

**<Example 55> Preparation of *N*-(4-(6-methoxy-7-(3-(4-methylpiperazin-1-yl)propoxy)quinazolin-4-yl)phenyl)-2-(3-(trifluoromethyl)phenyl)acetamide**

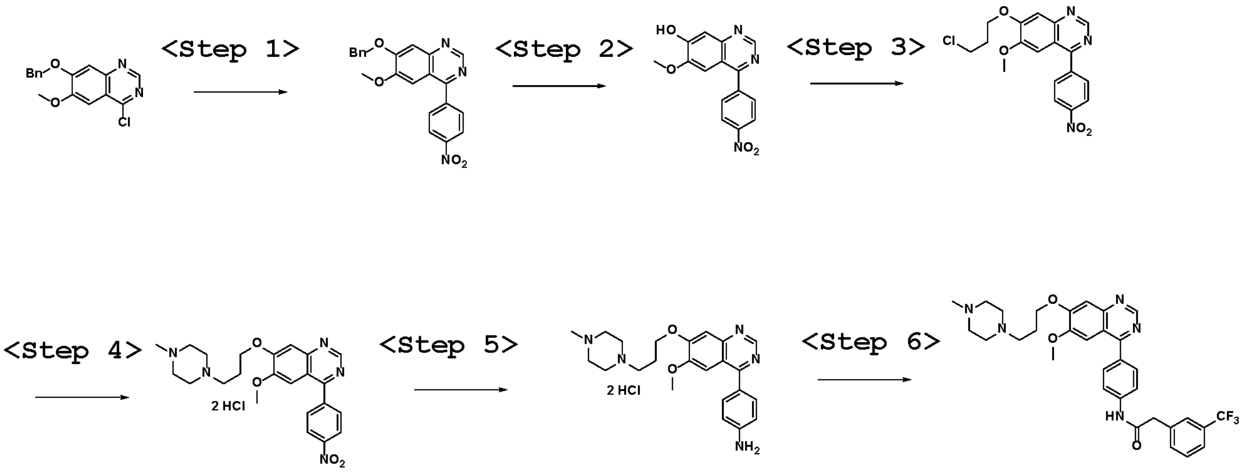


Step 1: After dissolving 7-(benzyloxy)-4-chloro-6-methoxyquinazoline (1.0g, 3.33mmol), (4-nitrophenyl)boronic

acid (0.83g, 4.99mmol) in 1,4-dioxane (12.4ml), 2M sodium carbonate (4.2ml), tetrakis(triphenylphosphine)palladium(0) (1.15g, 0.99mmol) was added dropwise, after bubbling with nitrogen, and then it was stirred overnight at 80°C. After confirming the completion of the reaction and adding distilled water, the organic matter was extracted with dichloromethane(x3). After washing the collected organic layer with brine, the remaining water was removed using sodium sulfate, and concentrated under reduced pressure. The concentrated mixture was solidified through dichloromethane, and the desired compound 7-(benzyloxy)-6-methoxy-4-(4-nitrophenyl)quinazoline (1.25g, 97 %) was obtained without further purification, and the following reaction was carried out.
MS(m/z): 388.2 $[M+H]^+$ UPLC r.t.(min): 1.76

Step 2: After dissolving 7-(benzyloxy)-6-methoxy-4-(4-nitrophenyl)quinazoline (1.27g, 3.28mmol) obtained in the step 1 in trifluoroacetic acid (5.0ml), and stirred at 75°C for 2 hours. Upon completion of the reaction, after concentration under reduced pressure, solid desired compound 6-methoxy-4-(4-nitrophenyl)quinazolin-7-ol 2,2,2-trifluoroacetic acid (0.95 g, 70%) was obtained and the following reaction was carried out without further purification.
MS(m/z): 298.1 $[M+H]^+$ UPLC r.t.(min): 1.28

Step 3: After dissolving 6-methoxy-4-(4-nitrophenyl)quinazolin-7-ol 2,2,2-trifluoroacetic acid (2.0g, 4.8 6mmol) obtained in the step 2, triphenylphosphine (2.55g, 9.73mmol) in dichloromethane (48.6ml), *N,N*-diisopropylethylamine (1.25g, 9.73mmol) was added dropwise. After adding diisopropyl azodicarboxylate (1.97g, 9.73mmol) dropwise to the reaction mixture, it was stirred overnight at room temperature. When the reaction was completed, it was concentrated under reduced pressure and purified through MPLC(EA:HEX=30-100%) to obtain the desired compound 7-(3-chloropropoxy)-6-methoxy-4-(4-nitrophenyl)quinazoline (1.82 g, 80% purity, and 100% yield).
MS(m/z): 374.1 $[M+H]^+$ UPLC r.t.(min): 1.66

Step 4: After dissolving 7-(3-chloropropoxy)-6-methoxy-4-(4-nitrophenyl)quinazoline (1.5g, 4.01mmol) obtained in the step 3 and 1-methylpiperazine (2.0g, 20.06mmol) in dimethylacetamide (20.0 ml), and it was stirred overnight at 80°C. After confirming the completion of the reaction and adding distilled water, the organic matter was extracted with ethyl acetate (x3). After washing the collected organic layer with brine, the remaining water was removed using sodium sulfate, and concentrated under reduced pressure. The concentrated mixture was dissolved in acetone (10.0 ml), acidified with 4M HCl in ether, and filtered the solid filtrate to obtain the desired compound 6-methoxy-7-(3-(4-methylpiperazin-1-yl)propoxy)-4-(4-nitrophenyl)quinazoline dihydrochloride (1.77g, 86%).
MS(m/z): 438.3 $[M+H]^+$ UPLC r.t.(min): 1.04

Step 5: After dissolving 6-methoxy-7-(3-(4-methylpiperazin-1-yl)propoxy)-4-(4-nitrophenyl)quinazoline dihydrochloride (1.77g, 3.47mmol) obtained in the step 4 in methanol (34ml), Pd/C (0.19g, 1.734mmol) was added. The reaction mixture was stirred at 40°C for 1 hour under hydrogen. The reactant was filtered through a celite filter, washed with methanol, and concentrated under reduced pressure to obtain the desired compound 4-(6-methoxy-7-(3-(4-methylpiperazin-1-yl)propoxy)quinazolin-4-yl)aniline dihydrochloride (1.6g, 50 %).
MS(m/z): 410.3 $[M+H]^+$ UPLC r.t.(min): 0.26

Step 6: After dissolving 2-(3-(trifluoromethyl)phenyl)acetic acid (6.0mg, 0.31mmol) and HATU (0.16g. 0.42mmol) in dichloromethane (0.7ml), *N,N*-diisopropylethylamine (0.11g, 0.83mmol) was added dropwise, and 4-(6-methoxy-7-(3-(4-methylpiperazin-1-yl)propoxy)quinazolin-4-yl)aniline dihydrochloride (0.1g, 0.21mmol) obtained in the step 5 was added dropwise. The mixture was stirred at room temperature overnight, distilled water was added after the reaction was completed, and the organic matter was extracted with ethyl acetate (x3). After washing the collected organic layer with brine, the remaining water was removed using sodium sulfate, and concentrated under reduced pressure. The concentrated mixture was purified through MPLC (DCM:MeOH=0-10%) to obtain the desired compound *N*-(4-(6-methoxy-7-(3-(4-methylpiperazin-1-yl)propoxy)quinazolin-4-yl)phenyl)-2-(3-(trifluoromethyl)phenyl)acetamide (3.0mg, 24 %).
MS(m/z): 594.4 $[M+H]^+$ UPLC r.t.(min): 1.25

**<Example 56> Preparation of *N*-(4-(6-methoxy-7-((1-methylpiperidin-4-yl)methoxy)quinazolin-4-yl)phenyl)-2-(4-(trifluoromethyl)phenyl)acetamide**

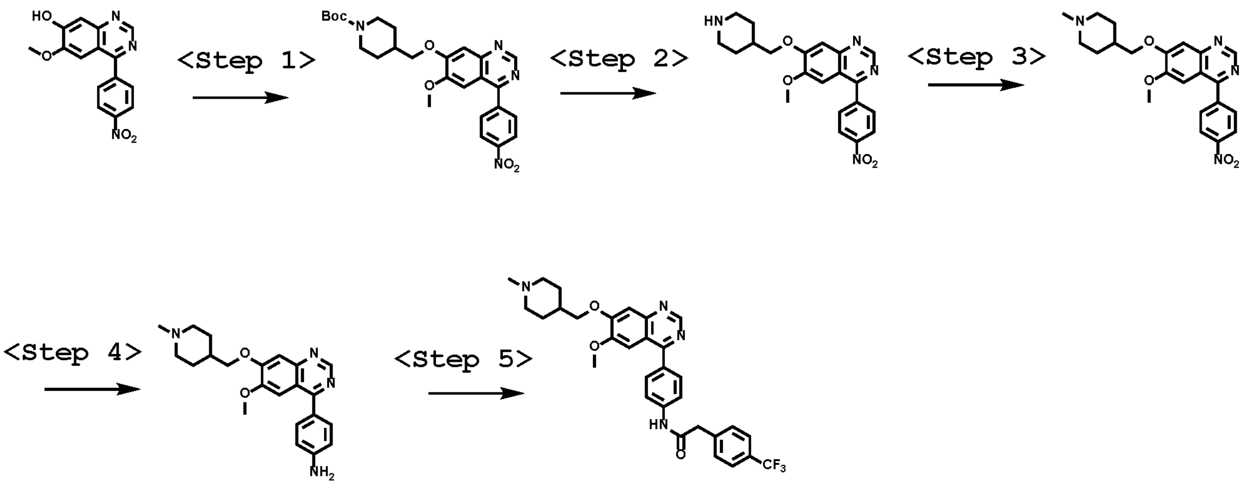


Step 1: After dissolving 6-methoxy-4-(4-nitrophenyl)quinazolin-7-ol 2,2,2-trifluoroacetic acid (5.0g, 12.16mmol) obtained in the step 2 of Example 55 and tert-butyl 4-((tosyloxy)methyl)piperidin-1-carboxylate (5.84g, 15.80mmol) in dimethylformamide (40.0ml), N,N-diisopropylethylamine (7.86g, 60.80mmol) and potassium carbonate (3.36g, 24.31mmol) was added dropwise. The reaction mixture was stirred at 70°C overnight, and when the reaction was completed, the solid filtrate was filtered with water to obtain the desired compound tert-butyl 4-(((6-methoxy-4-(4-nitrophenyl)quinazolin-7-yl)oxy)methyl)piperidin-1-carboxylate (6.20g, crude, 103 %). It was used in the following reaction without further purification.
MS(m/z): 495.3 $[M+H]^+$ UPLC r.t.(min): 1.86

Step 2: After dissolving tert-butyl 4-(((6-methoxy-4-(4-nitrophenyl)quinazolin-7-yl)oxy)methyl)piperidin-1-carboxylate (6.20g, 12.54mmol) in dichloromethane (62.0ml), 4M HCl (31.3ml, 125.00mmol) in dioxane was added dropwise. After stirring overnight at room temperature, when the reaction was completed, the solid compound was filtered to obtain the desired compound 6-methoxy-4-(4-nitrophenyl)-7-(piperidin-4-ylmethoxy)quinazolin hydrochloride (5.0g, 93%), and used in the following reaction without further purification.
MS(m/z): 395.2 $[M+H]^+$ UPLC r.t.(min): 1.17

Step 3: After dissolving 6-methoxy-4-(4-nitrophenyl)-7-(piperidin-4-ylmethoxy)quinazoline hydrochloride (0.50g, 1.16mmol) obtained in the step 2 and sodium triacetoxyborohydride (0.37g, 1.74mmol) in 1,4-dioxane (5.8ml), formaldehyde (0.94g, 11.60mmol) and *N,N*-diisopropylethylamine (0.30g, 2.32mmol) were added dropwise, and then the mixture was stirred overnight at room temperature. After completion of the reaction, distilled water was added, and the organic matter was extracted with dichloromethane (x3). After washing the collected organic layer with brine, the remaining water was removed using sodium sulfate, and concentrated under reduced pressure to obtain the desired compound 6-methoxy-7-((1-methylpiperidin-4-yl)methoxy)-4-(4-nitrophenyl)quinazoline. The concentrated mixture (0.45g, 95%) was used in the following reaction without further purification.
MS(m/z): 409.2 $[M+H]^+$ UPLC r.t.(min): 1.14

Step 4: After dissolving 6-methoxy-7-((1-methylpiperidin-4-yl)methoxy)-4-(4-nitrophenyl)quinazoline (0.45g, 1.10mmol) obtained in the step 3 in methanol (10.9ml), Pd/C (0.05g, 0.55mmol) was added. The reaction mixture was stirred at room temperature for 1 hour under hydrogen. The reactant was filtered through a celite filter, washed with methanol, and then concentrated under reduced pressure to obtain the desired compound 4-(6-methoxy-7-((1-methylpiperidin-4-yl)methoxy)quinazolin-4-yl)aniline 2,2,2-trifluoroacetic acid (0.30g, 72.3%).
MS(m/z): 397.3 $[M+H]^+$ UPLC r.t.(min): 0.84

Step 5: After dissolving 2-(4-(trifluoromethyl)phenyl)acetic acid (22.0mg, 0.11mmol) and HATU (54.0mg, 0.14mmol) in dichloromethane (0.7ml), *N,N*-diisopropylethylamine (3.7mg, 0.28mmol) was added dropwise. 4-(6-methoxy-7-((1-methylpiperidin-4-yl)methoxy)quinazolin-4-yl)aniline 2,2,2-trifluoroacetic acid (35.0mg, 0.07mmol) obtained in the step 4 into the reaction mixture, and the mixture was stirred at room temperature overnight. After the completion of the reaction, distilled water was added, and then the organic matter was extracted with ethyl acetate (x3). After washing the collected organic layer with brine, the remaining water was removed using sodium sulfate, and concentrated under reduced pressure. After separating and purifying the concentrated mixture MPLC (DCM:MeOH=0-20%), the desired compound N-(4-(6-methoxy-7-((1-methylpiperidin-4-yl)methoxy)quinazolin-4-yl)phenyl)-2-(4-(trifluoromethyl)phenyl)acetamide (20.0mg, 49.8%) was obtained.
MS(m/z): 565.4 $[M+H]^+$ UPLC r.t.(min): 1.34

## Examples 55 to 121

Examples 57 to 121 were prepared in a similar manner to Examples 55 and 56, and the compound names, NMR, mass, and UPLC analysis results of Examples 55 to 121 are summarized in Table 2 below.

[Table 2]

| Example | Structure | Compound Name | $^1$H NMR; MS(m/z) | UPLC r.t. (min) |
|---|---|---|---|---|
| 55 | 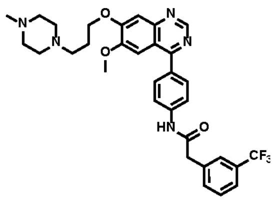 | *N*-(4-(6-methoxy-7-(3-(4-methylpiperazin-1-yl) propoxy)quinazoli n-4-yl)phenyl)-2-(3-(trifluoromethyl) phe nyl)acetamide | 594.3 [M+H]$^+$ | 1.25 |
| 56 | 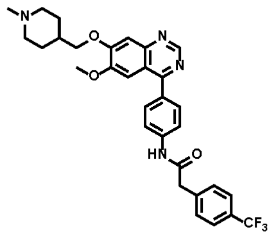 | *N*-(4-(6-methoxy-7-((1-methylpiperidin-4-yl) methoxy)quinazoli n-4-yl)phenyl)-2-(4-(trifluoromethyl) phe nyl) acetamide | $^1$H NMR (400 MHz, MeOD-$d_4$): δ 8.98 (s, 1H), 7.85 (d, *J* = 8.4 Hz, 2H), 7.76 - 7.71 (m, 2H), 7.66 (d, *J* = 8.0 Hz, 2H), 7.58 (d, *J* = 8.1 Hz, 2H), 7.33 - 7.27 (m, 2H), 4.14 - 4.06 (m, 2H), 3.88 - 3.81 (m, 5H), 3.53 - 3.45 (m, 2H), 3.03 - 2.92 (m, 2H), 2.79 (s, 3H), 2.26 - 2.08 (m, 3H), 1.83 - 1.69 (m, 2H)<br>565.4 [M+H]$^+$ | 1.34 |
| 57 | 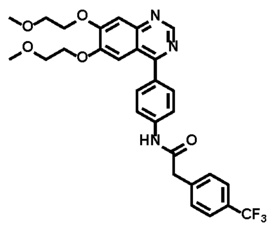 | *N*-(4-(6,7-bis(2-methoxyethoxy)quinaz olin-4-yl)phenyl)-2-(4-(trifluoromethyl) phe nyl)acetamide | 556.3 [M+H]$^+$ | 1.59 |
| 58 | 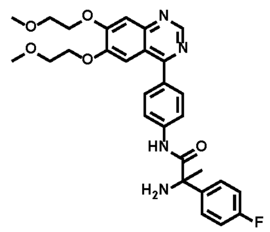 | 2-amino-N-(4-(6,7-bis (2-methoxyethoxy) quinaz olin-4-yl)phenyl)-2-(4-fluorophenyl) propana mide | 535.3 [M+H]$^+$ | 1.14 |
| 59 | 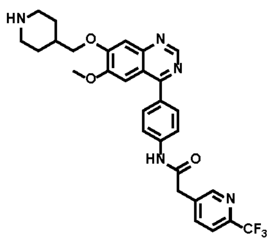 | *N*-(4-(6-methoxy-7-(piperidin-4-ylmethoxy)quinazolin -4-yl)phenyl)-2-(6-(trifluoromethyl)pyr idin-3-yl) acetamide | $^1$H NMR (400 MHz, DMSO-$d_6$): δ 10.61 (s, 1H), 9.08 (s, 1H), 8.75 (s, 1H), 8.07 (d, *J* = 8.3 Hz, 1H), 7.91 (d, *J* = 8.0 Hz, 1H), 7.83 (s, 4H), 7.38 (d, *J* = 24.9 Hz, 2H), 4.07 (d, J = 6.4 Hz, 2H), 3.94 (s, 2H), 3.85 (s, 3H), 2.61 (s, 2H), 1.99 (s, 1H), 1.79 (d, *J* = 10.9 Hz, 2H), 1.26 (d, *J* = 14.7 Hz, 2H).<br>552.3 [M+H]$^+$ | 1.17 |

(continued)

| Example | Structure | Compound Name | $^1$H NMR; MS(m/z) | UPLC r.t. (min) |
|---|---|---|---|---|
| 60 | 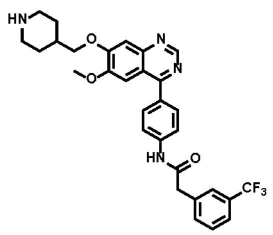 | N-(4-(6-methoxy-7-(piperidin-4-ylmethoxy)quinazolin -4-yl)phenyl)-2-(3-(trifluoromethyl) phe nyl) acetamide | $^1$H NMR (400 MHz, DMSO-$d_6$): δ 10.54 (s, 1H), 9.10 (s, 1H), 8.58 (s, 1H), 8.28 (s, 1H), 7.84 (s, 4H), 7.74 (s, 1H), 7.63 (dq, *J* = 15.0, 7.9 Hz, 3H), 7.52-7.41 (m, 1H), 7.37 (s, 1H), 4.15 (d, *J* = 6.3 Hz, 2H), 3.85 (d, *J* = 3.6 Hz, 5H), 2.96 (s, 2H), 2.20 (s, 1H), 1.98 (d, J = 13.8 Hz, 2H), 1.52 (d, *J* = 12.9 Hz, 2H).<br>551.3 [M+H]$^+$ | 1.30 |
| 61 | 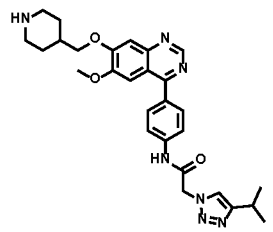 | 2-(4-isopropyl-1*H*-1,2,3-triazole-1-yl)-N-(4-(6-methoxy-7-(piperidin-4-ylmethoxy)quinazolin -4-yl)phenyl) acetamide | $^1$H NMR (400 MHz, DMSO-$d_6$): δ 10.78 (s, 1H), 9.11 (s, 1H), 8.60 (d, *J* = 11.1 Hz, 1H), 8.29 (d, J = 12.0 Hz, 1H), 7.90 (s, 1H), 7.85 (s, 4H), 7.47 (s, 1H), 7.37 (s, 1H), 4.15 (d, *J* = 6.3 Hz, 2H), 3.86 (s, 3H), 3.34 (d, *J* = 12.4 Hz, 2H), 3.00 (ddd, *J* = 21.2, 13.9, 8.6 Hz, 3H), 2.20 (t, *J* = 8.6 Hz, 1H), 1.98 (d, *J* = 14.0 Hz, 2H), 1.59-1.46 (m, 2H), 1.26 (d, *J* = 6.9 Hz, 6H).<br>516.4 [M+H]$^+$ | 1.06 |
| 62 | 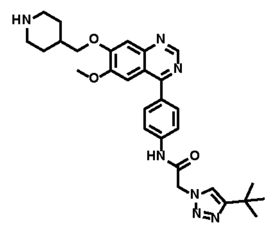 | 2-(4-(tert-butyl)-1H-1,2,3-triazole-1-yl)-N-(4-(6-methoxy-7-(piperidin-4-ylmethoxy) quinazolin -4-yl)phenyl) acetamide | $^1$H NMR (400 MHz, DMSO-$d_6$): δ 10.75 (s, 1H), 9.11 (d, *J* = 2.8 Hz, 1H), 8.53 (s, 1H), 8.25 (s, 1H), 7.87 (d, *J* = 21.5 Hz, 5H), 7.42 (d, *J* = 39.1 Hz, 2H), 5.33 (s, 2H), 4.15 (d, *J* = 6.4 Hz, 2H), 3.86 (s, 3H), 2.94 (d, *J* = 14.0 Hz, 2H), 2.21 (s, 1H), 1.98 (d, *J* = 13.9 Hz, 2H), 1.91 (d, *J* = 3.1 Hz, 1H), 1.58-1.46 (m, 2H), 1. 34-1. 27 (m, 9H).<br>530.4 [M+H]$^+$ | 1.12 |
| 63 | 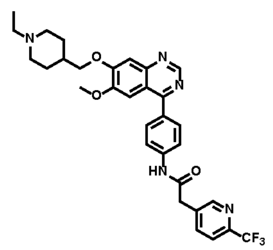 | N-(4-(7-((1-ethylpiperidin-4-yl) methoxy)-6-methoxyquinazolin-4-yl)phenyl)-2-(6-(trifluoromethyl)pyr idin-3-yl) acetamide | $^1$H NMR (400 MHz, DMSO-$d_6$): δ 10.58 (s, 1H), 9.08 (s, 1H), 8.75 (d, *J* = 2.0 Hz, 1H), 8.07 (dd, *J* = 8.0, 2.1 Hz, 1H), 7.91 (dd, *J* = 8.0, 0.9 Hz, 1H), 7.83 (s, 4H), 7.40 (s, 1H), 7.35 (s, 1H), 4.08 (d, *J* = 6.1 Hz, 2H), 3.94 (s, 2H), 3.85 (s, 3H), 2.99-2.85 (m, 2H), 1.88 (s, 2H), 1.79 (d, *J* = 13.3 Hz, 2H), 1.35 (q, *J* = 12.0 Hz, 2H), 1.24 (d, *J* = 4.0 Hz, 1H), 1.01 (t, *J* = 7.2 Hz, 3H).<br>580.3 [M+H]$^+$ | 1.19 |

(continued)

| Example | Structure | Compound Name | $^{1}H$ NMR; MS(m/z) | UPLC r.t. (min) |
|---|---|---|---|---|
| 64 | 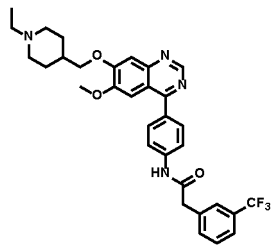 | N-(4-(7-((1-ethylpiperidin-4-yl) methoxy)-6-methoxyquinazolin-4-yl)phenyl)-2-(3-(trifluoromethyl) phe nyl)acetamide | 579.3 $[M+H]^+$ | 1.30 |
| 65 | 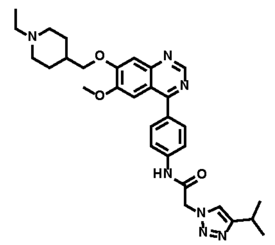 | N-(4-(7-((1-ethylpiperidin-4-yl) methoxy)-6-methoxyquinazolin-4-yl)phenyl)-2-(4-isopropyl-1H-1,2,3-triazole-1-yl)acetamide | $^{1}H$ NMR (400 MHz, DMSO-$d_6$): δ 10.75 (s, 1H), 9.09 (s, 1H), 7.94-7.76 (m, 5H), 7.38 (d, *J* = 22.6 Hz, 2H), 5.33 (s, 2H), 4.08 (d, J = 6.1 Hz, 2H), 3.85 (s, 3H), 2.90 (d, *J* = 10.7 Hz, 2H), 2.32 (q, *J* = 7.0 Hz, 2H), 2.09 (d, J = 2.8 Hz, 2H), 1.26-1.23 (m, 6H), 1.00 (t, *J* = 7.0 Hz, 4H), 0.91-0.82 (m, 4H).<br>544.4 $[M+H]^+$ | 1.07<br>1.13 |
| 66 | 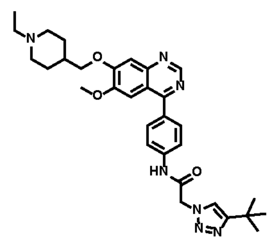 | 2-(4-(tert-butyl)-1H-1,2,3-triazole-1-yl)-*N*-(4-(7-((1-ethylpiperidin-4-yl) methoxy)-6-methoxyquinazolin-4-yl)phenyl)acetamide | $^{1}H$ NMR (400 MHz, DMSO-$d_6$): δ 10.75 (s, 1H), 9.08 (s, 1H), 7.96-7.73 (m, 5H), 7.38 (d, *J* = 21.8 Hz, 2H), 5.32 (s, 2H), 4.08 (d, *J* = 7.1 Hz, 2H), 3.84 (d, *J* = 10.5 Hz, 3H), 2.89 (s, 2H), 2.31 (s, 4H), 1.89 (d, *J* = 9.6 Hz, 4H), 1.30 (d, *J* = 6.4 Hz, 9H), 1.00 (t, *J* = 7.6 Hz, 4H).<br>558.4 $[M+H]^+$ | |
| 67 | 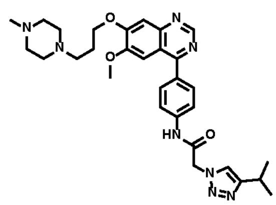 | 2-(4-isopropyl-1H-1,2,3-triazole-1-yl)-N-(4-(6-methoxy-7-(3-(4-methylpiperazin-1-yl) propoxy)quinazoli n-4-yl)phenyl)acetamide | 559.3 $[M+H]^+$ | 1.02 |
| 68 | 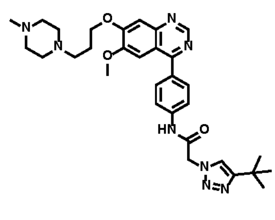 | 2-(4-(tert-butyl)-1H-1,2,3-triazole-1-yl)-N-(4-(6-methoxy-7-(3-(4-methylpiperazin-1-yl) propoxy)quinazoli n-4-yl)phenyl)acetamide | 573.4 $[M+H]^+$ | 1.08 |
| 69 | 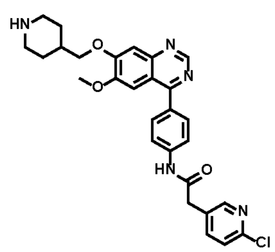 | 2-(6-chloropyridin-3-yl)-N-(4-(6-methoxy-7-(piperidin-4-ylmethoxy)quinazolin -4-yl) phenyl) acetamide | $^{1}H$ NMR (400 MHz, DMSO-$d_6$): δ 10.19 (s, 1H), 9.07 (s, 1H), 7.80 (d, *J* = 7.5 Hz, 4H), 7.44-7.03 (m, 9H), 3.99 (s, 2H), 3.85 (s, 3H), 2.71 (d, J = 7.2 Hz, 2H), 1.24 (s, 2H), 0.85 (d, J = 7.7 Hz, 2H) .<br>518.3 $[M+H]^+$ | 1.10 |

(continued)

| Example | Structure | Compound Name | $^1$H NMR; MS(m/z) | UPLC r.t. (min) |
|---|---|---|---|---|
| 70 | 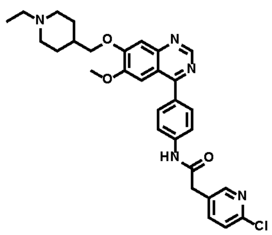 | 2-(6-chloropyridin-3-yl)-N-(4-(7-((1-ethylpiperidin-4-yl) methoxy)-6-methoxyquinazolin-4-yl)phenyl) acetamide | $^1$H NMR (400 MHz, DMSO-$d_6$): δ 10.54 (s, 1H), 9.18-8.96 (m, 2H), 8.39 (s, 1H), 7.89-7.78 (m, 5H), 7.56-7.44 (m, 2H), 7.38 (s, 1H), 4.16 (d, *J* = 6.0 Hz, 2H), 3.83 (d, J = 19.5 Hz, 5H), 3.55 (d, J = 11.8 Hz, 2H), 3.14-3.06 (m, 2H), 2.95 (d, J = 11.6 Hz, 2H), 2.05 (d, J = 14.0 Hz, 2H), 1.57 (d, J = 13.6 Hz, 2H), 1.24 (d, J = 6.6 Hz, 3H), 0.86 (t, J = 6.7 Hz, 1H). 546.3 $[M+H]^+$ | 1.13 |
| 71 | 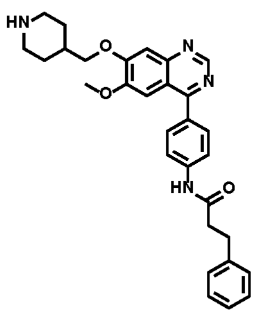 | *N*-(4-(6-methoxy-7-(piperidin-4-ylmethoxy)quinazolin-4-yl)phenyl)-3-phenylpropanamide | $^1$H NMR (400 MHz, DMSO-$d_6$): δ 10.19 (s, 1H), 9.07 (s, 1H), 7.80 (d, *J* = 7.5 Hz, 4H), 7.46-7.03 (m, 9H), 3.99 (s, 2H), 3.85 (s, 3H), 2.71 (d, *J* = 7.2 Hz, 2H), 2.08 (d, *J* = 6.8 Hz, 4H), 1.24 (s, 2H), 0.85 (d, *J* = 7.7 Hz, 2H) . 497.3 $[M+H]^+$ | 1.22 |
| 72 | 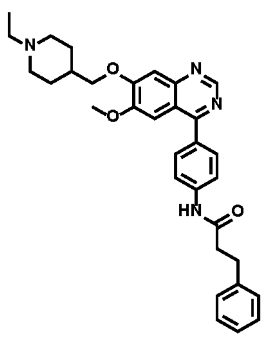 | N-(4-(7-((1-ethylpiperidin-4-yl) methoxy)-6-methoxyquinazolin-4-yl)phenyl)-3-phenylpropanamide | $^1$H NMR (400 MHz, DMSO-$d_6$): δ 10.20 (s, 1H), 9.09 (s, 1H), 7.82 (s, 4H), 7.51-7.15 (m, 7H), 4.14 (d, *J* = 6.2 Hz, 2H), 3.86 (s, 3H), 2.96 (t, *J* = 7.4 Hz, 2H), 2.82-2.65 (m, 4H), 2.14-1.88 (m, 5H), 1.60-1.44 (m, 2H), 1.27 (d, *J* = 17.5 Hz, 1H), 1.14 (t, *J* = 7.2 Hz, 3H), 0.90 - 0.78 (m, 1H). 525.4 $[M+H]^+$ | 1.25 |
| 73 | 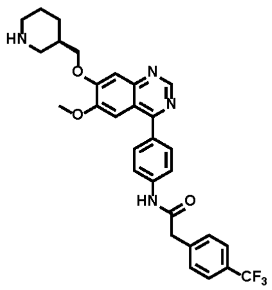 | (R)-N-(4-(6-methoxy-7-(piperidin-3-ylmethoxy)quinazolin-4-yl)phenyl)-2-(4-(trifluoromethyl) phe nyl) acetamide | $^1$H NMR (400 MHz, DMSO-$d_6$): δ 10.55 (s, 1H), 9.09 (d, *J* = 1.5 Hz, 1H), 7.84 (d, *J* = 1.9 Hz, 3H), 7.72 (d, *J* = 8.1 Hz, 2H), 7.60 (d, *J* = 8.0 Hz, 2H), 7.42 (s, 1H), 7.36 (s, 1H), 4.11 (d, *J* = 8.3 Hz, 2H), 3.85 (d, *J* = 2.4 Hz, 3H), 2.09 (s, 4H), 1.90 (s, 4H), 1.70 (s, 1H), 1.50 (s, 1H), 1.27 (d, *J* = 19.0 Hz, 2H). 551.3 $[M+H]^+$ | 1.32 |

(continued)

| Example | Structure | Compound Name | $^1$H NMR; MS(m/z) | UPLC r.t. (min) |
|---|---|---|---|---|
| 74 | 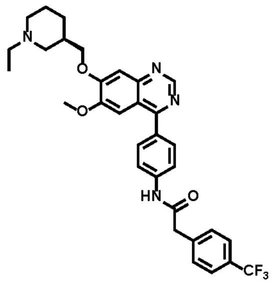 | (*R*)-*N*-(4-(7-((1-ethylpiperidin-3-yl) methoxy)-6-methoxyquinazolin-4-yl)phenyl)-2-(4-(trifluoromethyl) phe nyl)acetamide | $^1$H NMR (400 MHz, DMSO-$d_6$): δ 10.54 (s, 1H), 9.08 (s, 1H), 7.83 (d, *J* = 1.4 Hz, 4H), 7.72 (d, *J* = 8.1 Hz, 2H), 7.60 (d, *J* = 8.0 Hz, 2H), 7.41 (s, 1H), 7.36 (s, 1H), 4.11 (d, *J* = 6.5 Hz, 2H), 3.85 (d, *J* = 2.7 Hz, 5H), 2.98 (s, 1H), 2.81 (s, 1H), 2.41 (s, 1H), 2.01 (d, *J* = 81.3 Hz, 3H), 1.87-1.78 (m, 1H), 1.70 (d, *J* = 12.9 Hz, 1H), 1.52 (d, *J* = 12.8 Hz, 1H), 1.29-1.11 (m, 2H), 1.02 (t, *J* = 7.1 Hz, 3H).<br>579.3 [M+H]$^+$ | 1.34 |
| 75 | 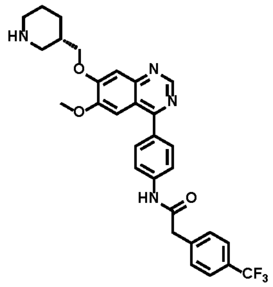 | (*S*)-*N*-(4-(6-methoxy-7-(piperidin-3-ylmethoxy)quinazolin -4-yl)phenyl)-2-(4-(trifluoromethyl) phe nyl)acetamide | $^1$H NMR (400 MHz, DMSO-$d_6$): δ 10.58 (s, 1H), 9.11 (s, 1H), 8.70 (d, *J* = 11.4 Hz, 1H), 8.45 (d, *J* = 11.3 Hz, 1H), 7.84 (d, *J* = 3.2 Hz, 3H), 7.73 (d, *J* = 8.1 Hz, 2H), 7.67-7.53 (m, 5H), 7.46 (s, 1H), 7.38 (s, 1H), 4.28-4.12 (m, 2H), 3.86 (s, 3H), 2.89-2.79 (m, 2H), 2.41-2.28 (m, 1H), 1.96-1.82 (m, 2H), 1.67 (dt, *J* = 13.3, 3.6 Hz, 1H), 1.40 (dd, *J* = 12.1, 3.3 Hz, 1H).<br>551.3 [M+H]$^+$ | 1.31 |
| 76 | 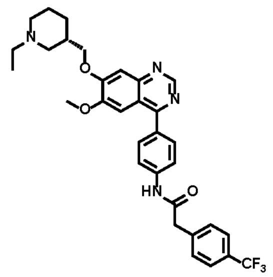 | (*S*)-*N*-(4-(7-((1-ethylpiperidin-3-yl) methoxy)-6-methoxyquinazolin-4-yl)phenyl)-2-(4-(trifluoromethyl) phe nyl)acetamide | $^1$H NMR (400 MHz, DMSO-$d_6$): δ 10.53 (s, 1H), 9.08 (d, *J* = 2.8 Hz, 1H), 7.83 (t, *J* = 2.3 Hz, 4H), 7.72 (d, *J* = 8.3 Hz, 2H), 7.60 (d, *J* = 7.9 Hz, 2H), 7.41 (s, 1H), 7.36 (s, 1H), 4.11 (d, *J* = 6.4 Hz, 2H), 3.85 (d, *J* = 2.8 Hz, 5H), 2.97 (s, 1H), 2.79 (s, 1H), 2.39 (s, 1H), 2.01 (d, *J* = 80.5 Hz, 3H), 1.81 (d, *J* = 12.1 Hz, 1H), 1.69 (s, 1H), 1.51 (s, 1H), 1.28-1.05 (m, 2H), 1.02 (t, *J* = 7.2 Hz, 3H).<br>579.3 [M+H]$^+$ | 1.32 |
| 77 | 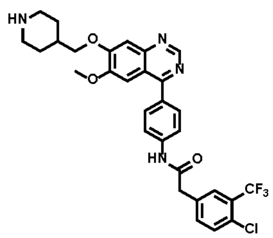 | 2-(4-chloro-3-(trifluoromethyl)phe nyl)-*N*-(4-(6-methoxy-7-(piperidin-4-ylmethoxy)quinazolin -4-yl)phenyl)acetamide | $^1$H NMR (400 MHz, DMSO-$d_6$): δ 10.51 (s, 1H), 9.08 (s, 1H), 7.85 (d, *J* = 17.2 Hz, 5H), 7.69 (q, J = 8.2 Hz, 2H), 7.43 (s, 1H), 7.36 (s, 1H), 4.10 (d, *J* = 6.2 Hz, 2H), 3.86 (d, *J* = 7.2 Hz, 4H), 2.71 (t, *J* = 12.3 Hz, 2H), 2.07 (d, *J* = 11.2 Hz, 2H), 1.89 (s, 4H), 1.36 (d, *J* = 12.5 Hz, 2H), 1.26 (d, *J* = 13.5 Hz, 1H).<br>585.3 [M+H]$^+$ | 1.37 |

(continued)

| Example | Structure | Compound Name | $^1$H NMR; MS(m/z) | UPLC r.t. (min) |
|---|---|---|---|---|
| 78 | 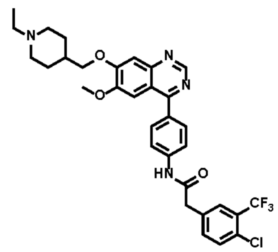 | 2-(4-chloro-3-(trifluoromethyl)phenyl)-*N*-(4-(7-((1-ethylpiperidin-4-yl) methoxy)-6-methoxyquinazolin-4-yl)phenyl)acetamide | $^1$H NMR (400 MHz, DMSO-$d_6$): δ 10.53 (s, 1H), 9.06 (d, *J* = 31.8 Hz, 2H), 7.85 (d, *J* = 14.6 Hz, 5H), 7.70 (q, *J* = 7.5 Hz, 2H), 7.47 (d, *J* = 6.4 Hz, 1H), 7.38 (s, 1H), 4.16 (d, *J* = 6.2 Hz, 2H), 3.86 (d, *J* = 5.9 Hz, 5H), 3.10 (s, 2H), 2.95 (d, *J* = 12.0 Hz, 2H), 2.13-2.03 (m, 2H), 1.92 (d, *J* = 5.5 Hz, 2H), 1.57 (d, *J* = 11.8 Hz, 2H), 1.23 (t, *J* = 6.3 Hz, 3H). 613.3 [M+H]$^+$ | 1.41 |
| 79 | 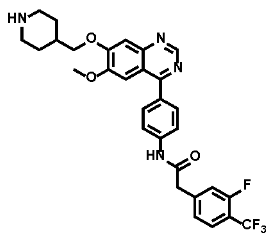 | 2-(3-fluoro-4-(trifluoromethyl)phenyl)-*N*-(4-(6-methoxy-7-(piperidin-4-ylmethoxy) quinazolin -4-yl)phenyl)acetamide | $^1$H NMR (400 MHz, DMSO-$d_6$): δ 10.54 (s, 1H), 9.09 (s, 1H), 7.89-7.75 (m, 5H), 7.51 (d, *J* = 12.1 Hz, 1H), 7.47-7.35 (m, 3H), 4.12 (d, *J* = 6.2 Hz, 2H), 3.87 (d, *J* = 13.2 Hz, 5H), 2.82 (d, *J* = 12.7 Hz, 2H), 2.21-2.03 (m, 2H), 1.95-1.87 (m, 4H), 1. 54-1. 39 (m, 2H). 569.3 [M+H]$^+$ | 1.34 |
| 80 | 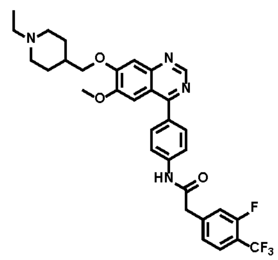 | *N*-(4-(7-((1-ethylpiperidin-4-yl) methoxy)-6-methoxyquinazolin-4-yl)phenyl)-2-(3-fluoro-4-(trifluoromethyl)phenyl) acetamide | $^1$H NMR (400 MHz, DMSO-$d_6$): δ 10.55 (s, 1H), 9.09 (s, 1H), 7.84 (s, 4H), 7.78 (t, *J* = 8.0 Hz, 1H), 7.52 (d, *J* = 11.9 Hz, 1H), 7.42 (d, *J* = 6.0 Hz, 2H), 7.36 (s, 1H), 4.10 (d, *J* = 6.2 Hz, 2H), 3.89 (s, 2H), 3.85 (s, 3H), 3.05-2.92 (m, 2H), 2.43 (s, 2H), 2.03 (s, 2H), 1. 92-1. 77 (m, 3H), 1.39 (q, *J* = 11.1 Hz, 2H), 1.03 (t, *J* = 7.1 Hz, 3H).<br>597.3 [M+H]$^+$ | 1.37 |
| 81 | 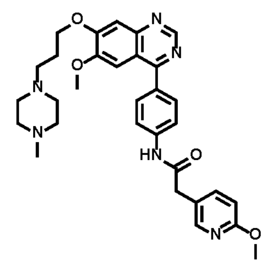 | *N*-(4-(6-methoxy-7-(3-(4-methylpiperazin-1-yl) propoxy)quinazoli n-4-yl)phenyl)-2-(6-methoxypyridin-3-yl) acetamide | 557.3 [M+H]$^+$ | 1.02 |
| 82 | 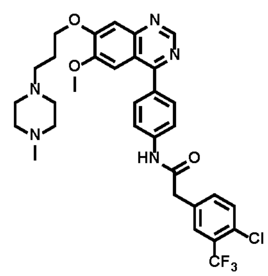 | 2-(4-chloro-3-(trifluoromethyl)phenyl)-N-(4-(6-methoxy-7-(3-(4-methylpiperazin-1-yl) propoxy)quinazoli n-4-yl)phenyl)acetamide | $^1$H NMR (400 MHz, MeOD-$d_4$): δ 9.00 (s, 1H), 7.87 - 7.79 (m, 3H), 7.77 - 7.72 (m, 2H), 7.64 - 7.57 (m, 2H), 7.34 (s, 2H), 4.29 (t, *J* = 6.0 Hz, 2H), 3.90 - 3.78 (m, 5H), 3.04 - 2.89 (m, 4H), 2.87 - 2.68 (m, 6H), 2.65 - 2.57 (m, 3H), 2.20 - 2.08 (m, 2H).<br>628.4 [M+H]$^+$ | 1.34 |

(continued)

| Example | Structure | Compound Name | $^1$H NMR; MS(m/z) | UPLC r.t. (min) |
|---|---|---|---|---|
| 83 | 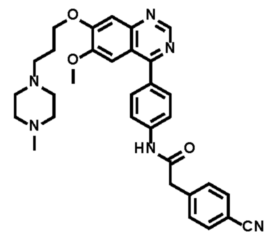 | 2-(4-cyanophenyl)-N-(4-(6-methoxy-7-(3-(4-methylpiperazin-1-yl) propoxy)quinazoli n-4-yl)phenyl) acetamide | $^1$H NMR (400 MHz, $CDCl_3$) δ 9.14 (s, 1H), 7.75-7.73 (m, 2H), 7.69-7.66 (m, 5H), 7.49-7.47 (m, 2H), 7.37 (s, 1H), 7.27 (d, *J* = 10Hz, 1H), 4.29-4.26 (m, 2H), 3.85-3.81 (m, 5H), 2.58-2.47 (m, 10H), 2.28 (s, 3H), 2.15-2.10 (m, 2H). 551.3 $[M+H]^+$ | 1.07 |
| 84 | 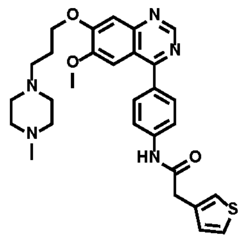 | *N*-(4-(6-methoxy-7-(3-(4-methylpiperazin-1-yl) propoxy)quinazoli n-4-yl)phenyl)-2-(thiophene-3-yl) acetamide | $^1$H NMR (400 MHz, $CDCl_3$) δ 9.15 (s, 1H), 7.78-7.72 (m, 2H), 7.69-7.63 (m, 2H), 7.46-7.42 (m, 1H), 7.38 (s, 1H), 7.36 (s, 1H), 7.31 (s, 1H), 7.30-7.28 (m, 1H), 7.12 (dd, J = 4.9, 1.4 Hz, 1H), 4.29 (t, *J* = 6.6 Hz, 2H), 3.87 (s, 3H), 3.84 (s, 2H), 2.84-2.37 (m, 10H), 2.31 (s, 3H), 2.19-2.07 (m, 2H). 532.3 $[M+H]^+$ | 1.08 |
| 85 | 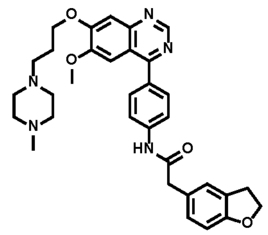 | 2-(2,3-dihydrobenzofuran-5-yl)-N-(4-(6-methoxy-7-(3-(4-methylpiperazin-1-yl) propoxy)quinazoli n-4-yl)phenyl)acetamide | $^1$H NMR (400 MHz, $CDCl_3$) δ 9.15 (s, 1H), 7.74 (d, *J* = 8.7 Hz, 2H), 7.66 (d, *J* = 8.6 Hz, 2H), 7.38 (s, 1H), 7.31 (s, 1H), 7.28 (s, 1H), 7.20 (s, 1H), 7.09 (dd, *J* = 8.1, 2.0 Hz, 1H), 6.83 (d, *J* = 8.1 Hz, 1H), 4.62 (t, *J* = 8.7 Hz, 2H), 4.29 (t, *J* = 6.6 Hz, 2H), 3.87 (s, 3H), 3.72 (s, 2H), 3.25 (t, *J* = 8.7 Hz, 2H), 2.81-2.44 (m, 10H), 2.38 (s, 3H), 2.13 (p, *J* = 6.6 Hz, 2H). 568.4 $[M+H]^+$ | 1.11 |
| 86 | 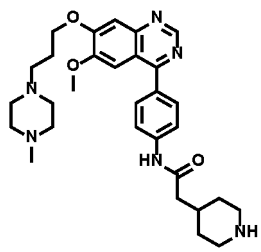 | *N*-(4-(6-methoxy-7-(3-(4-methylpiperazin-1-yl) propoxy)quinazoli n-4-yl)phenyl)-2-(piperidin-4-yl)acetamide | 533.4 $[M+H]^+$ | 0.80 |
| 87 | 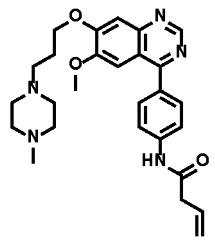 | *N*-(4-(6-methoxy-7-(3-(4-methylpiperazin-1-yl) propoxy)quinazoli n-4-yl)phenyl)but-3-enamide | 476.3 $[M+H]^+$ | 0.98 |

(continued)

| Example | Structure | Compound Name | $^1$H NMR; MS(m/z) | UPLC r.t. (min) |
|---|---|---|---|---|
| 88 | 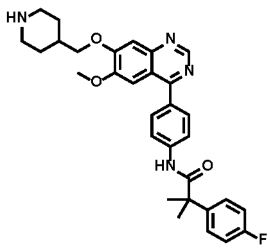 | 2-(4-fluorophenyl)-*N*-(4-(6-methoxy-7-(piperidin-4-ylmethoxy)quinazolin-4-yl)phenyl)-2-methylpropanamide | 529.4 $[M+H]^+$ | 1.34 |
| 89 | 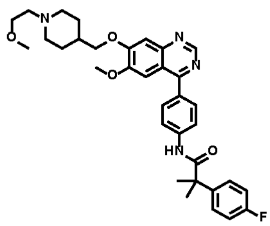 | 2-(4-fluorophenyl)-*N*-(4-(6-methoxy-7-((1-(2-methoxyethyl)piperid in-4-yl)methoxy)quinazolin-4-yl)phenyl)-2-methylpropanamide | $^1$H NMR (400 MHz, DMSO-$d_6$): δ 9.37 (s, 1H), 9.07 (s, 1H), 7.90-7.75 (m, 4H), 7.46-7.39 (m, 3H), 7.36 (s, 1H), 7.24-7.14 (m, 2H), 4.08 (d, *J* = 6.1 Hz, 2H), 3.85 (s, 3H), 3.43 (t, *J* = 5.9 Hz, 2H), 3.23 (s, 3H), 2.91 (d, *J* = 10.9 Hz, 2H), 2.46 (s, 2H), 1.98 (s, 1H), 1.80 (dd, *J* = 28.1, 11.3 Hz, 3H), 1.60 (s, 6H), 1.37 (t, *J* = 11.2 Hz, 2H) .<br>587.4 $[M+H]^+$ | 1.35 |
| 90 | 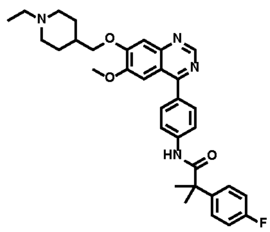 | *N*-(4-(7-((1-ethylpiperidin-4-yl) methoxy)-6-methoxyquinazolin-4-yl)phenyl)-2-(4-fluorophenyl) -2-methylpropanamide | $^1$H NMR (400 MHz, DMSO-$d_6$): δ 9.37 (s, 1H), 9.07 (s, 1H), 7.90-7.75 (m, 4H), 7.46-7.39 (m, 3H), 7.36 (s, 1H), 7.24-7.14 (m, 2H), 4.08 (d, J = 6.1 Hz, 2H), 3.85 (s, 3H), 3.43 (t, J = 5.9 Hz, 2H), 3.23 (s, 3H), 2.91 (d, *J* = 10.9 Hz, 2H), 2.46 (s, 2H), 1.98 (s, 1H), 1.80 (dd, *J* = 28.1, 11.3 Hz, 3H), 1.60 (s, 6H), 1.37 (t, *J* = 11.2 Hz, 2H).<br>557.4 $[M+H]^+$ | 1.36 |
| 91 | 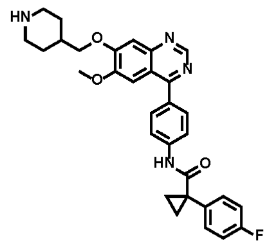 | 1-(4-fluorophenyl)-*N*-(4-(6-methoxy-7-(piperidin-4-ylmethoxy)quinazolin-4-yl)phenyl)cyclopropane-1-carboxamide | $^1$H NMR (400 MHz, DMSO-$d_6$): δ 9.27 (s, 1H), 9.07 (s, 1H), 7.78 (d, *J* = 8.5 Hz, 4H), 7.56-7.46 (m, 2H), 7.37 (d, *J* = 26.0 Hz, 2H), 7.20 (t, *J* = 8.7 Hz, 2H), 4.07 (d, *J* = 6.6 Hz, 2H), 3.84 (s, 3H), 3.06 (s, 2H), 2.60 (s, 2H), 2.08 (s, 1H), 1.78 (d, *J* = 12.4 Hz, 2H), 1.52-1.47 (m, 2H), 1.32-1.22 (m, 3H), 1.14 (dt, *J* = 8.9, 4.3 Hz, 2H).<br>527.3 $[M+H]^+$ | 1.32 |

(continued)

| Example | Structure | Compound Name | $^1$H NMR; MS(m/z) | UPLC r.t. (min) |
|---|---|---|---|---|
| 92 | 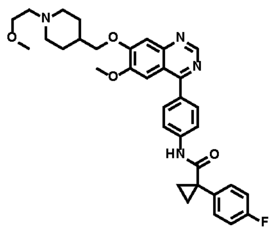 | 1-(4-fluorophenyl)-*N*-(4-(6-methoxy-7-((1-(2-methoxyethyl)piperid in-4-yl)methoxy)quinazoli n-4-yl)phenyl) cyclopropa ne-1-carboxamide | $^1$H NMR (400 MHz, DMSO-$d_6$): δ 9.29 (s, 1H), 9.07 (s, 1H), 7.83-7.76 (m, 4H), 7.52-7.45 (m, 2H), 7.39 (s, 1H), 7.34 (s, 1H), 7.24-7.17 (m, 2H), 4.08 (d, *J* = 6.1 Hz, 2H), 3.84 (s, 3H), 3.43 (t, *J* = 5.9 Hz, 2H), 3.23 (s, 3H), 2.91 (d, *J* = 10.9 Hz, 2H), 2.46 (s, 2H), 1.97 (d, *J* = 13.6 Hz, 2H), 1.78 (t, *J* = 16.1 Hz, 3H), 1.50 (q, *J* = 4.1 Hz, 2H), 1.35 (q, *J* = 11.7 Hz, 2H), 1.18-1.10 (m, 2H). 585.4 [M+H]$^+$ | 1.36 |
| 93 | 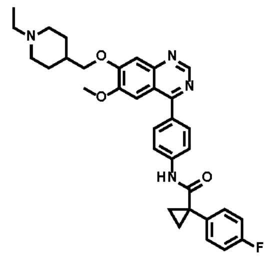 | *N*-(4-(7-((1-ethylpiperidin-4-yl) methoxy)-6-methoxyquinazolin-4-yl)phenyl)-1-(4-fluorophenyl)cyclopr opane-1-carboxamide | $^1$H NMR (400 MHz, DMSO-$d_6$): δ 9.28 (s, 1H), 9.07 (s, 1H), 7.79 (s, 4H), 7.54-7.44 (m, 2H), 7.40 (s, 1H), 7.34 (s, 1H), 7.20 (t, *J* = 8.3 Hz, 2H), 4.09 (d, *J* = 6.2 Hz, 2H), 3.84 (s, 3H), 2.95 (d, *J* = 10.9 Hz, 2H), 2.38 (s, 2H), 1.98 (s, 2H), 1.80 (d, *J* = 13.4 Hz, 2H), 1.49 (d, *J* = 5.6 Hz, 2H), 1.37 (d, *J* = 12.4 Hz, 2H), 1.25 (s, 1H), 1.14 (d, *J* = 5.9 Hz, 2H), 1.02 (t, *J* = 6.9 Hz, 3H). 555.4 [M+H]$^+$ | 1.35 |
| 94 | 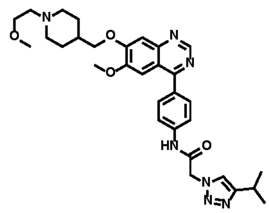 | 2-(4-isopropyl-1*H*-1,2,3-triazole-1-yl)-*N*-(4-(6-methoxy-7-((1- (2-methoxyethyl) piperid in-4-yl)methoxy) quinazoli n-4-yl)phenyl) acetamide | $^1$H NMR (400 MHz, DMSO-$d_6$): δ 10.74 (s, 1H), 9.09 (s, 1H), 7.90 (d, *J* = 0.7 Hz, 1H), 7.87-7.82 (m, 4H), 7.41 (s, 1H), 7.35 (s, 1H), 5.33 (s, 2H), 4.09 (d, *J* = 6.2 Hz, 2H), 3.85 (s, 3H), 3.45 (d, *J* = 5.8 Hz, 2H), 3.24 (s, 3H), 3.05-2.89 (m, 3H), 2.05 (d, *J* = 28.9 Hz, 2H), 1.78 (d, *J* = 13.4 Hz, 3H), 1.43-1.33 (m, 2H), 1.26 (d, *J* = 6.9 Hz, 6H). 574.4 [M+H]$^+$ | 1.11 |
| 95 | 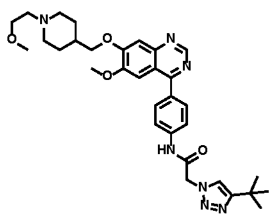 | 2-(4-(tert-butyl)-1*H*-1,2,3-triazole-1-yl)-N-(4-(6-methoxy-7-((1- (2-methoxyethyl) piperid in-4-yl)methoxy) quinazoli n-4-yl)phenyl) acetamide | $^1$H NMR (400 MHz, DMSO-$d_6$): δ 10.75 (s, 1H), 9.09 (s, 1H), 7.90 (s, 1H), 7.89-7.81 (m, 4H), 7.41 (s, 1H), 7.35 (s, 1H), 5.33 (s, 2H), 4.08 (d, *J* = 6.1 Hz, 2H), 3.85 (s, 3H), 3.43 (t, *J* = 5.9 Hz, 2H), 3.23 (s, 3H), 2.92 (d, *J* = 10.9 Hz, 2H), 2.00 (s, 2H), 1. 87-1. 72 (m, 3H), 1.38 (t, *J* = 11.7 Hz, 2H), 1.31 (s, 9H). 588.4 [M+H]$^+$ | 1.16 |

(continued)

| Example | Structure | Compound Name | $^1$H NMR; MS(m/z) | UPLC r.t. (min) |
|---|---|---|---|---|
| 96 | 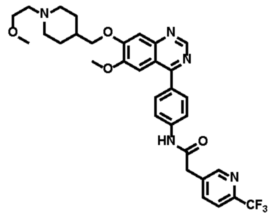 | *N*-(4-(6-methoxy-7-((1-(2-methoxyethyl) piperid in-4-yl)methoxy) quinazoli n-4-yl)phenyl)-2-(6-(trifluoromethyl)pyr idin-3-yl)acetamide | $^1$H NMR (400 MHz, DMSO-$d_6$): δ 10.60 (s, 1H), 9.08 (s, 1H), 8.75 (d, *J* = 2.1 Hz, 1H), 8.07 (dd, J = 8.1, 2.1 Hz, 1H), 7.91 (d, *J* = 8.0 Hz, 1H), 7.83 (s, 4H), 7.41 (s, 1H), 7.35 (s, 1H), 4.09 (d, *J* = 6.2 Hz, 2H), 3.94 (s, 2H), 3.85 (s, 3H), 3.46 (d, *J* = 5.8 Hz, 2H), 3.24 (s, 3H), 2.98 (d, *J* = 11.0 Hz, 2H), 2.61-2.53 (m, 2H), 2.10 (d, *J* = 12.8 Hz, 2H), 1.84 (dd, *J* = 37.3, 15.6 Hz, 3H), 1. 48-1. 31 (m, 2H). 610.4 [M+H]$^+$ | 1.23 |
| 97 | 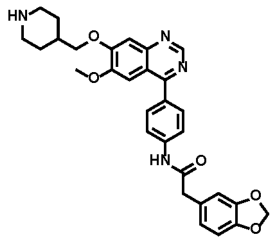 | 2-(benzo[*d*][1,3]dioxol -5-yl)-*N*-(4-(6-methoxy-7-(piperidin-4-ylmethoxy)quinazolin -4-yl)phenyl)acetamide | $^1$H NMR (400 MHz, DMSO-$d_6$): δ 10.51 (s, 1H), 9.08 (s, 1H), 7.83 (s, 4H), 7.72 (d, *J* = 7.7 Hz, 2H), 7.60 (d, *J* = 7.7 Hz, 2H), 7.39 (d, *J* = 21.8 Hz, 2H), 4.11 (s, 2H), 3.85 (s, 3H), 2.08 (s, 2H), 1.90 (s, 4H), 1.69 (s, 1H), 1.55-1.45 (m, 1H), 1.28 (d, *J* = 26.4 Hz, 3H), 0.86 (s, 1H) . 527.4 [M+H]$^+$ | 1.17 |
| 98 | 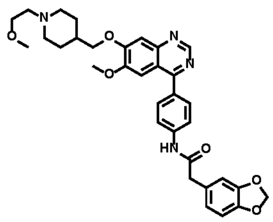 | 2-(benzo[*d*][1,3]dioxol -5-yl)-*N*-(4-(6-methoxy-7-((1-(2-methoxyethyl) piperid in-4-yl)methoxy) quinazoli n-4-yl)phenyl) acetamide | $^1$H NMR (400 MHz, DMSO-$d_6$): δ 10.41 (s, 1H), 9.08 (s, 1H), 7.87-7.80 (m, 4H), 7.37 (d, *J* = 17.2 Hz, 2H), 6.95 (d, *J* = 1.7 Hz, 1H), 6.91-6.81 (m, 2H), 6.00 (s, 2H), 4.08 (d, *J* = 6.2 Hz, 2H), 3.85 (s, 3H), 3.61 (s, 2H), 2.95 (dt, *J* = 11.9, 3.4 Hz, 2H), 2.39 (q, *J* = 7.2 Hz, 2H), 2.02-1.95 (m, 2H), 1.84-1.78 (m, 2H), 1.38 (qd, *J* = 12.0, 3.6 Hz, 2H), 1.02 (t, *J* = 7.1 Hz, 3H). 585.4 [M+H]$^+$ | 1.21 |
| 99 | 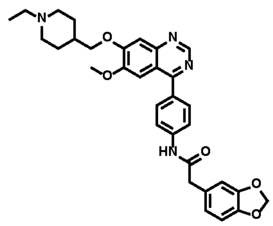 | 2-(benzo[*d*][1,3]dioxol -5-yl)-*N*-(4-(7-((1-ethylpiperidin-4-yl) methoxy)-6-methoxyquinazolin-4-yl)phenyl)acetamide | $^1$H NMR (400 MHz, DMSO-$d_6$): δ 10.41 (s, 1H), 9.08 (s, 1H), 7.87-7.80 (m, 4H), 7.37 (d, *J* = 17.2 Hz, 2H), 6.95 (d, *J* = 1.7 Hz, 1H), 6.91-6.81 (m, 2H), 6.00 (s, 2H), 4.08 (d, *J* = 6.2 Hz, 2H), 3.85 (s, 3H), 3.61 (s, 2H), 2.95 (dt, *J* = 11.9, 3.4 Hz, 2H), 2.39 (q, *J* = 7.2 Hz, 2H), 2.02-1.95 (m, 2H), 1.84-1.78 (m, 2H), 1.38 (qd, *J* = 12.0, 3.6 Hz, 2H), 1.02 (t, *J* = 7.1 Hz, 3H). 555.4 [M+H]$^+$ | 1.21 |

(continued)

| Example | Structure | Compound Name | $^1$H NMR; MS(m/z) | UPLC r.t. (min) |
|---|---|---|---|---|
| 100 | 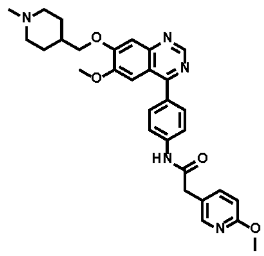 | *N*-(4-(6-methoxy-7-((1-methylpiperidin-4-yl) methoxy)quinazoli n-4-yl)phenyl)-2-(6-methoxypyridin-3-yl) acetamide | 528.4 [M+H]$^+$ | 1.09 |
| 101 | 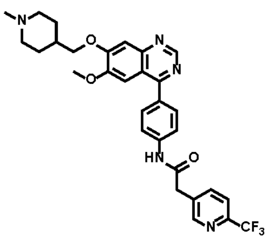 | *N*-(4-(6-methoxy-7-((1-methylpiperidin-4-yl) methoxy)quinazoli n-4-yl)phenyl)-2-(6-(trifluoromethyl)pyr idin-3-yl)acetamide | 566.4 [M+H]$^+$ | 1.20 |
| 102 | 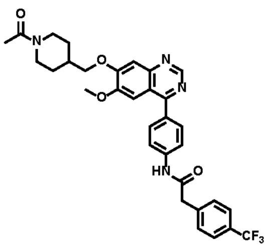 | *N*-(4-(7-((1-acetylpiperidin-4-yl) methoxy)-6-methoxyquinazolin-4-yl)phenyl)-2-(4-(trifluoromethyl) phe nyl)acetamide | 593.4 [M+H]$^+$ | 1.57 |
| 103 | 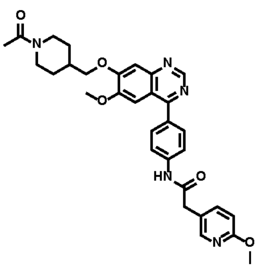 | *N*-(4-(7-((1-acetylpiperidin-4-yl) methoxy)-6-methoxyquinazolin-4-yl)phenyl)-2-(6-methoxypyridin-3-yl) acetamide | 556.3 [M+H]$^+$ | 1.29 |
| 104 | 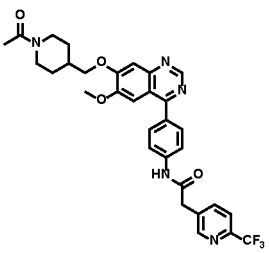 | *N*-(4-(7-((1-acetylpiperidin-4-yl) methoxy)-6-methoxyquinazolin-4-yl)phenyl)-2-(6-(trifluoromethyl)pyr idin-3-yl)acetamide | 594.3 [M+H]$^+$ | 1.42 |
| 105 | 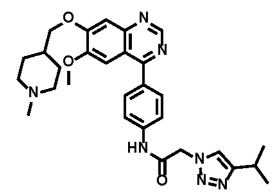 | 2-(4-isopropyl-1*H*-1,2,3-triazole-1-yl)-*N*-(4-(6-methoxy-7-((1-methylpiperidin-4-yl)methoxy)quinazoli n-4-yl)phenyl)acetamide | 530.3 [M+H]$^+$ | 1.09 |

(continued)

| Example | Structure | Compound Name | $^1$H NMR; MS(m/z) | UPLC r.t. (min) |
|---|---|---|---|---|
| 106 | 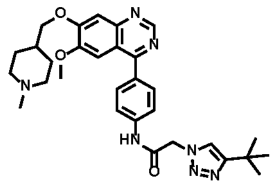 | 2-(4-(tert-butyl)-1H-1,2,3-triazole-1-yl)-*N*-(4-(6-methoxy-7-((1-methylpiperidin-4-yl)methoxy)quinazoli n-4-yl)phenyl) acetamide | 544.3 [M+H]$^+$ | 1.13 |
| 107 | 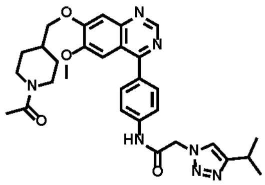 | *N*-(4-(7-((1-acetylpiperidin-4-yl) methoxy)-6-methoxyquinazolin-4-yl)phenyl)-2-(4-isopropyl-1*H*-1,2,3-triazole-1-yl)acetamide | 558.3 [M+H]$^+$ | 1.28 |
| 108 | 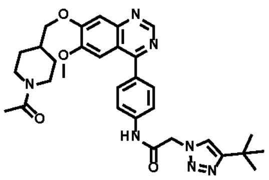 | *N*-(4-(7-((1-acetylpiperidin-4-yl) methoxy)-6-methoxyquinazolin-4-yl)phenyl)-2-(4-(tert-butyl)-1*H*-1,2,3-triazole-1-yl)acetamide | 572.4 [M+H]$^+$ | 1.34 |
| 109 | 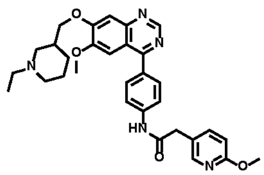 | *N*-(4-(7-((1-ethylpiperidin-3-yl) methoxy)-6-methoxyquinazolin-4-yl)phenyl)-2-(6-methoxypyridin-3-yl) acetamide | 542.3 [M+H]$^+$ | 1.12 |
| 110 | 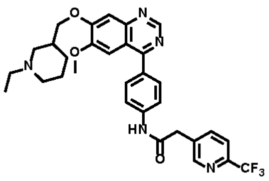 | *N*-(4-(7-((1-ethylpiperidin-3-yl) methoxy)-6-methoxyquinazolin-4-yl)phenyl)-2-(6-(trifluoromethyl)pyr idin-3-yl)acetamide | 580.3 [M+H]$^+$ | 1.22 |
| 111 | 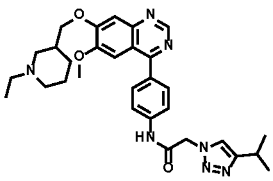 | *N*-(4-(7-((1-ethylpiperidin-3-yl) methoxy)-6-methoxyquinazolin-4-yl)phenyl)-2-(4-isopropyl-1*H*-1,2,3-triazole-1-yl)acetamide | 544.4 [M+H]$^+$ | 1.12 |

(continued)

| Example | Structure | Compound Name | $^1$H NMR; MS(m/z) | UPLC r.t. (min) |
|---|---|---|---|---|
| 112 | 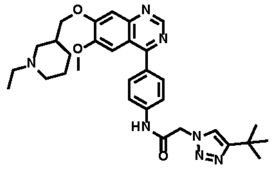 | 2-(4-(tert-butyl)-1*H*-1,2,3-triazole-1-yl)-N-(4-(7-((1-ethylpiperidin-3-yl) methoxy)-6-methoxyquinazolin-4-yl)phenyl)acetamide | $^1$H NMR (400 MHz, MeOD-$d_4$): δ 8.97 (s, 1H), 7.88 - 7.82 (m, 3H), 7.78 - 7.71 (m, 2H), 7.29 - 7.23 (m, 2H), 5.37 (s, 2H), 4.23 - 4.15 (m, 1H), 4.12 - 4.06 (m, 1H), 3.85 (s, 3H), 3.72 - 3.63 (m, 1H), 3.58 - 3.50 (m, 1H), 3.22 - 3.12 (m, 2H), 2.91 - 2.78 (m, 2H), 2.55 - 2.40 (m, 1H), 2.08 - 1.98 (m, 2H), 1.93 - 1.84 (m, 1H), 1.58 - 1.44 (m, 1H), 1.39 - 1.33 (m, 12H) . 558.4 $[M+H]^+$ | 1.16 |
| 113 | 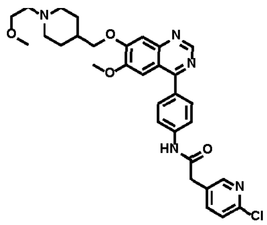 | 2-(6-chloropyridin-3-yl)-*N*-(4-(6-methoxy-7-((1-(2-methoxyethyl) piperid in-4-yl)methoxy) quinazoli n-4-yl)phenyl) acetamide | $^1$H NMR (400 MHz, DMSO-$d_6$): δ 10.53 (s, 1H), 9.07 (s, 1H), 8.38 (d, *J* = 2.4 Hz, 1H), 7.87-7.77 (m, 5H), 7.50 (d, *J* = 8.2 Hz, 1H), 7.39 (s, 1H), 7.34 (s, 1H), 4.07 (d, *J* = 6.2 Hz, 2H), 3.84 (s, 3H), 3.79 (s, 2H), 3.44 (s, 2H), 3.23 (s, 3H), 2.95 (d, *J* = 10.6 Hz, 2H), 2.53 (s, 2H), 1. 89-1. 74 (m, 3H), 1.39 (t, *J* = 12.5 Hz, 2H).<br>576.3 $[M+H]^+$ | 1.17 |
| 114 | 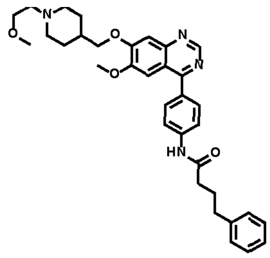 | *N*-(4-(6-methoxy-7-((1-(2-methoxyethyl) piperid in-4-yl)methoxy) quinazoli n-4-yl)phenyl)-4-phenylbutanamide | $^1$H NMR (400 MHz, DMSO-$d_6$): δ 10.18 (s, 1H), 9.08 (s, 1H), 7.82 (s, 4H), 7.46-7.14 (m, 12H), 4.09 (d, *J* = 6.2 Hz, 2H), 3.86 (s, 3H), 3.02-2.91 (m, 4H), 2.82 (t, *J* = 7.5 Hz, 2H), 2.70 (t, J = 7.7 Hz, 2H), 2.09 (s, 2H), 1.79 (d, *J* = 12.6 Hz, 2H), 1.40 (s, 2H), 1.25 (s, 1H), 0.95-0.79 (m, 1H).<br>555.4 $[M+H]^+$ | 1.30 |
| 115 | 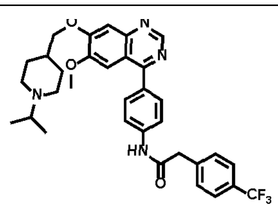 | *N*-(4-(7-((1-isopropylpiperidin-4-yl) methoxy)-6-methoxyquinazolin-4-yl)phenyl)-2-(4-(trifluoromethyl) phe nyl)acetamide | $^1$H NMR (400 MHz, DMSO-$d_6$): δ 10.53 (s, 1H), 9.08 (s, 1H), 7.88-7.77 (m, 4H), 7.72 (d, *J* = 8.1 Hz, 2H), 7.60 (d, *J* = 8.0 Hz, 2H), 7.37 (d, *J* = 17.4 Hz, 2H), 4.07 (d, *J* = 6.0 Hz, 2H), 3.85 (s, 5H), 2.88-2.80 (m, 2H), 2.74-2.65 (m, 1H), 2.22-2.11 (m, 2H), 1.85-1.75 (m, 3H), 1.39-1.26 (m, 2H), 0.98 (d, *J* = 6.5 Hz, 6H).<br>593 $[M+H]^+$ | 1.38 |

(continued)

| Example | Structure | Compound Name | $^1H$ NMR; MS(m/z) | UPLC r.t. (min) |
|---|---|---|---|---|
| 116 | 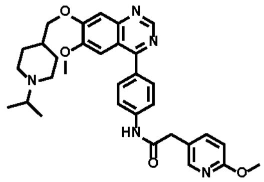 | *N*-(4-(7-((1-isopropylpiperidin-4-yl) methoxy)-6-methoxyquinazolin-4-yl)phenyl)-2-(6-methoxypyridin-3-yl) acetamide | $^1H$ NMR (400 MHz, DMSO-$d_6$): δ 10.49 (s, 1H), 9.08 (s, 1H), 8.23-8.03 (m, 1H), 7.89-7.78 (m, 3H), 7.70 (dd, J = 8.5, 2.5 Hz, 1H), 7.38 (d, *J* = 18.5 Hz, 2H), 6.81 (dd, *J* = 8.5, 0.7 Hz, 1H), 4.08 (d, *J* = 6.0 Hz, 2H), 3.94-3.76 (m, 6H), 3.68 (s, 2H), 3.08-2.61 (m, 3H), 2.46-2.01 (m, 2H), 1.82 (d, *J* = 12.8 Hz, 3H), 1.53-1.27 (m, 2H), 1.01 (d, *J* = 6.6 Hz, 6H).<br>556.4 $[M+H]^+$ | 1.14 |
| 117 | 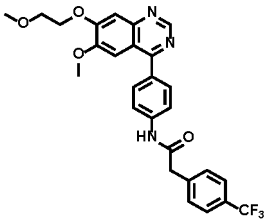 | *N*-(4-(6-methoxy-7-(2-methoxyethoxy)quinazolin-4-yl)phenyl)-2-(4-(trifluoromethyl) phe nyl)acetamide | $^1H$ NMR (400 MHz, $CDCl_{3-}$ $d_3$): δ 9.17 (s, 1H), 7.79-7.73 (m, 2H), 7.71-7.65 (m, 5H), 7.51 (d, *J* = 8.0 Hz, 2H), 7.41-7.35 (m, 2H), 7.31 (s, 1H), 4.08 (s, 3H), 3.90 (s, 3H), 3.85 (s, 2H), 3.53-3.45 (m, 4H).<br>512.3 $[M+H]^+$ | 1.61 |
| 118 | 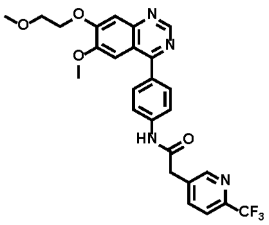 | *N*-(4-(6-methoxy-7-(2-methoxyethoxy)quinazolin-4-yl)phenyl)-2-(6-(trifluoromethyl)pyridin-3-yl) acetamide | $^1H$ NMR (400 MHz, $CDCl_{3-}$ $d_3$) δ 9.18 (s, 1H), 8.72 (s, 1H), 7.98 (d, *J* = 7.6 Hz, 1H), 7.83-7.76 (m, 2H), 7.75-7.68 (m, 3H), 7.48 (s, 1H), 7.41-7.36 (m, 1H), 7.32 (s, 1H), 4.08 (s, 3H), 3.90 (s, 3H), 3.86 (s, 2H), 3.59-3.40 (m, 4H).<br>513.3 $[M+H]^+$ | 1.45 |
| 119 | 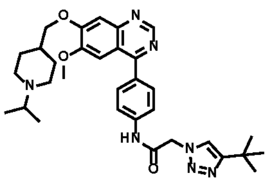 | 2-(4-(tert-butyl)-1H-1,2,3-triazole-1-yl)-*N*-(4-(7-((1-isopropylpiperidin-4-yl) methoxy)-6-methoxyquinazolin-4-yl)phenyl) acetamide | $^1H$ NMR (400 MHz, $CDCl_{3-}$ $d_3$): δ 9.15 (s, 1H), 8.57-8.41 (m, 1H), 7.82-7.75 (m, 2H), 7.72 (d, J = 8.6 Hz, 2H), 7.50 (s, 1H), 7.33 (s, 1H), 5.20 (s, 2H), 4.12-3.99 (m, 2H), 3.87 (s, 3H), 3.04-2.88 (m, 2H), 2.82-2.66 (m, 1H), 2.32-2.11 (m, 2H), 2.07-1.84 (m, 4H), 1.43-1.38 (m, 9H), 1.14-1.00 (m, 6H).<br>572.4 $[M+H]^+$ | 1.20 |
| 120 | 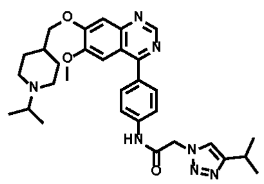 | 2-(4-isopropyl-1*H*-1,2,3-triazole-1-yl)-*N*-(4-(7-((1-isopropylpiperidin-4-yl) methoxy)-6-methoxyquinazolin-4-yl)phenyl) acetamide | $^1H$ NMR (400 MHz, $CDCl_{3-}$ $d_3$): δ 9.14 (s, 1H), 8.54-8.31 (m, 1H), 7.81-7.74 (m, 2H), 7.72 (d, *J* = 8.4 Hz, 2H), 7.50 (s, 1H), 7.33 (s, 1H), 5.21 (s, 2H), 4.13-3.96 (m, 2H), 3.86 (s, 3H), 3.23-3.09 (m, 1H), 3.05-2.87 (m, 2H), 2.84-2.63 (m, 1H), 2.31-2.10 (m, 2H), 2.05-1.86 (m, 3H), 1.36 (d, *J* = 6.9 Hz, 6H), 1.16-0.98 (m, 6H).<br>558.4 $[M+H]^+$ | 1.15 |

(continued)

| Example | Structure | Compound Name | $^1$H NMR; MS(m/z) | UPLC r.t. (min) |
|---|---|---|---|---|
| 121 | 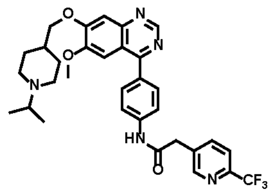 | *N*-(4-(7-((1-isopropylpiperidin-4-yl) methoxy)-6-methoxyquinazolin-4-yl)phenyl)-2-(6-(trifluoromethyl)pyridin-3-yl) acetamide | $^1$H NMR (400 MHz, $CDCl_{3-}$ $d_3$): δ 9.15 (s, 1H), 8.75-8.68 (m, 1H), 8.01-7.94 (m, 1H), 7.78 (d, *J* = 8.6 Hz, 2H), 7.74-7.68 (m, 3H), 7.59-7.49 (m, 1H), 7.34 (s, 1H), 7.29 (s, 1H), 4.06 (d, J = 6.5 Hz, 2H), 3.87 (s, 3H), 3.86 (s, 2H), 3.10-2.87 (m, 2H), 2.82-2.66 (m, 1H), 2.33-2.13 (m, 2H), 2.08-1.87 (m, 4H), 1.55-1.38 (m, 2H), 1.18-0.97 (m, 6H).<br>594.4 $[M+H]^+$ | 1.26 |

**<Example 122> Preparation of N-(4-(6-(2-(diethylamino)ethoxy)-7-methoxyquinazolin-4-yl)phenyl)-2-(4-fluorophenyl)acetamide**

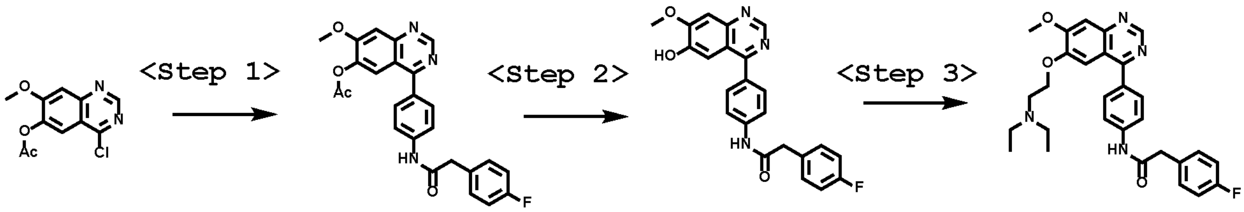


Step 1: After dissolving 4-chloro-7-methoxyquinazolin-6-yl acetate (0.10g, 0.40mmol) and (4-(2-(4-fluorophenyl)acetamido)phenyl)boronic acid (0.11g, 0.40mmol) in 1,4-dioxane (1.0ml) and 1M sodium carbonate (0.3ml), tetrakis(triphenylphosphine)palladium(0) (0.14g, 0.12mmol) was added dropwise. After bubbling with nitrogen, it was stirred overnight at 80°C. After completion of the reaction, it was filtered through a celite filter, washed with methanol, and then concentrated under reduced pressure to obtain desired compound 4-(4-(2-(4-fluorophenyl)acetamido)phenyl)-7-methoxyquinazolin-6-yl acetate (250.0mg, 70% purity, 99% yield). It was used in the following reaction without further purification.
MS(m/z): 446.2 $[M+H]^+$ UPLC r.t.(min): 1.55

Step 2: After dissolving 4-(4-(2-(4-fluorophenyl)acetamido)phenyl)-7-methoxyquinazolin-6-yl acetate (0.25g, 0.56mmol) obtained in the step 1 in 7N (4.0ml) of ammonia solution in methanol, and then it was stirred at room temperature for 30 minutes. After completion of the reaction, it was filtered through a celite filter, washed with dichloromethane, and concentrated under reduced pressure. A solid desired compound 2-(4-fluorophenyl)-N-(4-(6-hydroxy-7-methoxyquinazolin-4-yl)phenyl)acetamide (0.23 g, 80% purity, 81% yield) was obtained using dichloromethane and ether. It was used in the following reaction without further purification.
MS(m/z): 404.1 $[M+H]^+$ UPLC r.t.(min): 1.33

Step 3: After dissolving 2-(4-fluorophenyl)-N-(4-(6-hydroxy-7-methoxyquinazolin-4-yl)phenyl)acetamide (0.04g, 0.10mmol) obtained in the step 2, 2-chloro-N,N-diethylethane-1-amine hydrochloride (1.9mg, 0.1mmol), and potassium carbonate (3.0mg, 0.22mmol) in acetonitrile (0.3ml) and distilled water (0.3ml), and it was stirred at 80 °C for 2 hours. After the completion of the reaction, distilled water was added, and the organic matter was extracted with ethyl acetate (x3). After washing the collected organic layer with brine, the remaining water was removed using sodium sulfate, and concentrated under reduced pressure. After separating and purifying the concentrated mixture by MPLC (DCM:MeOH=0-10%), the desired compound N-(4-(6-(2-(diethylamino)ethoxy)-7-methoxyquinazolin-4-yl)phenyl)-2-(4-fluorophenyl)acetamide (17.0mg, 34.1%) was obtained.
MS(m/z): 503.3 $[M+H]^+$ UPLC r.t.(min): 1.46

**<Example 123> Preparation of N-(4-(6-((1-ethylpiperidin-4-yl)oxy)-7-methoxyquinazolin-4-yl)phenyl)-2-(4-(trifluoromethyl)phenyl)acetamide**

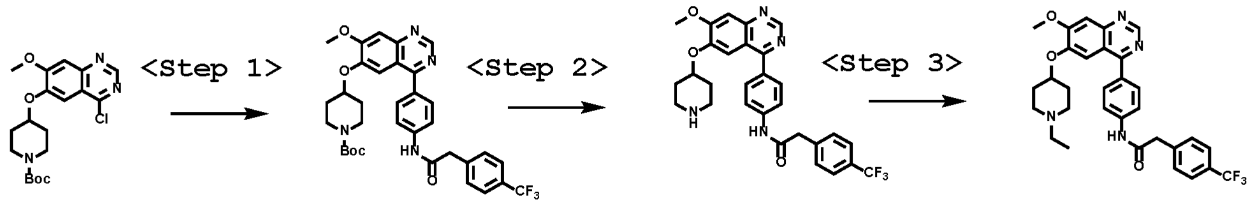


Step 1: After dissolving tert-butyl 4-((4-chloro-7-methoxyquinazolin-6-yl)oxy)piperidin-1-carboxylate (0.30g, 0.75mmol) and (4-(2-(4-trifluoromethylphenyl)acetamido)phenyl)boronic acid (0.36g, 1.12mmol) in 1,4-dioxane (2.1ml) and 2M sodium carbonate (0.9ml), tetrakis(triphenylphosphine)palladium(0) (0.26g, 0.23mmol) was added dropwise. After bubbling with nitrogen, it was stirred overnight at 80°C. After completion of the reaction, it was filtered through a celite filter and concentrated under reduced pressure to obtain the desired compound tert-butyl 4-((7-methoxy-4-(4-(2-(4-(trifluoromethyl)phenyl)acetamido)phenyl)quinazolin-6-yl)oxy)piperidin-1-carboxylate (0.26g, 54.5 %). It was used in the following reaction without further purification.
MS(m/z): 637.4 [M+H]$^+$ UPLC r.t.(min): 1.87

Step 2: After dissolving tert-butyl 4-((7-methoxy-4-(4-(2-(4-(trifluoromethyl)phenyl)acetamido)phenyl)quinazolin-6-yl)oxy)piperidin-1-carboxylate (0.26g, 0.41mmol) obtained in the step 1 in dichloromethane (4.1ml), trifluoroacetic acid (0.47g, 4.08mmol) was added dropwise and stirred at room temperature overnight. After the completion of the reaction, it was concentrated under reduced pressure, and then the desired compound N-(4-(7-methoxy-6-(piperidin-4-yloxy)quinazolin-4-yl)phenyl)-2-(4-(trifluoromethyl)phenyl)acetamide 2,2,2-trifluoroacetic acid (0.2 g, 75%) was separated and purified by prep-HPLC.
MS(m/z): 537.3 [M+H]$^+$ UPLC r.t.(min): 1.25

Step 3: After dissolving N-(4-(7-methoxy-6-(piperidin-4-yloxy)quinazolin-4-yl)phenyl)-2-(4-(trifluoromethyl)phenyl)acetamide 2,2,2-trifluoroacetic acid (0.03g, 0.05mmol) obtained in the step 2 and sodium triacetoxyborohydride (0.02g, 0.09mmol) in 1,4-dioxane (0.5 ml), acetaldehyde (3.05mg, 0.07mmol) and N,N-diisopropylethylamine (0.03g, 0.23mmol) were added dropwise, followed by stirring at room temperature overnight. After completion of the reaction, it was concentrated under reduced pressure and purified through prep-HPLC to obtain the desired compound N-(4-(6-((1-ethylpiperidin-4-yl)oxy)-7-methoxyquinazolin-4-yl)phenyl)-2-(4-(trifluoromethyl)phenyl)acetamide (15.0mg, 57.6%).
MS(m/z): 565.3 [M+H]+ UPLC r.t.(min): 1.29

**Examples 122 to 164**

Examples 124 to 164 were prepared in a similar manner to Examples 122 and 123, and the compound names, NMR, mass, and UPLC analysis results of Examples 122 to 164 are summarized in Table 3 below.

[Table 3]

| Example | Structure | Compound Name | $^1$H NMR; MS(m/z) | UPLC r.t. (min) |
|---|---|---|---|---|
| 122 | 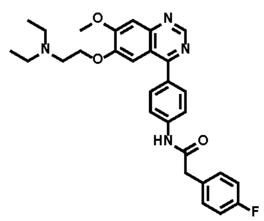 | N-(4-(6-(2-(diethylamino) ethoxy )-7-methoxyquinazolin-4-yl)phenyl)-2-(4-fluorophenyl)acetami de | $^1$H NMR (400 MHz, $CDCl_3$-$d_3$): δ 9.15 (s, 1H), 7.75-7.63 (m, 4H), 7.39-7.33 (m, 3H), 7.31 (s, 1H), 7.20 (s, 1H), 7.13 (t, $J$ = 8.6 Hz, 2H), 4.10-4.05 (m, 2H), 4.04 (s, 3H), 3.78 (s, 2H), 2.93 (t, $J$ = 6.6 Hz, 2H), 2.62 (q, $J$ = 7.1 Hz, 4H), 1.04 (t, $J$ = 7.1 Hz, 6H). 503.3 [M+H]$^+$ | 1.46 |

(continued)

| Example | Structure | Compound Name | $^1H$ NMR; MS(m/z) | UPLC r.t. (min) |
|---|---|---|---|---|
| 123 | 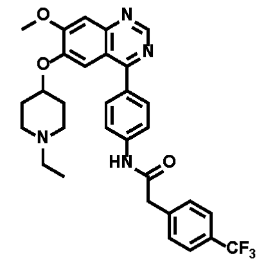 | *N*-(4-(6-((1-ethylpiperidin-4-yl) oxy)-7-methoxyquinazolin-4-yl)phenyl)-2-(4-(trifluoromethyl)phe nyl) acetamide | 565.3 [M+H]$^+$ | 1.30 |
| 124 | 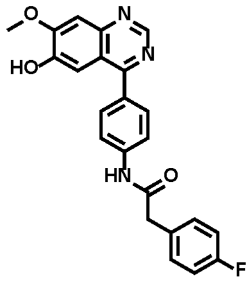 | 2-(4-fluorophenyl)-*N*-(4-(6-hydroxy-7-methoxyquinazolin-4-yl)phenyl)acetamide | $^1H$ NMR (400 MHz, DMSO-$d_6$): δ 10.44 (s, 1H), 9.03 (s, 1H), 7.81 (d, *J* = 8.6 Hz, 2H), 7.71 (d, *J* = 8.6 Hz, 2H), 7.64-7.60 (m, 2H), 7.58-7.54 (m, 2H), 7.41-7.39 (m, 1H), 7.17 (t, *J* = 8.8 Hz, 2H), 4.00 (s, 3H), 3.71 (s, 2H). 404.3 [M+H]$^+$ | 1.43 |
| 125 | 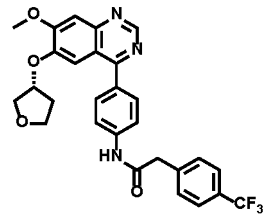 | (*R*)-*N*-(4-(7-methoxy-6-((tetrahydrofuran-3-yl)oxy) quinazolin-4-yl)phenyl)-2-(4-(trifluoromethyl)phe nyl) acetamide | $^1H$ NMR (400 MHz, DMSO-$d_6$): δ 10.56 (s, 1H), 9.10 (s, 1H), 7.87-7.78 (m, 4H), 7.72 (d, *J* = 8.1 Hz, 2H), 7.60 (d, *J* = 8.0 Hz, 2H), 7.44 (s, 1H), 7.28 (s, 1H), 5.08-4.99 (m, 1H), 4.00 (s, 3H), 3.92 (d, *J* = 10.5 Hz, 1H), 3.89-3.79 (m, 4H), 3.73 (td, *J* = 8.3, 5.0 Hz, 1H), 2.22-2.03 (m, 2H). 524.3 [M+H]$^+$ | 1.62 |
| 126 | 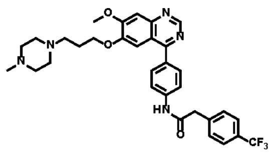 | *N*-(4-(7-methoxy-6-(3-(4-methylpiperazin-1-yl)propoxy) quinazoli n-4-yl)phenyl)-2-(4-(trifluoromethyl)phenyl) acetamide | $^1H$ NMR (400 MHz, DMSO-$d_6$): δ 10.53 (s, 1H), 9.09 (s, 1H), 7.82 (q, *J* = 8.8 Hz, 4H), 7.72 (d, *J* = 8.0 Hz, 2H), 7.60 (d, *J* = 8.0 Hz, 2H), 7.42 (s, 1H), 7.33 (s, 1H), 4.05 (t, *J* = 6.4 Hz, 2H), 4.00 (s, 3H), 3.84 (s, 2H), 2.40 (t, *J* = 7.0 Hz, 8H), 2.12 (s, 3H), 1.92-1.86 (m, 2H). 594.3 [M+H]$^+$ | 1.22 |

(continued)

| Example | Structure | Compound Name | $^1H$ NMR; MS(m/z) | UPLC r.t. (min) |
|---|---|---|---|---|
| 127 | 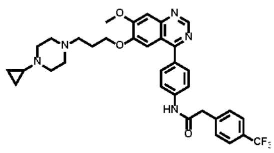 | *N*-(4-(6-(3-(4-cyclopropylpiperazin -1-yl) propoxy)-7-methoxyquinazolin-4-yl) phenyl)-2-(4-(trifluoromethyl) phe nyl)acetamide | $^1H$ NMR (400 MHz, DMSO-$d_6$): δ 10.29 (s, 1H), 8.85 (s, 1H), 7.58 (q, *J* = 8.7 Hz, 4H), 7.48 (d, *J* = 8.0 Hz, 2H), 7.36 (d, *J* = 8.0 Hz, 2H), 7.18 (s, 1H), 7.10 (s, 1H), 3.81 (t, *J* = 6.4 Hz, 2H), 3.76 (s, 3H), 3.60 (s, 2H), 3.16 (s, 2H), 2.23 (s, 8H), 2.14 (t, *J* = 7.1 Hz, 2H), 1.66 (q, *J* = 6.6 Hz, 2H), 1.31 (tt, *J* = 6.7, 3.6 Hz, 1H), 0.12 (td, *J* = 6.4, 4.1 Hz, 2H). 620.4 $[M+H]^+$ | 1.34 |
| 128 | 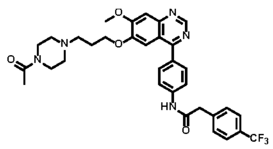 | *N*-(4-(6-(3-(4-acetylpiperazin-1-yl)propoxy)-7-methoxyquinazolin-4-yl) phenyl)-2-(4-(trifluoromethyl) phe nyl)acetamide | $^1H$ NMR (400 MHz, DMSO-$d_6$): δ 10.53 (s, 1H), 9.09 (s, 1H), 7.82 (q, *J* = 8.5 Hz, 4H), 7.72 (d, *J* = 8.0 Hz, 2H), 7.60 (d, *J* = 7.9 Hz, 2H), 7.42 (s, 1H), 7.35 (s, 1H), 4.08 (t, *J* = 6.4 Hz, 2H), 4.01 (s, 3H), 3.84 (s, 2H), 3.39 (s, 2H), 2.43 (t, *J* = 7.1 Hz, 2H), 2.34 (t, *J* = 5.0 Hz, 2H), 2.28 (t, *J* = 5.1 Hz, 2H), 1.96 (s, 3H), 1.92 (t, *J* = 6.9 Hz, 2H) . 622.4 $[M+H]^+$ | 1.28 |
| 129 | 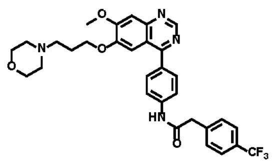 | *N*-(4-(7-methoxy-6-(3-morpholinopropoxy)qu inazolin-4-yl)phenyl)-2-(4-(trifluoromethyl)phe nyl) acetamide | $^1H$ NMR (400 MHz, DMSO-$d_6$): δ 10.54 (s, 1H), 9.09 (d, *J* = 0.9 Hz, 1H), 7.82 (q, *J* = 8.7 Hz, 4H), 7.72 (d, *J* = 8.0 Hz, 2H), 7.60 (d, *J* = 8.0 Hz, 2H), 7.42 (s, 1H), 7.33 (s, 1H), 4.06 (t, *J* = 6.3 Hz, 2H), 4.01 (s, 3H), 3.85 (s, 2H), 3.53 (t, *J* = 4.6 Hz, 4H), 2.40 (t, *J* = 7.2 Hz, 2H), 2.33 (t, *J* = 4.5 Hz, 4H), 1.91 (p, *J* = 6.7 Hz, 2H). 581.3 $[M+H]^+$ | 1.30 |
| 130 | 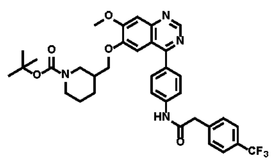 | *tert*-butyl 3-(((7-methoxy-4-(4-(2-(4-(trifluoromethyl)phe nyl)acetamido)phenyl ) quinazolin-6-yl)oxy)methyl) piperi din-1-carboxylate | 651.4 $[M+H]^+$ | 1.92 |

(continued)

| Example | Structure | Compound Name | $^1$H NMR; MS(m/z) | UPLC r.t. (min) |
|---|---|---|---|---|
| 131 | 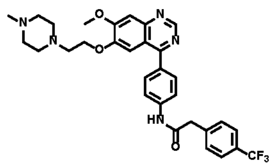 | *N*-(4-(7-methoxy-6-(2-(4-methylpiperazin-1-yl)ethoxy) quinazolin -4-yl)phenyl)-2-(4-(trifluoromethyl)phe nyl) acetamide | $^1$H NMR (400 MHz, DMSO-$d_6$): δ 10.55 (s, 1H), 9.10 (s, 1H), 7.88-7.79 (m, 4H), 7.75-7.69 (m, 2H), 7.60 (d, *J* = 8.0 Hz, 2H), 7.43 (s, 1H), 7.38 (s, 1H), 4.16 (t, *J* = 5.5 Hz, 2H), 4.01 (s, 3H), 3.85 (s, 2H), 2.94 (s, 1H), 2.82 (d, *J* = 30.3 Hz, 4H), 1.91 (s, 3H), 1.31-1.21 (m, 2H), 0.98-0.79 (m, 2H). 580.3 [M+H]$^+$ | 1.34 |
| 132 | 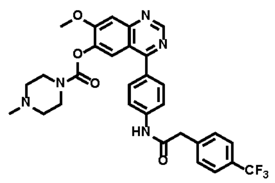 | 7-methoxy-4-(4-(2-(4-(trifluoromethyl)phe nyl) acetamido)phenyl ) quinazolin-6-yl 4-methylpiperazin-1-carboxylate | $^1$H NMR (400 MHz, DMSO-$d_6$): δ 10.55 (s, 1H), 9.21 (s, 1H), 7.87-7.82 (m, 2H), 7.76-7.70 (m, 5H), 7.62-7.56 (m, 3H), 4.01 (s, 3H), 3.85 (s, 2H), 3.59 (s, 2H), 3.43-3.38 (m, 2H), 2.35 (s, 4H), 2.22 (s, 3H). 580.3 [M+H]$^+$ | 1.29 |
| 133 | 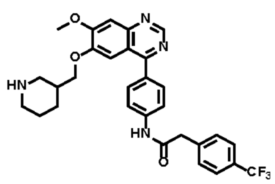 | *N*-(4-(7-methoxy-6-(piperidin-3-ylmethoxy)quinazolin -4-yl) phenyl)-2-(4-(trifluoromethyl) phe nyl) acetamide | $^1$H NMR (400 MHz, DMSO-$d_6$): δ 10.67 (s, 1H), 9.08 (s, 1H), 7.82 (q, *J* = 8.8 Hz, 4H), 7.72 (d, *J* = 8.1 Hz, 2H), 7.60 (d, *J* = 8.1 Hz, 2H), 7.41 (s, 1H), 7.32 (s, 1H), 4.01 (s, 3H), 3.87 (d, *J* = 9.6 Hz, 4H), 1.91 (s, 2H), 1.55 (s, 2H), 1.46-1.13 (m, 4H), 0.89-0.81 (m, 1H). 551.3 [M+H]$^+$ | 1.31 |
| 134 | 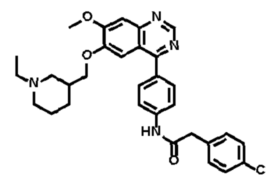 | *N*-(4-(6-((1-ethylpiperidin-3-yl) methoxy)-7-methoxyquinazolin-4-yl) phenyl)-2-(4-(trifluoromethyl) phe nyl)acetamide | $^1$H NMR (400 MHz, DMSO-$d_6$): δ 10.54 (s, 1H), 9.09 (s, 1H), 7.87-7.76 (m, 4H), 7.72 (d, *J* = 8.0 Hz, 2H), 7.60 (d, *J* = 8.0 Hz, 2H), 7.43 (s, 1H), 7.33 (s, 1H), 4.01 (s, 3H), 3.92 (d, *J* = 6.0 Hz, 2H), 3.84 (s, 2H), 1.74 (s, 2H), 1.48 (s, 2H), 1.24 (s, 3H), 1.06-0.93 (m, 3H), 0.90-0.79 (m, 1H). 579.3 [M+H]$^+$ | 1.34 |
| 135 | 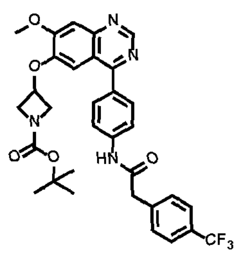 | *tert*-butyl 3-((7-methoxy-4-(4-(2-(4-(trifluoromethyl)phe nyl)acetamido)phenyl ) quinazolin-6-yl)oxy)azetidin-1-carboxylate | 609.3 [M+H]$^+$ | 1.80 |

(continued)

| Example | Structure | Compound Name | $^1$H NMR; MS(m/z) | UPLC r.t. (min) |
|---|---|---|---|---|
| 136 | 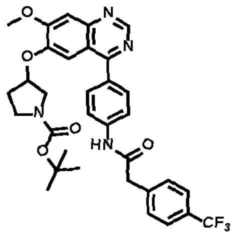 | *tert*-butyl 3-((7-methoxy-4-(4-(2-(4-(trifluoromethyl)phe nyl)acetamido)phenyl ) quinazolin-6-yl)oxy)pyrrolidin-1-carboxylate | 623.3 [M+H]$^+$ | 1.82 |
| 137 | 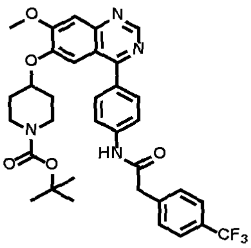 | *tert*-butyl 4-((7-methoxy-4-(4-(2-(4-(trifluoromethyl)phe nyl)acetamido)phenyl ) quinazolin-6-yl)oxy)piperidin-1-carboxylate | 637.4 [M+H]$^+$ | 1.88 |
| 138 | 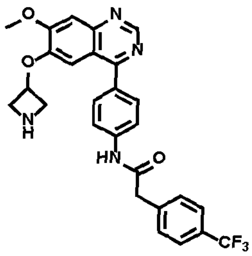 | *N*-(4-(6-(azetidin-3-yloxy)-7-methoxyquinazolin-4-yl) phenyl)-2-(4-(trifluoromethyl) phe nyl)acetamide | 509.3 [M+H]$^+$ | 1.25 |
| 139 | 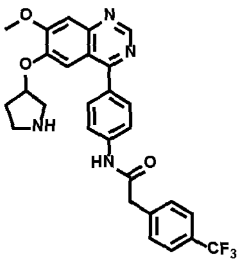 | *N*-(4-(7-methoxy-6-(pyrrolidin-3-yloxy)quinazolin-4-yl) phenyl)-2-(4-(trifluoromethyl) phe nyl)acetamide | 523.3 [M+H]$^+$ | 1.25 |
| 140 | 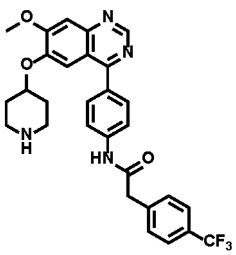 | *N*-(4-(7-methoxy-6-(piperidin-4-yloxy)quinazolin-4-yl) phenyl)-2-(4-(trifluoromethyl) phe nyl)acetamide | 537.3 [M+H]$^+$ | 1.25 |
| 141 | 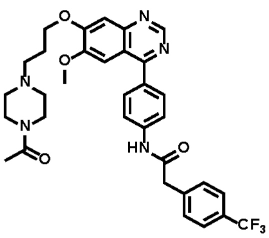 | *N*-(4-(7-(3-(4-acetylpiperazin-1-yl)propoxy)-6-methoxyquinazolin-4-yl) phenyl)-2-(4-(trifluoromethyl) phe nyl)acetamide | 622.4 [M+H]$^+$ | 1.30 |

(continued)

| Example | Structure | Compound Name | $^1$H NMR; MS(m/z) | UPLC r.t. (min) |
|---|---|---|---|---|
| 142 | 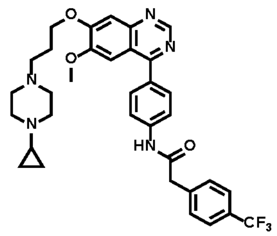 | *N*-(4-(7-(3-(4-cyclopropylpiperazin -1-yl) propoxy)-6-methoxyquinazolin-4-yl) phenyl)-2-(4-(trifluoromethyl) phe nyl)acetamide | 620.4 [M+H]+ | 1.35 |
| 143 | 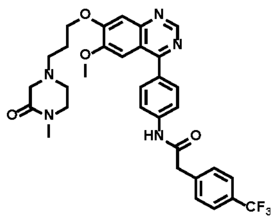 | *N*-(4-(6-methoxy-7-(3-(4-methyl-3-oxopiperazin-1-yl) propoxy)quinazoli n-4-yl) phenyl)-2-(4-(trifluoromethyl) phe nyl)acetamide | 608.3 [M+H]+ | 1.32 |
| 144 | 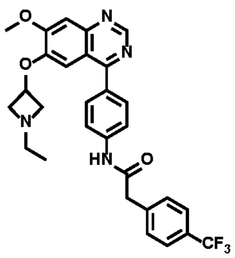 | *N*-(4-(6-((1-ethylazetidin-3-yl) oxy)-7-methoxyquinazolin-4-yl)phenyl)-2-(4-(trifluoromethyl)phe nyl) acetamide | 537.3 [M+H]+ | 1.30 |
| 145 | 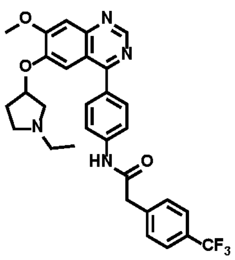 | *N*-(4-(6-((1-ethylpyrrolidin-3-yl)oxy)-7-methoxyquinazolin-4-yl)phenyl)-2-(4-(trifluoromethyl)phe nyl) acetamide | $^1$H NMR (400 MHz, MeOD-*d$_4$*): δ 9.03 (s, 1H), 7.86 (d, *J* = 8.4 Hz, 2H), 7.74 (d, *J* = 8.1 Hz, 2H), 7.66 (d, *J* = 8.2 Hz, 2H), 7.58 (d, *J* = 8.1 Hz, 2H), 7.41 (s, 1H), 7.32 (s, 1H), 4.06 (s, 3H), 3.86 (s, 2H), 3.50 - 3.40 (m, 1H), 3.38 - 3.31 (m, 2H), 3.16 - 2.96 (m, 3H), 2.46 - 2.33 (m, 1H), 2.30 - 2.18 (m, 1H), 1.27 (t, *J* = 7.2 Hz, 3H). 551.3 [M+H]+ | 1.30 |
| 146 | 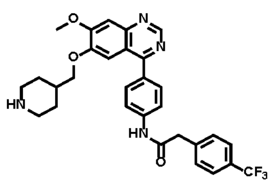 | *N*-(4-(7-methoxy-6-(piperidin-4-ylmethoxy) quinazolin -4-yl) phenyl)-2-(4-(trifluoromethyl) phe nyl)acetamide | $^1$H NMR (400 MHz, MeOD-*d$_4$*): δ 9.05 (s, 1H), 7.89-7.85 (m, 2H), 7.80-7.75 (m, 2H), 7.68 (d, *J* = 8.2 Hz, 2H), 7.61 (d, *J* = 8.1 Hz, 2H), 7.41 (d, *J* = 14.1 Hz, 2H), 4.08 (s, 3H), 3.98 (d, *J* = 5.7 Hz, 2H), 3.88 (s, 2H), 3.48-3.39 (m, 2H), 3.04 (td, *J* = 12.8, 3.1 Hz, 2H), 2.11-2.04 (m, 2H), 1.73-1.60 (m, 2H). 551.3 [M+H]+ | 1.32 |

(continued)

| Example | Structure | Compound Name | $^1$H NMR; MS(m/z) | UPLC r.t. (min) |
|---|---|---|---|---|
| 147 | 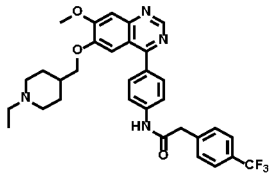 | *N*-(4-(6-((1-ethylpiperidin-4-yl) methoxy)-7-methoxyquinazolin-4-yl) phenyl)-2-(4-(trifluoromethyl) phe nyl) acetamide | $^1$H NMR (400 MHz, DMSO-$d_6$): δ 10.54 (s, 1H), 9.08 (s, 1H), 7.86-7.78 (m, 4H), 7.73 (d, *J* = 8.0 Hz, 2H), 7.60 (d, *J* = 8.0 Hz, 2H), 7.42 (s, 1H), 7.33 (s, 1H), 4.01 (s, 3H), 3.92-3.80 (m, 4H), 2.90 (d, *J* = 10.9 Hz, 2H), 2.33 (q, *J* = 7.3 Hz, 2H), 1.90 (q, *J* = 11.2, 8.1 Hz, 2H), 1.73 (d, *J* = 12.9 Hz, 2H), 1.30 (ddd, J = 23.4, 16.7, 9.5 Hz, 3H), 1.00 (t, *J* = 7.1 Hz, 3H). 579.3 [M+H]$^+$ | 1.33 |
| 148 | 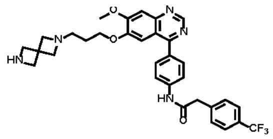 | *N*-(4-(6-(3-(2,6-diazaspiro[3.3] hepta ne-2-yl)propoxy)-7-methoxyquinazolin-4-yl) phenyl)-2-(4-(trifluoromethyl) phe nyl) acetamide | $^1$H NMR (400 MHz, DMSO-$d_6$): δ 10.63 (s, 1H), 9.08 (s, 1H), 7.87-7.76 (m, 4H), 7.72 (d, *J* = 8. 1 Hz, 2H), 7.60 (d, *J* = 8.0 Hz, 2H), 7.41 (s, 1H), 7.30 (s, 1H), 4.00 (s, 5H), 3.85 (s, 2H), 3.53 (s, 4H), 2.41 (t, *J* = 6.9 Hz, 2H), 1.85 (s, 5H), 1. 74-1. 66 (m, 2H). 592.3 [M+H]$^+$ | 1.26 |
| 149 | 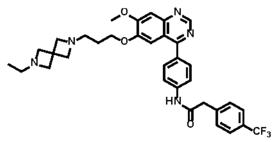 | *N*-(4-(6-(3-(6-ethyl-2,6-diazaspiro[3.3]hepta ne-2-yl) propoxy)-7-methoxyquinazolin-4-yl) phenyl)-2-(4-(trifluoromethyl) phe nyl) acetamide | $^1$H NMR (400 MHz, DMSO-$d_6$): δ 10.53 (s, 1H), 9.08 (s, 1H), 7.87-7.77 (m, 4H), 7.72 (d, *J* = 8.0 Hz, 2H), 7.60 (d, *J* = 8.0 Hz, 2H), 7.41 (s, 1H), 7.31 (s, 1H), 4.00 (s, 5H), 3.85 (s, 2H), 2.44 (t, *J* = 6.9 Hz, 2H), 2.37-2.30 (m, 2H), 1.91 (s, 5H), 1.72 (q, *J* = 6.7, 5.4 Hz, 2H), 1.43-1.08 (m, 2H), 0.89-0.79 (m, 4H). 620.4 [M+H]$^+$ | 1.23 |
| 150 | 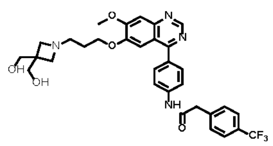 | *N*-(4-(6-(3-(3,3-bis (hydroxymethyl) az etidin-1-yl) propoxy)-7-methoxyquinazolin-4-yl) phenyl)-2-(4-(trifluoromethyl) phe nyl) acetamide | $^1$H NMR (400 MHz, DMSO-$d_6$): δ 10.54 (s, 1H), 9.15-9.00 (m, 1H), 7.88-7.67 (m, 6H), 7.60 (d, *J* = 7.8 Hz, 2H), 7.44 (s, 1H), 7.32 (s, 1H), 4.03 (d, *J* = 12.2 Hz, 5H), 3.85 (s, 2H), 2.88 (s, 2H), 2.09 (d, *J* = 2.8 Hz, 4H), 1.89 (d, *J* = 12.4 Hz, 6H). 611.4 [M+H]$^+$ | 1.38 |

(continued)

| Example | Structure | Compound Name | $^1$H NMR; MS(m/z) | UPLC r.t. (min) |
|---|---|---|---|---|
| 151 | 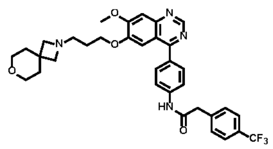 | *N*-(4-(6-(3-(7-oxa-2-azaspiro [3.5]nonane-2-yl)propoxy)-7-methoxyquinazolin-4-yl) phenyl)-2-(4-(trifluoromethyl) phe nyl) acetamide | $^1$H NMR (400 MHz, DMSO-$d_6$): δ 10.54 (s, 1H), 9.10 (s, 1H), 7.82 (q, *J* = 8.1 Hz, 4H), 7.73 (d, *J* = 7.8 Hz, 2H), 7.60 (d, *J* = 7.9 Hz, 2H), 7.44 (s, 1H), 7.32 (s, 1H), 4.03 (d, *J* = 17.4 Hz, 5H), 3.85 (s, 2H), 2.09 (s, 4H), 1.88 (d, *J* = 25.3 Hz, 6H), 1.65 (s, 4H), 1.25 (s, 1H), 0.85 (dt, *J* = 10.7, 6.3 Hz, 1H). 621.3 [M+H]$^+$ | 1.37 |
| 152 | 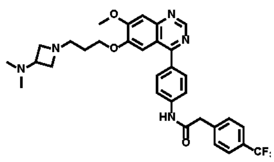 | *N*-(4-(6-(3-(3-(dimethylamino) azeti din-1-yl)propoxy)-7-methoxyquinazolin-4-yl) phenyl)-2-(4-(trifluoromethyl) phe nyl) acetamide | $^1$H NMR (400 MHz, DMSO-$d_6$): δ 10.55 (s, 1H), 9.11 (s, 1H), 7.89-7.67 (m, 6H), 7.60 (d, *J* = 7.8 Hz, 2H), 7.46 (s, 1H), 7.33 (s, 1H), 4.05 (d, *J* = 22.0 Hz, 6H), 3.85 (s, 2H), 2.10-2.08 (m, 8H), 1. 98-1. 90 (m, 4H), 1.25 (s, 1H), 0.85 (d, *J* = 10.0 Hz, 1H). 594.3 [M+H]$^+$ | 1.24 |
| 153 | 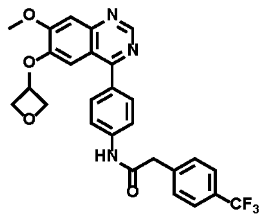 | *N*-(4-(7-methoxy-6-(oxetan-3-yloxy)quinazolin-4-yl)phenyl)-2-(4-(trifluoromethyl)phe nyl) acetamide | $^1$H NMR (400 MHz, DMSO-$d_6$): δ 10.59 (s, 1H), 9.11 (s, 1H), 7.85 (d, *J* = 8.2 Hz, 2H), 7.79-7.68 (m, 4H), 7.60 (d, *J* = 7.9 Hz, 2H), 7.48 (s, 1H), 6.93 (s, 1H), 5.42-5.31 (m, 1H), 4.82 (t, *J* = 7.0 Hz, 2H), 4.75-4.58 (m, 2H), 4.04 (s, 3H), 3.86 (s, 2H). 510.2 [M+H]$^+$ | 1.71 |
| 154 | 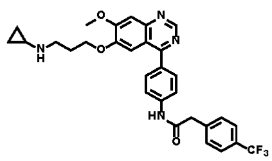 | *N*-(4-(6-(3-(cyclopropylamino) pr opoxy)-7-methoxyquinazolin-4-yl) phenyl)-2-(4-(trifluoromethyl) phe nyl)acetamide | $^1$H NMR (400 MHz, DMSO-$d_6$): δ 10.52 (d, *J* = 2.6 Hz, 1H), 9.09 (d, *J* = 2.6 Hz, 1H), 7.82 (qd, *J* = 8.8, 2.5 Hz, 4H), 7.72 (dd, *J* = 8.4, 2.5 Hz, 2H), 7.60 (dd, *J* = 8.4, 2.5 Hz, 2H), 7.42 (d, *J* = 2.7 Hz, 1H), 7.34 (d, *J* = 2.6 Hz, 1H), 4.07 (td, *J* = 6.5, 2.6 Hz, 2H), 4.01 (d, *J* = 2.6 Hz, 3H), 3.85 (s, 1H), 2.71 (dt, *J* = 9.0, 4.6 Hz, 2H), 2.03 (dq, *J* = 6.6, 3.3 Hz, 1H), 1.90 (d, *J* = 2.6 Hz, 4H), 0.32 (tq, *J* = 6.3, 3.7, 3.2 Hz, 2H), 0.19-0.11 (m, 2H). 551.3 [M+H]$^+$ | 1.41 |

(continued)

| Example | Structure | Compound Name | $^1$H NMR; MS(m/z) | UPLC r.t. (min) |
|---|---|---|---|---|
| 155 | 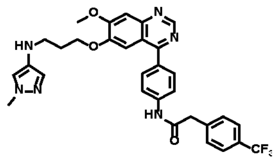 | *N*-(4-(7-methoxy-6-(3-((1-methyl-1H-pyrazol-4-yl) amino)propoxy) qui nazolin-4-yl)phenyl)-2-(4-(trifluoromethyl)phe nyl) acetamide | $^1$H NMR (400 MHz, DMSO-*d*$_6$): δ 10.57 (s, 1H), 9.09 (s, 1H), 7.81 (t, *J* = 9.1 Hz, 4H), 7.72 (d, *J* = 8.0 Hz, 2H), 7.60 (d, *J* = 7.8 Hz, 2H), 7.43 (s, 1H), 7.34 (s, 1H), 7.03 (s, 1H), 6.91 (s, 1H), 4.10 (d, *J* = 6.4 Hz, 2H), 4.02 (s, 3H), 3.85 (s, 2H), 3.65 (s, 3H), 2.97 (s, 2H), 1.98 (s, 2H). 591.3 [M+H]$^+$ | 1.44 |
| 156 | 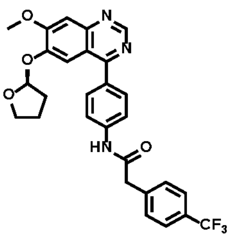 | (*S*)-*N*-(4-(7-methoxy-6-((tetrahydrofuran-2-yl)oxy) quinazolin-4-yl)phenyl)-2-(4-(trifluoromethyl)phe nyl) acetamide | $^1$H NMR (400 MHz, DMSO-*d*$_6$): δ 10.58 (s, 1H), 9.09 (d, *J* = 7.2 Hz, 1H), 7.93-7.65 (m, 6H), 7.60 (d, *J* = 7.9 Hz, 2H), 7.44 (s, 1H), 7.28 (s, 1H), 5.04 (s, 1H), 4.00 (s, 3H), 3.91-3.80 (m, 4H), 2.18 (q, *J* = 6.7 Hz, 1H), 1.76 (s, 1H), 1.27 (d, *J* = 18.1 Hz, 1H), 0.86 (t, *J* = 7.7 Hz, 1H). 524.3 [M+H]$^+$ | 1.61 |
| 157 | 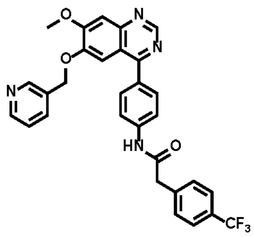 | *N*-(4-(7-methoxy-6-(pyridin-3-ylmethoxy)quinazolin -4-yl) phenyl)-2-(4-(trifluoromethyl) phe nyl)acetamide | $^1$H NMR (400 MHz, DMSO-*d*$_6$): δ 10.55 (s, 1H), 9.10 (s, 1H), 8.67 (dd, *J* = 2.3, 0.8 Hz, 1H), 8.58 (dd, *J* = 4.8, 1.7 Hz, 1H), 7.89-7.82 (m, 3H), 7.72 (td, *J* = 7.3, 1.4 Hz, 4H), 7.63-7.57 (m, 2H), 7.49-7.42 (m, 3H), 5.27 (s, 2H), 4.02 (s, 3H), 3.85 (s, 2H). 561.3 [M+H]$^+$ | 1.45 |
| 158 | 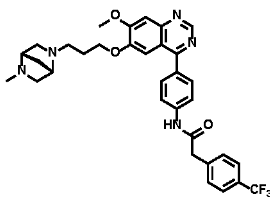 | *N*-(4-(7-methoxy-6-(3-((1*s*,4*s*)-5-methyl-2,5-diazabicyclo [2.2.1]h eptane-2-yl)propoxy) quinazoli n-4-yl)phenyl)-2-(4-(trifluoromethyl) phe nyl) acetamide | $^1$H NMR (400 MHz, MeOD-*d*$_4$): δ 9.10 (s, 1H), 7.87 (d, *J* = 8.2 Hz, 2H), 7.77 (d, *J* = 8.3 Hz, 2H), 7.68 (d, *J* = 8.1 Hz, 2H), 7.60 (d, *J* = 8.0 Hz, 2H), 7.41 (d, *J* = 9.6 Hz, 2H), 4.13 (t, *J* = 6.0 Hz, 2H), 4.09 (d, *J* = 4.9 Hz, 3H), 3.89 (d, *J* = 5.0 Hz, 2H), 3.87-3.82 (m, 1H), 3.74-3.68 (m, 1H), 3.03-2.92 (m, 3H), 2.87-2.80 (m, 1H), 2.71 (s, 2H), 2.26 (s, 3H), 2.03 (q, *J* = 4.9, 3.3 Hz, 4H), 1.35-1.29 (m, 1H). 606.4 [M+H]$^+$ | 1.16 |

(continued)

| Example | Structure | Compound Name | $^1$H NMR; MS(m/z) | UPLC r.t. (min) |
|---|---|---|---|---|
| 159 | 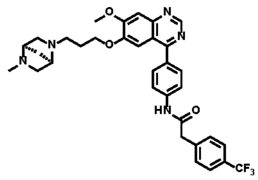 | *N*-(4-(7-methoxy-6-(3-((1R, 4R)-5-methyl-2,5-diazabicyclo [2.2.1]h eptane-2-yl)propoxy) quinazoli n-4-yl)phenyl)-2-(4-(trifluoromethyl)phenyl) acetamide | $^1$H NMR (400 MHz, MeOD-*d$_4$*): δ 9.03 (s, 1H), 7.87 (d, *J* = 8.4 Hz, 2H), 7.78-7.73 (m, 2H), 7.68 (d, *J* = 8.1 Hz, 2H), 7.60 (d, *J* = 8.1 Hz, 2H), 7.37 (d, *J* = 10.2 Hz, 2H), 4.12 (t, *J* = 6.0 Hz, 2H), 4.07 (d, *J* = 1.3 Hz, 3H), 3.89 (d, *J* = 10.3 Hz, 3H), 3.73 (d, *J* = 2.3 Hz, 1H), 3.06 (d, *J* = 11.8 Hz, 1H), 2.97 (ddd, *J* = 11.6, 8.9, 2.4 Hz, 3H), 2.82 (dt, *J* = 11.9, 7.0 Hz, 1H), 2.05 (d, *J* = 3.7 Hz, 3H), 1.96 (s, 4H), 1.33-1.28 (m, 1H). 606.4 [M+H]$^+$ | 1.16 |
| 160 | 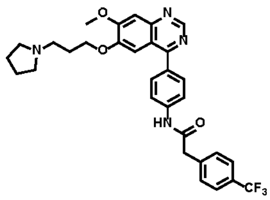 | *N*-(4-(7-methoxy-6-(3-(pyrrolidin-1-yl)propoxy) quinazoli n-4-yl)phenyl)-2-(4-(trifluoromethyl)phe nyl) acetamide | $^1$H NMR (400 MHz, DMSO-*d$_6$*): δ 10.52 (s, 1H), 9.08 (s, 1H), 7.87-7.77 (m, 4H), 7.72 (d, *J* = 8.1 Hz, 2H), 7.60 (d, *J* = 8.0 Hz, 2H), 7.42 (s, 1H), 7.33 (s, 1H), 4.07 (t, *J* = 6.4 Hz, 2H), 4.01 (s, 3H), 3.84 (s, 2H), 2.56 (s, 2H), 1.90 (s, 6H), 1. 69-1. 61 (m, 4H). 565.3 [M+H]$^+$ | 1.31 |
| 161 | 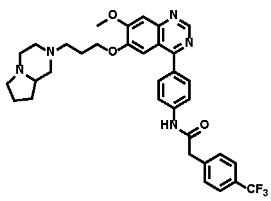 | *N*-(4-(6-(3-(hexahydropyrrolo [1, 2-a]pyrazin-2(1H)-yl) propoxy)-7-methoxyquinazolin-4-yl) phenyl)-2-(4-(trifluoromethyl) phe nyl) acetamide | $^1$H NMR (400 MHz, DMSO-*d$_6$*): δ 10.57 (s, 1H), 9.11 (s, 1H), 7.83 (q, *J* = 8.1, 7.7 Hz, 4H), 7.73 (d, *J* = 7.9 Hz, 2H), 7.60 (d, *J* = 7.9 Hz, 2H), 7.45 (s, 1H), 7.35 (s, 1H), 4.09 (t, *J* = 6.0 Hz, 2H), 4.02 (s, 3H), 3.85 (s, 3H), 2.86 (s, 5H), 2.00 (d, *J* = 70.5 Hz, 9H), 1.25 (s, 1H), 0.86 (s, 1H). 620.4 [M+H]$^+$ | 1.24 |
| 162 | 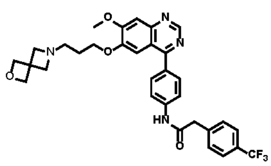 | *N*-(4-(6-(3-(2-oxa-6-azaspiro [3.3]heptane -6-yl)propoxy)-7-methoxyquinazolin-4-yl) phenyl)-2-(4-(trifluoromethyl) phe nyl)acetamide | $^1$H NMR (400 MHz, DMSO-*d$_6$*): δ 10.54 (s, 1H), 9.08 (s, 1H), 7.88-7.76 (m, 4H), 7.75-7.68 (m, 2H), 7.63-7.56 (m, 2H), 7.41 (s, 1H), 7.30 (s, 1H), 4.55 (s, 4H), 4.05-3.93 (m, 5H), 3.85 (s, 2H), 3.20 (s, 4H), 2.41 (t, *J* = 6.9 Hz, 2H), 1. 77-1. 66 (m, 2H) . 593.4 [M+H]$^+$ | 1.30 |

(continued)

| Example | Structure | Compound Name | $^1$H NMR; MS(m/z) | UPLC r.t. (min) |
|---|---|---|---|---|
| 163 | 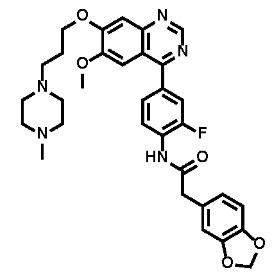 | 2-(benzo[*d*][1,3]dioxol -5-yl)-N-(2-fluoro-4-(6-methoxy-7-(3-(4-methylpiperazin-1-yl) propoxy)quinazoli n-4-yl) phenyl)acetamide | $^1$H NMR (400 MHz, $CDCl_3$): δ 9.15 (s, 1H), 8.56 (t, *J* = 8.2 Hz, 1H), 7.70 - 7.65 (m, 2H), 7.48 (dd, *J* = 7.7, 2.8 Hz, 2H), 7.38 (s, 1H), 6.91 - 6.79 (m, 3H), 6.01 (s, 2H), 4.29 (t, *J* = 6.6 Hz, 2H), 3.89 (s, 3H), 3.74 (s, 2H), 2.59 (t, *J* = 7.1 Hz, 10H), 2.33 (s, 3H), 2.15 - 2.10 (m, 2H). 588.2 [M+H]+ | 1.38 |
| 164 | 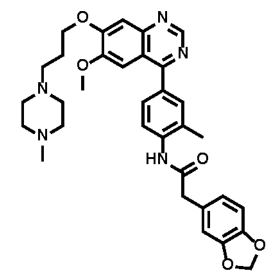 | 2-(benzo[*d*][1,3]dioxol -5-yl)-*N*-(4-(6-methoxy-7-(3-(4-methylpiperazin-1-yl)propoxy) quinazoli n-4-yl)-2-methylphenyl)acetami de | $^1$H NMR (400 MHz, $CDCl_3$): δ 9.14 (s, 1H), 8.24 (d, *J* = 8.7 Hz, 1H), 7.61 (d, *J* = 7.1 Hz, 2H), 7.35 (d, *J* = 16.8 Hz, 2H), 7.20 (s, 1H), 6.92 - 6.83 (m, 3H), 6.02 (s, 2H), 4.29 (t, *J* = 6.6 Hz, 2H), 3.87 (s, 3H), 3.75 (s, 2H), 2.62 - 2.36 (m, 10H), 2.30 (s, 3H), 2.13 (t, *J* = 7.0 Hz, 2H), 2.07 (s, 3H). 584.2 $[M+H]^+$ | 1.33 |

**<Example 165> Preparation of 4-(6-methoxy-7-(3-(4-methylpiperazin-1-yl)propoxy)quinazolin-4-yl)-N-(4-(trifluoromethyl)benzyl)benzamide**

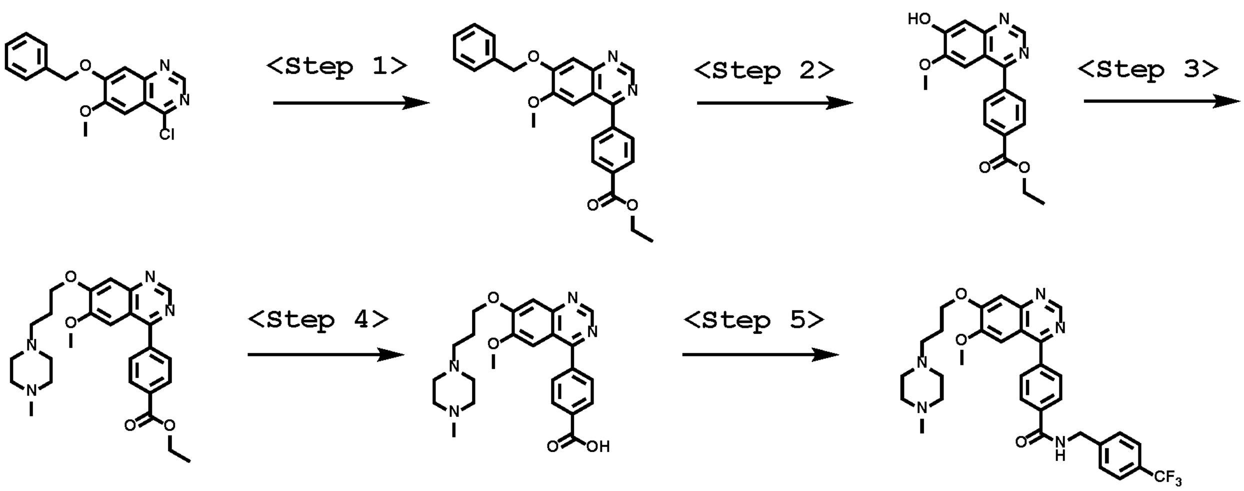


Step 1: After dissolving 7-(benzyloxy)-4-chloro-6-methoxyquinazoline (1g, 3.33mmol) and (4-(ethoxycarbonyl)phenyl)boronic acid (0.774g, 3.99mmol) in 1,4-dioxane (11.08ml) and 1M sodium carbonate (7.32ml), tetrakis(triphenylphosphine)palladium(0) (0.19g, 0.17mmol) was added dropwise. After bubbling with nitrogen, it was stirred at 80°C for 2.5 hours. After completion of the reaction, it was filtered through a celite filter, washed with methanol, concentrated under reduced pressure, and then separated and purifiedby MPLC (EA:HEX=0-80%), and the desired compound ethyl 4-(7-(benzyloxy)-6-methoxyquinazolin-4-yl)benzoate (850.0mg, yield 61.7%) was obtained.
MS(m/z): 414.9 $[M+H]^+$ UPLC r.t.(min): 2.07

Step 2: After dissolving ethyl 4-(7-(benzyloxy)-6-methoxyquinazolin-4-yl)benzoate (1.34g, 3.23mmol) obtained in the step 1 in trifluoroacetic acid (4.74ml), it was stirred overnight at 80°C. When the reaction was completed, after concentration under reduced pressure, the solid desired compound ethyl 4-(7-hydroxy-6-methoxyquinazolin-4-yl)benzoate 2,2,2-trifluoroacetic acid (0.9g, 90 %) was obtained. The following reaction was carried out without further purification.
MS(m/z): 325.0 $[M+H]^+$ UPLC r.t.(min): 1.57

Step 3: After dissolving ethyl 4-(7-hydroxy-6-methoxyquinazolin-4-yl)benzoate 2,2,2-trifluoroacetic acid (0.56g, 1.33mmol) obtained in the Step 2 and potassium carbonate (0.733g, 5.30mmol) in acetonitrile (3.79ml), N,N-diisopropylethylamine (0.857g, 6.63mmol) was added dropwise. After adding 1-(3-chloropropyl)-4-methylpiperazine dihydrochloride (0.993g, 3.98mmol) dropwise to the reaction mixture, it was stirred at 70°C overnight. Upon completion of the reaction, it was concentrated under reduced pressure and purified through MPLC(DCM:MeOH=0-20%) to obtain the desired compound ethyl 4-(6-methoxy-7-(3-(4-methylpiperazin-1-yl)propoxy)quinazolin-4-yl)benzoate (0.29g, yield 47.1%) was obtained.
MS(m/z): 465.3 $[M+H]^+$ UPLC r.t.(min): 1.32

Step 4: After dissolving ethyl 4-(6-methoxy-7-(3-(4-methylpiperazin-1-yl)propoxy)quinazolin-4-yl)benzoate (0.29g, 0.624mmol) obtained in the step 3 and lithium hydroxide hydrate (52.4mg, 1.248mmol) in methanol (1.24ml) and distilled water (1.24ml), it was stirred at room temperature overnight. After completion of the reaction, 1N hydrochloric acid and distilled water were added, and the organic matter was extracted with ethyl acetate(x3). After washing the collected organic layer with brine, the remaining water was removed using sodium sulfate, and concentrated under reduced pressure. After concentration, the desired compound 4-(6-methoxy-7-(3-(4-methylpiperazin-1-yl)propoxy)quinazolin-4-yl)benzoic acid (180mg, 66%) was obtained.
MS(m/z): 437.2 $[M+H]^+$ UPLC r.t.(min): 1.09

Step 5: After dissolving (4-(trifluoromethyl)phenyl)methanamine (108mg, 0.61mmol) and HATU (235.0mg, 0.61mmol) in dichloromethane (0.8ml), N,N-diisopropylethylamine (133mg, 1.03mmol) was added dropwise. 4-(6-methoxy-7-(3-(4-methylpiperazin-1-yl)propoxy)quinazolin-4-yl)benzoic acid (108mg, 0.619mmol) obtained in the step 4 was added to the reaction mixture, and then stirred at room temperature for 2 hours. After the completion of the reaction, distilled water was added, and then the organic matter was extracted with ethyl acetate(x3). After washing the collected organic layer with brine, the remaining water was removed using sodium sulfate, and concentrated under reduced pressure. After separating and purifying the concentrated mixture by MPLC (DCM:MeOH=0-20%), the desired compound 4-(6-methoxy-7-(3-(4-methylpiperazin-1-yl)propoxy)quinazolin-4-yl)-N-(4-(trifluoromethyl)benzyl)benzamide (21mg, 17.1 %) was obtained.
MS(m/z): 594.3 $[M+H]^+$ UPLC r.t.(min): 1.52

## Examples 165 to 167

Examples 166 to 167 were prepared in a similar manner to Example 165, and the compound names, NMR, mass, and UPLC analysis results of Examples 165 to 167 are summarized in Table 4 below.

[Table 4]

| Example | Structure | Compound Name | $^1H$ NMR; MS(m/z) | UPLC r.t. (min) |
|---|---|---|---|---|
| 165 | 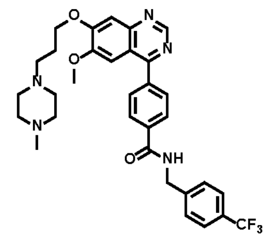 | 4-(6-methoxy-7-(3-(4-methylpiperazin-1-yl) propoxy)quinazoli n-4-yl)-N-(4-(trifluoromethyl) ben zyl) benzamide | $^1H$ NMR (400 MHz, DMSO-$d_6$) δ 9.39 (t, *J* = 6.0 Hz, 1H), 9.16 (s, 1H), 8.13 (d, *J* = 8.1 Hz, 2H), 7.95 (d, *J* = 8.1 Hz, 2H), 7.73 (d, *J* = 8.0 Hz, 2H), 7.59 (d, *J* = 8.0 Hz, 2H), 7.46 (s, 1H), 7.31 (s, 1H), 4.61 (d, *J* = 5.9 Hz, 2H), 4.28 (t, *J* = 6.4 Hz, 2H), 3.85 (s, 3H), 2.94 (d, *J* = 19.4 Hz, 2H), 2.88-2.72 (m, 8H), 2.61-2.55 (m, 3H), 2.02 (p, *J* = 6.4, 5.9 Hz, 2H).<br>594.3$[M+H]^+$ | 1.52 |

(continued)

| Example | Structure | Compound Name | $^1H$ NMR; MS(m/z) | UPLC r.t. (min) |
|---|---|---|---|---|
| 166 | 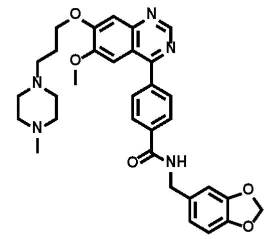 | *N*-(benzo[d][1,3]dioxol -5-ylmethyl)-4-(6-methoxy-7-(3-(4-methylpiperazin-1-yl)propoxy)quinazoli n-4-yl)benzamide | $^1H$ NMR (400 MHz, $CDCl_3$) δ 9.20 (s, 1H), 7.99 (d, *J* = 8.2 Hz, 2H), 7.85 (d, *J* = 8.2 Hz, 2H), 7.41 (s, 1H), 7.21 (s, 1H), 6.92-6.78 (m, 3H), 6.43 (t, *J* = 5.7 Hz, 1H), 5.98 (s, 2H), 4.61 (d, *J* = 5.6 Hz, 2H), 4.30 (t, *J* = 6.6 Hz, 2H), 3.87 (s, 3H), 2.59 (t, *J* = 7.2 Hz, 4H), 2.32 (s, 3H), 2.14 (t, *J* = 6.9 Hz, 2H), 1.25 (s, 3H), 0.92-0.80 (m, 2H). 570.1[M+H]$^+$ | 1.25 |
| 167 | 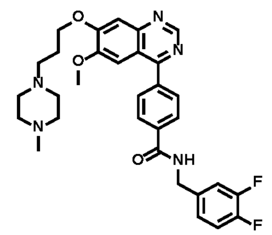 | *N*-(3,4-difluorobenzyl)-4-(6-methoxy-7-(3-(4-methylpiperazin-1-yl) propoxy)quinazoli n-4-yl) benzamide | $^1H$ NMR (400 MHz, $CDCl_3$) δ 9.19 (s, 1H), 8.03-7.97 (m, 2H), 7.88-7.82 (m, 2H), 7.41 (s, 1H), 7.24-7.07 (m, 3H), 6.63 (t, *J* = 5.9 Hz, 1H), 4.67 (d, *J* = 5.9 Hz, 2H), 4.30 (t, *J* = 6.6 Hz, 2H), 3.87 (s, 3H), 2.61 (t, *J* = 7.2 Hz, 4H), 2.38 (s, 3H), 2.14 (p, J = 6.8 Hz, 2H), 1.25 (s, 6H), 0.87 (dt, *J* = 8.2, 4.2 Hz, 2H). 562.2[M+H]$^+$ | 1.39 |

**<Example 168> Preparation of 1-(4-chloro-3-(trifluoromethyl)phenyl)-3-(4-(6-methoxy-7-(3-(4-methylpiperazin-1-yl)propoxy)quinazolin-4-yl)phenyl)urea**

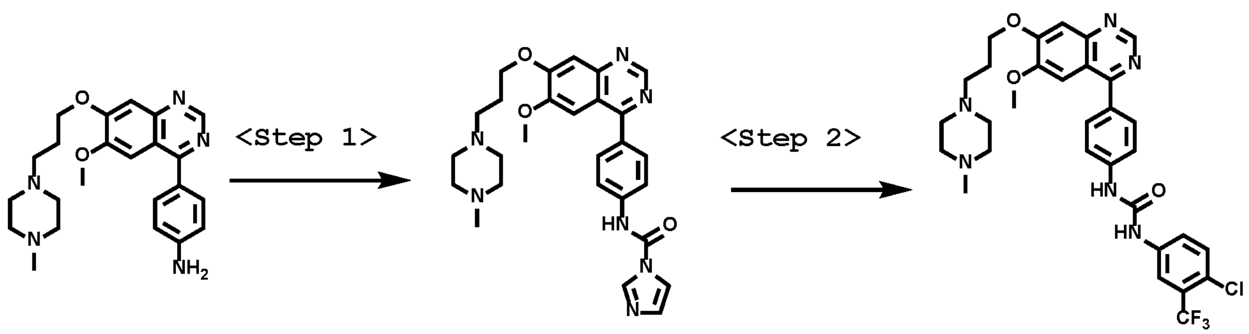


Step 1: After dissolving 4-(6-methoxy-7-(3-(4-methylpiperazin-1-yl)propoxy)quinazolin-4-yl)aniline (128mg, 0.314mmol) and 1,1'-carbonyldiimidazole(CDI) (102mg, 0.628mmol) in dichloromethane (0.8ml), *N,N*-diisopropylethylamine (60.9mg, 0.471mmol) was added dropwise. After stirring at room temperature for 1 hour and the end of the reaction, distilled water was added and the organic matter was extracted with dichloromethane(x3). After washing the collected organic layer with brine, the remaining water was removed with sodium sulfate, and concentrated under reduced pressure to obtain the desired compound N-(4-(6-methoxy-7-(3-(4-methylpiperazin-1-yl)propoxy)quinazolin-4-yl)phenyl)-1H-imidazole-1-carboxamide (136mg, 86%) was obtained. It was used in the following reaction without separate purification.
MS(m/z): 466.0 [M+H]$^+$ UPLC r.t.(min): 1.12

Step 2: After dissolving N-(4-(6-methoxy-7-(3-(4-methylpiperazin-1-yl)propoxy)quinazolin-4-yl)phenyl)-1H-imidazole-1-carboxamide (136mg, 0.271mmol) obtained in the step 1 in dichloromethane (0.8ml), N,N-diisopropylethylamine (60.9mg, 0.471mmol) and 4-chloro-3-(trifluoromethyl)aniline (80mg, 0.407mmol) was added dropwise. After stirring at 50°C for 1 hour and the completion of the reaction, distilled water was added and the organic matter was extracted with dichloromethane(x3). After washing the collected organic layer with brine, the remaining water was removed using

sodium sulfate, concentrated under reduced pressure, and the desired compound 1-(4-chloro-3-(trifluoromethyl)phenyl)-3-(4-(6-methoxy-7-(3-(4-methylpiperazin-1-yl)propoxy)quinazolin-4-yl)phenyl)urea (38.7mg, 22.6 %) was separated and purified using prep-HPLC.
MS(m/z): 629.2 [M+H]+ UPLC r.t.(min): 1.60

**Examples 168 to 169**

Example 169 was prepared in a similar manner to Example 168, and the compound names, NMR, mass, and UPLC analysis results of Examples 168 to 169 are summarized in Table 5 below.

[Table 5]

| Example | Structure | Compound Name | $^1$H NMR; MS(m/z) | UPLC r.t. (min) |
|---|---|---|---|---|
| 168 | 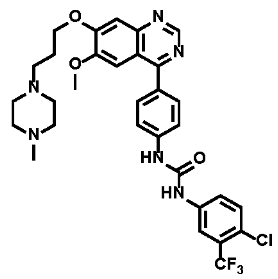 | 1-(4-chloro-3-(trifluoromethyl)phenyl)-3-(4-(6-methoxy-7-(3-(4-methylpiperazin-1-yl)propoxy)quinazoli n-4-yl)phenyl)urea | $^1$H NMR (400 MHz, DMSO-$d_6$) δ 9.39 (s, 1H), 9.28 (s, 1H), 9.09 (s, 1H), 8.17 (d, *J* = 2.4 Hz, 1H), 7.84 (d, *J* = 8.5 Hz, 2H), 7.76-7.62 (m, 4H), 7.41 (d, *J* = 1.5 Hz, 2H), 4.26 (t, *J* = 6.4 Hz, 2H), 3.88 (s, 3H), 2.65-2.52 (m, 6H), 2.25 (s, 4H), 1.99 (p, *J* = 6.7 Hz, 2H), 1.25 (q, *J* = 4.9, 3.8 Hz, 3H).<br>629.2 [M+H]+ | 1.60 |
| 169 | 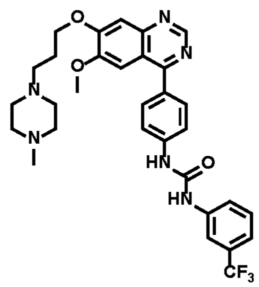 | 1-(4-(6-methoxy-7-(3-(4-methylpiperazin-1-yl) propoxy)quinazoli n-4-yl) phenyl)-3-(3-(trifluoromethyl)phenyl)urea | $^1$H NMR (400 MHz, DMSO-$d_6$) δ 9.21 (s, 1H), 9.17 (s, 1H), 9.09 (s, 1H), 8.07 (d, *J* = 2.0 Hz, 1H), 7.87-7.80 (m, 2H), 7.76-7.69 (m, 2H), 7.62 (d, *J* = 8.4 Hz, 1H), 7.55 (t, *J* = 7.9 Hz, 1H), 7.41 (s, 2H), 7.35 (d, *J* = 7.7 Hz, 1H), 4.26 (t, *J* = 6.4 Hz, 2H), 3.88 (s, 3H), 2.53 (d, *J* = 1.9 Hz, 6H), 2.21 (s, 4H), 1.98 (t, *J* =6.9 Hz, 2H), 1.24 (s, 3H).<br>595.2[M+H]+ | 1.50 |

**Experimental Example 1: Evaluation of enzyme inhibitory activity for PDGFR-α, PDGFR-β, c-KIT kinase**

In order to evaluate the inhibitory activity of the compounds synthesized according to the above examples on PDGFR-α(wt), PDGFR-β(wt), c-KIT(wt), and c-KIT(D816V) kinases, the following method was performed. The compounds of examples were reacted with purified human PDGFR-α(wt) (Signalchem #P12-18G) enzyme, human PDGFR-β(wt) (Signalchem #P13-11G) enzyme, human c-KIT(wt) (Signalchem #K06-11BG) enzyme, human c-KIT(D816V) (Signalchem #C06-12LG) enzyme, and the enzyme inhibitory abilities were evaluated by the following method.

The reaction buffer was used as a composition of 40mM Tris-HCl pH7.4, 20mM $MgCl_2$, 0.5mg/ml BSA, 50μM DTT, and the reactions of all the tests were carried out on the reaction buffer. The compound was diluted in 12 steps using a serial dilution method from a 10mM DMSO stock, and the enzyme activity was measured at a final compound concentration of 10-0.00005645 μM. During the test, the enzyme activity was confirmed using *in vitro* ADP-Glo™ kinase assay(Promega) after reacting with the proper concentration of human PDGFR-α(wt) enzyme, human PDGFR-β(wt), human c-KIT(wt) enzyme, and human c-KIT(D816V) enzyme with purified ATP and enzyme substrate at 25 °C for 1~2 hours. At a ratio of 2:2:1, an enzyme-active reaction solution, an ADP-Glo reaction solution, and an enzyme-active detection solution were reacted to measure the inhibition of the enzyme's activity with luminescence. Based on the fluorescence of the enzyme activity of the solvent control that was not treated with the compound, the degree of enzyme activity inhibition was calculated according to the treatment concentration of each compound, and the concentration of each compound that inhibits 50% of enzyme activity was determined as $IC_{50}$ (nM) value and the value was obtained by using GraphPad Prism 8.3.0 (GraphPad software Inc., San Diego) software. The results are shown in Table 6 below.

(A: $IC_{50} \leq 50nM$; B: $50nM < IC_{50} \leq 100nM$; C: $100nM < IC_{50} \leq 500nM$; D: $IC_{50} > 500nM$) .

[Table 6]

| Example | PDGFR-α | PDGFR-β | c-KIT (WT) | c-KIT (D816V) | Example | PDGFR-α | PDGFR-β | c-KIT (WT) | c-KIT (D816V) |
|---|---|---|---|---|---|---|---|---|---|
| 1 | A | A | A | A | 2 | A | A | A | A |
| 3 | A | A | A | A | 4 | - | A | - | - |
| 9 | A | A | A | A | 10 | A | C | - | - |
| 11 | A | A | A | A | 30 | - | A | - | - |
| 31 | - | A | - | - | 32 | - | A | - | - |
| 37 | - | A | - | - | 40 | - | A | - | - |
| 43 | - | A | - | - | 48 | - | A | - | - |
| 49 | - | A | - | - | 50 | - | A | - | - |
| 51 | - | A | - | - | 54 | - | A | - | - |
| 56 | - | A | - | - | 57 | A | A | A | A |
| 58 | A | A | - | - | 59 | - | A | - | - |
| 63 | - | A | - | - | 64 | - | A | - | - |
| 69 | - | A | - | - | 70 | - | A | - | - |
| 72 | A | A | - | - | 74 | - | A | - | - |
| 75 | - | A | - | - | 76 | - | A | - | - |
| 81 | - | A | - | - | 82 | - | A | - | - |
| 83 | A | A | - | - | 84 | A | A | - | - |
| 85 | A | A | - | - | 89 | A | A | - | - |
| 90 | A | A | - | - | 96 | - | A | - | - |
| 97 | - | A | - | - | 98 | - | A | - | - |
| 99 | - | A | - | - | 100 | - | A | - | - |
| 101 | - | A | - | - | 109 | - | A | - | - |
| 110 | - | A | - | - | 113 | - | A | - | - |
| 115 | - | A | - | - | 116 | - | A | - | - |
| 121 | - | A | - | - | 122 | A | B | A | A |
| 124 | A | C | - | - | 129 | - | A | - | - |
| 130 | - | A | - | - | 131 | - | A | - | - |
| 137 | - | A | - | - | 140 | A | A | - | - |
| 142 | - | A | - | - | 147 | A | A | - | - |
| 149 | A | A | - | - | 150 | - | A | - | - |
| 151 | A | A | - | - | 158 | - | A | - | - |
| 161 | - | A | - | - | 163 | - | A | - | - |
| 164 | - | A | - | - | 165 | - | A | - | - |
| 166 | - | A | - | - | 167 | - | A | - | - |
| 168 | - | B | - | - | | | | | |

As shown in the Table 6, it was confirmed that the compounds of the present disclosure have effects of inhibiting the activity of PDGFR-α, PDGFR-β, c-KIT(wt), and c-KIT(D816V) kinases. These indicate that the compounds of the present disclosure may be usefully used for preventing or treating diseases related to PDGFR-α, PDGFR-β, c-KIT(wt), or c-KIT(D816V).

## Experimental Example 2: Evaluation of proliferation inhibitory activity on Ba/F3 cell

Ba/F3-TEL-PDGFRα(WT), Ba/F3-TEL-PDGFRβ(WT), Ba/F3-c-KIT(D816V) (Kyinno, #KC-0138), c-KIT V560G/D816V, c-KIT D814Y were seeded at $3\times10^3$/100 μl/well in a clear bottom white 96-well plate. A culture solution containing compounds of 12 concentrations (0.00001-2 mM) serially diluted in 3 folds and the DMSO control group was added at 0.5 μl/well each to a final concentration of 0.00005-10 μM, and then incubated in a $CO_2$ incubator at 37 °C for 72 hours. After 72 hours, the plate treated with the compound was taken out, CellTiter-Glo® Assay(Promega) solution was treated with 100 μl/well, followed by mixing well. After mixing well for about 10 minutes at room temperature, fluorescence was measured using a microplate reader. The data were presented as percentages in proportion to treated cells based on the vehicle, $GI_{50}$ (nM) values were calculated using GraphPad Prism 8.3.0(GraphPad software Inc., San Diego).

The inhibitory activities of Ba/F3 expressing PDGFR-α, PDGFR-β, c-KIT(D816V), c-KIT V560G/D816V, and c-KIT D814Y measured as described above were measured and shown in Table 7 below (A: $GI_{50} \leq$ 200nM; B: 200nM $< GI_{50} \leq$ 500nM; C: $GI_{50} >$ 500nM) .

[Table 7]

| Exampl e | Ba/F3(nM) | | | Cell (nM) | | Exampl e | Ba/F3(nM) | | | Cell (nM) | |
|---|---|---|---|---|---|---|---|---|---|---|---|
| | Tel-PDGFRα | Tel-PDGFRβ | c-KIT (D816V ) | c-KIT V560G/ D816V | c-KIT D814Y | | Tel-PDGFRα | Tel-PDGFRβ | c-KIT (D816V ) | c-KIT V560G/ D816V | c-KIT D814Y |
| 1 | A | A | A | A | | 2 | A | A | A | B | |
| 3 | A | A | A | A | A | 4 | | A | A | A | A |
| 9 | A | A | A | A | A | 10 | A | B | A | C | |
| 11 | A | A | A | A | A | 14 | | | C | | |
| 15 | | | | C | | 16 | | | A | | |
| 17 | | | | A | | 18 | | A | A | A | A |
| 19 | | | A | | | 20 | | | C | | |
| 21 | | | A | | | 22 | | | A | A | A |
| 23 | | | A | B | | 24 | | | A | C | |
| 25 | | | A | A | | 26 | | | A | | |
| 27 | | | A | C | | 28 | | | A | C | |
| 29 | | | A | A | | 30 | | | A | A | A |
| 31 | | | A | A | A | 32 | | | A | A | |
| 33 | | | A | A | | 34 | | | A | A | |
| 35 | | | A | C | | 36 | | | A | C | |
| 37 | | | A | A | | 38 | | | A | | |
| 39 | | | A | B | | 40 | | | A | A | A |
| 41 | | | A | A | | 42 | | | A | A | |
| 43 | | | A | A | A | 44 | | | A | A | |
| 45 | | | | | | 48 | | A | | | |
| 49 | | A | | | | 50 | | A | | | |
| 51 | | A | | | | 52 | | A | | | |
| 53 | | A | | | | 54 | | A | | | |
| 55 | | | A | A | | 56 | | | | A | |

(continued)

| Exampl e | Ba/F3(nM) | | | Cell (nM) | | Exampl e | Ba/F3(nM) | | | Cell (nM) | |
|---|---|---|---|---|---|---|---|---|---|---|---|
| | Tel-PDGFRα | Tel-PDGFRβ | c-KIT (D816V ) | c-KIT V560G/ D816V | c-KIT D814Y | | Tel-PDGFRα | Tel-PDGFRβ | c-KIT (D816V ) | c-KIT V560G/ D816V | c-KIT D814Y |
| 57 | A | A | | A | | 58 | A | A | A | C | |
| 59 | | | B | | | 60 | | | A | B | |
| 61 | | | C | | | 62 | | | C | | |
| 63 | | | A | A | | 64 | | | A | A | |
| 65 | | | A | | | 66 | | | A | | |
| 67 | | | A | | | 68 | | | A | | |
| 69 | | | A | | | 70 | | | A | A | |
| 71 | | | A | B | | 72 | A | A | A | A | |
| 73 | | | A | A | | 74 | | | A | A | |
| 75 | | | A | A | | 76 | | | A | A | |
| 77 | | | A | B | | 78 | | | A | A | |
| 79 | | | A | | | 80 | | | A | A | |
| 81 | | | A | A | | 82 | | | A | A | |
| 83 | A | A | A | A | | 84 | A | A | A | A | |
| 85 | A | A | A | B | | 86 | | | C | | |
| 87 | | | A | | | 88 | | | | B | |
| 89 | A | A | | A | | 90 | A | A | | A | |
| 91 | | | | B | | 92 | | | | B | |
| 93 | | | | B | | 94 | | | | C | |
| 95 | | | | C | | 96 | | | | A | |
| 97 | | A | | C | | 98 | | A | | A | |
| 99 | | A | | A | | 100 | | A | | A | |
| 101 | | | | A | | 102 | | | | A | |

(continued)

| Exampl e | Ba/F3(nM) | | | Cell (nM) | | Exampl e | Ba/F3(nM) | | | Cell (nM) | |
|---|---|---|---|---|---|---|---|---|---|---|---|
| | Tel-PDGFRα | Tel-PDGFRβ | c-KIT (D816V ) | c-KIT V560G/ D816V | c-KIT D814Y | | Tel-PDGFRα | Tel-PDGFRβ | c-KIT (D816V ) | c-KIT V560G/ D816V | c-KIT D814Y |
| 103 | | | | B | | 104 | | | | B | |
| 105 | | | | C | | 106 | | | | C | |
| 107 | | | | C | | 108 | | | | C | |
| 109 | | A | | A | | 110 | | A | | A | |
| 111 | | | | C | | 112 | | | | C | |
| 113 | | A | | A | | 114 | | | | B | |
| 115 | | A | | A | | 116 | | A | | A | |
| 117 | | | | A | | 118 | | A | | A | |
| 119 | | | | C | | 120 | | | | C | |
| 121 | | A | | A | | 122 | A | A | | | |
| 123 | | | A | A | | 124 | A | A | | | |
| 125 | | | A | C | C | 126 | | | A | A | A |
| 127 | | | A | B | | 128 | | | A | | |
| 129 | | | A | B | | 130 | | | B | | |
| 131 | | | A | B | B | 132 | | A | A | C | |
| 133 | | | A | B | C | 134 | | | A | A | A |
| 135 | | | B | | | 136 | | | A | | |
| 137 | | | B | | | 138 | | | A | B | A |
| 139 | | | A | C | | 140 | A | A | A | C | C |
| 141 | | | A | A | | 142 | | A | A | A | |
| 143 | | | A | A | | 144 | | | A | B | A |
| 145 | | | A | B | A | 146 | | | C | | |
| 147 | A | A | A | | | 148 | | | C | | |

(continued)

| Exampl e | Ba/F3(nM) | | | Cell (nM) | | Exampl e | Ba/F3(nM) | | | Cell (nM) | |
|---|---|---|---|---|---|---|---|---|---|---|---|
| | Tel-PDGFRα | Tel-PDGFRβ | c-KIT (D816V ) | c-KIT V560G/ D816V | c-KIT D814Y | | Tel-PDGFRα | Tel-PDGFRβ | c-KIT (D816V ) | c-KIT V560G/ D816V | c-KIT D814Y |
| 149 | A | A | C | | | 150 | | | C | | |
| 151 | A | A | A | | | 152 | | | A | | |
| 153 | | | A | | | 154 | | | A | | |
| 155 | | | A | | | 156 | | | A | B | |
| 157 | | | A | C | | 158 | | A | A | B | |
| 159 | | | A | B | | 160 | | | A | A | |
| 161 | | A | A | A | | 163 | | A | | | |
| 164 | | A | | | | 165 | | A | | | |
| 166 | | A | | | | 167 | | A | | | |
| 169 | | A | | | | | | | | | |

As shown in Table 7, the compounds of the present disclosure showed activity of inhibiting the proliferation of over-activated cell lines of PDGFR-$\alpha$, PDGFR-$\beta$, c-KIT(D816V), c-KIT V560G/D816V, and c-KIT D814Y.

**Experimental Example 3: Evaluation of inhibitory activity against various kinases**

The activities of the compounds according to the present disclosure on various enzymes were evaluated. Specifically, it was commissioned DiscoverX to measure enzyme selectivity for the compounds of Examples 3, 9, and 83 above, and the experiment was conducted using a panel for scanMAX™ Kinase analysis. Herein, a solution prepared by dissolving in DMSO at a concentration of 1 $\mu$M was used as the drug treated with the enzyme, and the control percentage (%control) was determined by the method of Equation 1 below, and the results are shown in Table 8 below.

[Equation 1]

$$\text{Control Percentage}(\%\text{control}) = \frac{(\text{The Example compound} - \text{positive control})}{(\text{negative control} - \text{positive control})} \text{ X } 100$$

In the equation, the positive control group and the negative control group mean a compound exhiting control percentage of 0% and DMSO exhibiting control percentage of 100%, respectively. Herein, when the calculated percentage value for each enzyme was less than 35%, it was determined to have inhibitory activity for the enzyme. Accordingly, among the series of enzymes tested, the enzymes showing the inhibitory activities of the compounds of the present disclosure are summarized and shown in Table 8 below along with the control percentages thereof. (◎: less than 10%; O: 10% or more to less than 350).

[Table 8]

| Kinase | Example | | | Kinase | Example | | |
|---|---|---|---|---|---|---|---|
| | 3 | 9 | 83 | | 3 | 9 | 83 |
| KIT | ◎ | ◎ | ◎ | KIT (A829P) | ◎ | ◎ | ◎ |
| KIT(D816H) | ◎ | ◎ | ◎ | KIT (D816V) | ◎ | ◎ | ◎ |
| KIT(L576P) | ◎ | ○ | ◎ | KIT(V559D) | ◎ | ○ | ◎ |
| KIT(V559D,T670I) | ○ | | ○ | KIT(V559D,V654A) | ◎ | | ○ |
| KIT-autoinhibited | ○ | | | PDGFRA | ◎ | | ◎ |
| PDGFRB | ◎ | | ◎ | | | | |

Since a specific part of the present disclosure has been described in detail above, it will be clear to those skilled in the art that the specific technology is merely a preferred embodiment, and the scope of the present disclosure is not limited thereby. Accordingly, the substantial scope of the present disclosure will be defined by the appended claims and their equivalents.

## Claims

1. A compound represented by the Chemical Formula 1 below, a stereoisomer thereof, or a pharmaceutically acceptable salt thereof:

[Chemical Formula 1]

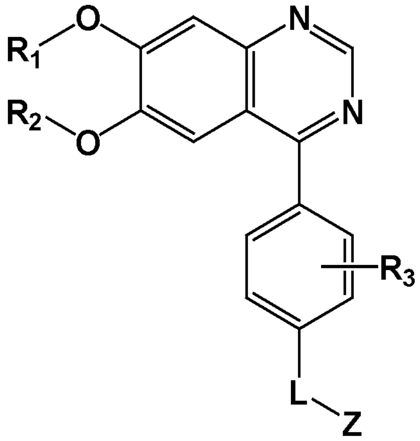

in the Chemical Formula 1,

$R_1$ and $R_2$ are each independently -H, -(C1-C6)alkyl, $-(CH_2)_m-R_x$, or $-(C=O)-R_X$ {wherein, $R_1$ and $R_2$ cannot be methyl at the same time};
$R_X$ is -(C1-C6)alkoxy, $-NR^AR^B$, -halo, cycloalkyl, heterocycloalkyl, aryl, heteroaryl, or heterohydroaryl {wherein at least one H in the cycloalkyl or heterocycloalkyl ring may be substituted with -(C1-C6)alkyl, - (C1-C6) hydroxyalkyl, -(C1-C6)alkyl-0-(C1-C6)alkyl, -(C=O)-(C1-C6)alkyl, -(C=O)-O-(C1-C6)alkyl, $-NR^CR^D$, -(=O)-, cycloalkyl, or benzyl, [wherein, at least one H in the benzyl ring may be substituted with -(C1-C6)alkyl, -(C1-C6)alkoxy, -(C1-C6)haloalkyl, or halo]; at least one H in the aryl, heteroaryl, or heterohydroaryl ring may be substituted with -(C1-C6)alkyl, -(C1-C6)haloalkyl, or halo};
$R^A$ and $R^B$ are each independently -H, -(C1-C6)alkyl, cycloalkyl, heterocycloalkyl, aryl, or heteroaryl {wherein, at the least one H in the cycloalkyl, heterocycloalkyl, aryl, or heteroaryl ring may be substituted with -(C1-C6)alkyl};
$R^C$ and $R^D$ are each independently -H or -(C1-C6)alkyl;
m is 0, 1, 2, 3, or 4;
$R_3$ is -H, -(C1-C6)alkyl, -(C1-C6)alkoxy, or -halo;
L is $-NH(C=O)-L_1-(CH_2)_n-L_2-$, $-NH-S(=O)_2-L_1-(CH_2)_n-L_2-$, $-(C=O)NH-L_1-(CH_2)_n-L_2-$, $-L_1-(CH_2)_n-L_2-(C=O)NH-$, or -NH(C=O)NH-;
$L_1$ and $L_2$ are each independently $-CR^ER^F-$, -C(=O)NH-, or null;
$R^E$ and $R^E$ are each independently -H, -(C1-C6)alkyl, $-NH_2$, - NH(C=0)0-(C1-C6)alkyl, -OH, or -halo, or $R^E$ and $R^E$ are combined with carbon to form -(C3-C6)cycloalkyl;
n is 0, 1, 2, 3, or 4;
Z is aryl, heteroaryl, heterohydroaryl, cycloalkyl, heterocycloalkyl, or -(C2-C6)alkenyl {wherein, at least one H in the aryl, heteroaryl, or heterohydroaryl ring may be substituted with -(C1-C6)alkyl, -(C1-C6) haloalkyl, -(C1-C6) hydroxyalkyl, - (C1-C6)cyanoalkyl, -CN, $-NR^GR^H$, -(C1-C6)alkoxy, or -halo; at least one H in the cycloalkyl or heterocycloalkyl ring may be substituted with -(C1-C6)alkyl};
$R^G$ and $R^H$ are each independently H or -(C1-C6)alkyl.

**2.** The compound represented by Chemical Formula 1, the stereoisomer thereof, or the pharmaceutically acceptable salt thereof of claim 1, wherein

$R_1$ and $R_2$ are each independently -H, -(C1-C6)alkyl, $-(CH_2)_m-R_X$, or $-(C=O)-R_x$ {wherein, $R_1$ and $R_2$ cannot be methyl at the same time};
$R_x$ is -(C1-C6)alkoxy, $-NR^AR^B$, -halo, heterocycloalkyl, aryl, heteroaryl, or heterohydroaryl {wherein, at least one H in the heterocycloalkyl ring may be substituted with -(C1-C6) alkyl, - (C1-C6)hydroxyalkyl, -(C1-C6)alkyl-O-(C1-C6)alkyl, -(C=0)-(C1-C6)alkyl, -(C=O) -O-(C1-C6) alkyl, $-NR^CR^D$, -(=O)-, cycloalkyl, or benzyl [wherein, at least one H in the benzyl ring may be substituted with -(C1-C6)alkyl, -(C1-C6)alkoxy, -(C1-C6)haloalkyl, or halo]; at least one H in the aryl, heteroaryl, or heterohydroaryl ring may be substituted with -(C1-C6)haloalkyl};
$R^A$ and $R^B$ are each independently -H, -(C1-C6)alkyl, cycloalkyl, or heteroaryl {wherein, at least one H in the cycloalkyl or heteroaryl ring may be substituted with -(C1-C6)alkyl};
$R^C$ and $R^D$ are each independently -H or -(C1-C6)alkyl;

m is 0, 1, 2, 3, or 4;
$R_3$ is -H, -(C1-C6)alkyl, or -halo;
L is $-NH(C=O)-L_1-(CH_2)_n-L_2-$, $-NH-S(=O)_2-L_1-(CH_2)_n-L_2-$, $-(C=O)NH-L_1-(CH_2)_n-L_2-$, or -NH (C=O) NH-;
$L_1$ and $L_2$ are each independently $-CR^ER^F-$ or null;
$R^E$ and $R^F$ are each independently -H, -(C1-C6)alkyl, or $-NH_2$, or $R^E$ and $R^F$ are combined with carbon to form -(C3-C6)cycloalkyl;
n is 0, 1, 2, 3, or 4;
Z is phenyl, 5-10 membered heteroaryl, 5-10 membered heterohydroaryl, 5-10 membered heterocycloalkyl, or -(C2-C6)alkenyl {wherein, at least one H in the phenyl, 5-10 membered heteroaryl, or 5-10 membered heterohydroaryl ring may be substituted with -(C1-C6)alkyl, -(C1-C6)haloalkyl, -CN, -(C1-C6)alkoxy, or -halo; at least one H in the 5-10 membered heterocycloalkyl ring may be substituted with -(C1-C6)alkyl}.

3. The compound represented by Chemical Formula 1, the stereoisomer thereof, or the pharmaceutically acceptable salt thereof of claim 1, wherein

$R_1$ and $R_2$ are each independently -H, -(C1-C6)alkyl, $-(CH_2)_m-R_x$, or $-(C=O)-R_x$ {wherein, $R_1$ and $R_2$ cannot be methyl at the same time};
$R_x$ is -(C1-C6)alkoxy, $-NR^AR^B$, -halo, heterocycloalkyl, aryl, heteroaryl, or heterohydroaryl {wherein, the heterocycloalkyl is a monocyclic ring, a spiro ring, a bridged ring, or a fused ring, at least one H in the heterocycloalkyl ring may be substituted with -(C1-C6)alkyl, -(C1-C6)hydroxyalkyl, -(C1-C6)alkyl-0-(C1-C6)alkyl, -(C=O)-(C1-C6)alkyl, -(C=O)-O-(C1-C6)alkyl, $-NR^CR^D$, -(=O)-, cycloalkyl, or benzyl [wherein, at least one H in the benzyl ring may be substituted with -(C1-C6)alkyl, -(C1-C6)alkoxy, -(C1-C6)haloalkyl, or halo]; at least one H in the aryl, heteroaryl, or heterohydroaryl ring may be substituted with -(C1-C6)haloalkyl};
$R^A$ and $R^B$ are each independently -H, -(C1-C6)alkyl, cycloalkyl, or heteroaryl {wherein, at least one H in the cycloalkyl or heteroaryl ring may be substituted with -(C1-C6)alkyl};
$R^C$ and $R^D$ are each independently -H or -(C1-C6)alkyl;
m is 0, 1, 2, or 3.

4. The compound represented by Chemical Formula 1, the stereoisomer thereof, or the pharmaceutically acceptable salt thereof of claim 1, wherein the compound represented by Chemical Formula 1 is represented by Chemical Formula 2 below:

[Chemical Formula 2]

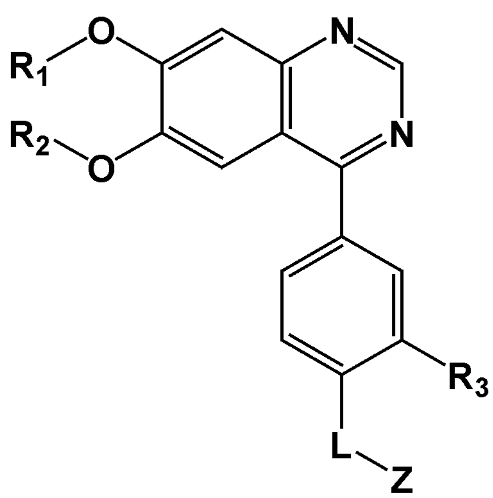

In Chemical Formula 2,
$R_3$ is -H, -(C1-C6)alkyl, or -halo.

5. The compound represented by Chemical Formula 1, the stereoisomer thereof, or the pharmaceutically acceptable salt thereof of claim 1, wherein

L is $-NH(C=O)-L_1-(CH_2)_n-L_2-$, $-NH-S(=O)_2-L_1-(CH_2)_n-L_2-$, $-(C=O)NH-L_1-(CH_2)_n-L_2-$, or -NH (C=O) NH-;
$L_1$ and $L_2$ are each independently $-CR^ER^F-$ or null;
$R^E$ and $R^F$ are each independently -H, -(C1-C6)alkyl, or $-NH_2$, or $R^E$ and $R^F$ are combined with carbon to form -(C3-C6)cycloalkyl;
n is 0, 1, 2, or 3.

**6.** The compound represented by Chemical Formula 1, the stereoisomer thereof, or the pharmaceutically acceptable salt thereof of claim 1, wherein
Z is phenyl, 5-10 membered heteroaryl, 5-10 membered heterohydroaryl, 5-10 membered heterocycloalkyl, or -(C2-C6)alkenyl {wherein, at least one H in the phenyl, 5-10 membered heteroaryl, or 5-10 membered heterohydroaryl ring may be substituted with -(C1-C6)alkyl, -(C1-C6)haloalkyl, -CN, -(C1-C6)alkoxy, or -halo; at least one H in the 5-10 membered heterocycloalkyl ring may be substituted with -(C1-C6)alkyl}.

**7.** The compound represented by Chemical Formula 1, the stereoisomer thereof, or the pharmaceutically acceptable salt thereof of claim 1, wherein the compound represented by the Chemical Formula 1 is selected from the group consisting of the following compounds:

(1) *N*-(4-(7-(2-(diethylamino)ethoxy)-6-methoxyquinazolin-4-yl)phenyl)-2-(4-(trifluoromethyl)phenyl)acetamide;
(2) *N*-(4-(6-methoxy-7-((1-methylpiperidin-4-yl)oxy)quinazolin-4-yl)phenyl)-2-(4-(trifluoromethyl)phenyl)acetamide;
(3) *N*-(4-(6-methoxy-7-(2-(4-methylpiperazin-1-yl)ethoxy)quinazolin-4-yl)phenyl)-2-(4-(trifluoromethyl)phenyl)acetamide;
(4) *N*-(4-(7-((1-ethylpiperidin-4-yl)methoxy)-6-methoxyquinazolin-4-yl)phenyl)-2-(4-(trifluoromethyl)phenyl)acetamide;
(5) *N*-(4-(7-(benzyloxy)-6-methoxyquinazolin-4-yl)phenyl)-2-(4-(trifluoromethyl)phenyl)acetamide;
(6) *N*-(4-(7-hydroxy-6-methoxyquinazolin-4-yl)phenyl)-2-(4-(trifluoromethyl)phenyl)acetamide;
(7) 2-amino-*N*-(4-(7-(benzyloxy)-6-methoxyquinazolin-4-yl)phenyl)-2-(4-fluorophenyl)propanamide;
(8) 2-amino-2-(4-fluorophenyl)-*N*-(4-(7-hydroxy-6-methoxyquinazolin-4-yl)phenyl)propanamide;
(9) *N*-(4-(6-methoxy-7-(3-(4-methylpiperazin-1-yl)propoxy)quinazolin-4-yl)phenyl)-2-(4-(trifluoromethyl)phenyl)acetamide;
(10) 2-amino-2-(4-fluorophenyl)-*N*-(4-(7-isobutoxy-6-methoxyquinazolin-4-yl)phenyl)propanamide;
(11) *N*-(4-(7-(3-(diethylamino)propoxy)-6-methoxyquinazolin-4-yl)phenyl)-2-(4-(trifluoromethyl)phenyl)acetamide;
(12) *N*-(4-(7-(benzyloxy)-6-methoxyquinazolin-4-yl)phenyl)-2-methyl-2-(4-(trifluoromethyl)phenyl)propanamide;
(13) *N*-(4-(7-hydroxy-6-methoxyquinazolin-4-yl)phenyl)-2-methyl-2-(4-(trifluoromethyl)phenyl)propanamide;
(14) *tert*-butyl 3-(((6-methoxy-4-(4-(2-(4-(trifluoromethyl)phenyl)acetamido)phenyl)quinazolin-7-yl)oxy)methyl)piperidin-1-carboxylate;
(15) *tert*-butyl 4-(((6-methoxy-4-(4-(2-(4-(trifluoromethyl)phenyl)acetamido)phenyl)quinazolin-7-yl)oxy)methyl)piperidin-1-carboxylate;
(16) *N*-(4-(6-methoxy-7-(piperidin-3-ylmethoxy)quinazolin-4-yl)phenyl)-2-(4-(trifluoromethyl)phenyl)acetamide;
(17) *N*-(4-(6-methoxy-7-(piperidin-4-ylmethoxy)quinazolin-4-yl)phenyl)-2-(4-(trifluoromethyl)phenyl)acetamide;
(18) *N*-(4-(7-((1-ethylpiperidin-3-yl)methoxy)-6-methoxyquinazolin-4-yl)phenyl)-2-(4-(trifluoromethyl)phenyl)acetamide;
(19) *tert*-butyl 6-(3-((6-methoxy-4-(4-(2-(4-(trifluoromethyl)phenyl)acetamido)phenyl)quinazolin-7-yl)oxy)propoxy)-2,6-diazaspiro[3.3]heptane-2-carboxylate;
(20) *N*-(4-(7-(3-(2,6-diazaspiro[3.3]heptane-2-yl)propoxy)-6-methoxyquinazolin-4-yl)phenyl)-2-(4-(trifluoromethyl)phenyl)acetamide;
(21) *N*-(4-(7-(3-(6-ethyl-2,6-diazaspiro[3.3]heptane-2-yl)propoxy)-6-methoxyquinazolin-4-yl)phenyl)-2-(4-(trifluoromethyl)phenyl)acetamide;
(22) *N*-(4-(7-(3-(3-(dimethylamino)azetidine-1-yl)propoxy)-6-methoxyquinazolin-4-yl)phenyl)-2-(4-(trifluoromethyl)phenyl)acetamide;
(23) *N*-(4-(7-(3-chloropropoxy)-6-methoxyquinazolin-4-yl)phenyl)-2-(4-(trifluoromethyl)phenyl)acetamide;
(24) *N*-(4-(7-(azetidine-3-yloxy)-6-methoxyquinazolin-4-yl)phenyl)-2-(4-(trifluoromethyl)phenyl)acetamide;
(25) *N*-(4-(7-(3-(cyclopropylamino)propoxy)-6-methoxyquinazolin-4-yl)phenyl)-2-(4-(trifluoromethyl)phenyl)acetamide;
(26) *N*-(4-(6-methoxy-7-(3-((1-methyl-1*H*-pyrazol-4-yl)amino)propoxy)quinazolin-4-yl)phenyl)-2-(4-(trifluoromethyl)phenyl)acetamide;
(27) *N*-(4-(6-methoxy-7-((1-(4-methoxybenzyl)azetidin-3-yl)oxy)quinazolin-4-yl)phenyl)-2-(4-(trifluoromethyl)phenyl)acetamide;
(28) *N*-(4-(6-methoxy-7-((1-(2-methoxyethyl)azetidin-3-yl)oxy)quinazolin-4-yl)phenyl)-2-(4-(trifluoromethyl)phenyl)acetamide;
(29) *N*-(4-(6-methoxy-7-(pyridin-3-ylmethoxy)quinazolin-4-yl)phenyl)-2-(4-(trifluoromethyl)phenyl)acetamide;
(30) *N*-(4-(7-(2-(1*H*-pyrazol-1-yl)ethoxy)-6-methoxyquinazolin-4-yl)phenyl)-2-(4-(trifluoromethyl)phenyl)acetamide;
(31) *N*-(4-(6-methoxy-7-(3-((1*R*,4*R*)-5-methyl-2,5-diazabicyclo[2.2.1]heptane-2-yl)propoxy)quinazolin-4-yl)phenyl)-2-(4-(trifluoromethyl)phenyl)acetamide;
(32) *N*-(4-(6-methoxy-7-(3-((1*S*,4*S*)-5-methyl-2,5-diazabicyclo[2.2.1]heptane-2-yl)propoxy)quinazolin-4-yl)phenyl)-2-(4-(trifluoromethyl)phenyl)acetamide;
(33) *N*-(4-(7-(3-(hexahydropyrrolo[1,2-*a*]pyrazin-2(1*H*)-yl)propoxy)-6-methoxyquinazolin-4-yl)phenyl)-2-(4-(trifluoromethyl)phenyl)acetamide;
(34) *N*-(4-(6-methoxy-7-(3-(3-(trifluoromethyl)-5,6-dihydro-[1,2,4]triazole[4,3-a]pyrazin-7(8H)-yl)propoxy)quinazolin-4-yl)phenyl)-2-(4-(trifluoromethyl)phenyl)acetamide;
(35) *tert*-butyl (*R*)-3-(((6-methoxy-4-(4-(2-(4-(trifluoromethyl)phenyl)acetamido)phenyl)quinazolin-7-yl)oxy)methyl)pyrrolidin-1-carboxylate;
(36) *tert*-butyl (*S*)-3-(((6-methoxy-4-(4-(2-(4-(trifluoromethyl)phenyl)acetamido)phenyl)quinazolin-7-yl)oxy)methyl)pyrrolidin-1-carboxylate;
(37) *N*-(4-(6-methoxy-7-(3-(3-methyl-3,6-diazabicyclo[3.1.1]heptane-6-yl)propoxy)quinazolin-4-yl)phenyl)-2-(4-(trifluoromethyl)phenyl)acetamide;
(38) (*R*)-*N*-(4-(6-methoxy-7-(pyrrolidin-3-ylmethoxy)quinazolin-4-yl)phenyl)-2-(4-(trifluoromethyl)phenyl)acetamide;
(39) (*S*)-*N*-(4-(6-methoxy-7-(pyrrolidin-3-ylmethoxy)quinazolin-4-yl)phenyl)-2-(4-(trifluoromethyl)phenyl)acetamide;
(40) (*R*)-*N*-(4-(7-((1-ethylpyrrolidin-3-yl)methoxy)-6-methoxyquinazolin-4-yl)phenyl)-2-(4-(trifluoromethyl)phenyl)acetamide;
(41) (*S*)-*N*-(4-(7-((1-ethylpyrrolidin-3-yl)methoxy)-6-methoxyquinazolin-4-yl)phenyl)-2-(4-(trifluoromethyl)phenyl)acetamide;
(42) *N*-(4-(6-methoxy-7-((1-(2-methoxyethyl)piperidin-3-yl)methoxy)quinazolin-4-yl)phenyl)-2-(4-(trifluoromethyl)phenyl)acetamide;
(43) *N*-(4-(6-methoxy-7-((1-(2-methoxyethyl)piperidin-4-yl)methoxy)quinazolin-4-yl)phenyl)-2-(4-(trifluoromethyl)phenyl)acetamide;
(44) *N*-(4-(6-methoxy-7-(3-(4-methylpiperidin-1-yl)propoxy)quinazolin-4-yl)phenyl)-2-(4-(trifluoromethyl)phenyl)acetamide;
(45) *N*-(4-(6-methoxy-7-(3-(4-methylpiperazin-1-yl)propoxy)quinazolin-4-yl)phenyl)-1-phenylmethanesulfonamide;
(46) *N*-(4-(6-methoxy-7-(3-(4-methylpiperazin-1-yl)propoxy)quinazolin-4-yl)phenyl)-1-(4-(trifluoromethyl)phenyl)methanesulfonamide;
(47) 1-(4-bromophenyl)-*N*-(4-(6-methoxy-7-(3-(4-methylpiperazin-1-yl)propoxy)quinazolin-4-yl)phenyl)methanesulfonamide;
(48) 2-(furan-2-yl)-*N*-(4-(6-methoxy-7-(3-(4-methylpiperazin-1-yl)propoxy)quinazolin-4-yl)phenyl)propanamide;
(49) *N*-(4-(6-methoxy-7-(3-(4-methylpiperazin-1-yl)propoxy)quinazolin-4-yl)-2-methylphenyl)-2-(4-(trifluoromethyl)phenyl)acetamide;
(50) *N*-(2-fluoro-4-(6-methoxy-7-(3-(4-methylpiperazin-1-yl)propoxy)quinazolin-4-yl)phenyl)-2-(4-(trifluoromethyl)phenyl)acetamide;
(51) *N*-(4-(6-methoxy-7-(3-(4-methylpiperazin-1-yl)propoxy)quinazolin-4-yl)phenyl)-2-(thiazole-4-yl)acetamide;
(52) 2-(benzo[*d*][1,3]dioxol-5-yl)-*N*-(2-fluoro-4-(6-methoxy-7-((1-(2-methoxyethyl)piperidin-4-yl)methoxy)quinazolin-4-yl)phenyl)acetamide;
(53) 2-(benzo[*d*][1,3]dioxol-5-yl)-*N*-(4-(6-methoxy-7-((1-(2-methoxyethyl)piperidin-4-yl)methoxy)quinazolin-4-yl)-2-methylphenyl)acetamide;
(54) 2-(benzo[*d*][1,3]dioxol-5-yl)-*N*-(4-(6-methoxy-7-(3-(4-methylpiperazin-1-yl)propoxy)quinazolin-4-yl)phenyl)acetamide;
(55) *N*-(4-(6-methoxy-7-(3-(4-methylpiperazin-1-yl)propoxy)quinazolin-4-yl)phenyl)-2-(3-(trifluoromethyl)phenyl)acetamide;

(56) *N*-(4-(6-methoxy-7-((1-methylpiperidin-4-yl)methoxy)quinazolin-4-yl)phenyl)-2-(4-(trifluoromethyl)phenyl)acetamide;
(57) *N*-(4-(6,7-bis(2-methoxyethoxy)quinazolin-4-yl)phenyl)-2-(4-(trifluoromethyl)phenyl)acetamide;
(58) 2-amino-*N*-(4-(6,7-bis(2-methoxyethoxy)quinazolin-4-yl)phenyl)-2-(4-fluorophenyl)propanamide;
(59) *N*-(4-(6-methoxy-7-(piperidin-4-ylmethoxy)quinazolin-4-yl)phenyl)-2-(6-(trifluoromethyl)pyridin-3-yl)acetamide;
(60) *N*-(4-(6-methoxy-7-(piperidin-4-ylmethoxy)quinazolin-4-yl)phenyl)-2-(3-(trifluoromethyl)phenyl)acetamide;
(61) 2-(4-isopropyl-1*H*-1,2,3-triazole-1-yl)-*N*-(4-(6-methoxy-7-(piperidin-4-ylmethoxy)quinazolin-4-yl)phenyl)acetamide;
(62) 2-(4-(*tert*-butyl)-1*H*-1,2,3-triazole-1-yl)-*N*-(4-(6-methoxy-7-(piperidin-4-ylmethoxy)quinazolin-4-yl)phenyl)acetamide;
(63) *N*-(4-(7-((1-ethylpiperidin-4-yl)methoxy)-6-methoxyquinazolin-4-yl)phenyl)-2-(6-(trifluoromethyl)pyridin-3-yl)acetamide;
(64) *N*-(4-(7-((1-ethylpiperidin-4-yl)methoxy)-6-methoxyquinazolin-4-yl)phenyl)-2-(3-(trifluoromethyl)phenyl)acetamide;
(65) *N*-(4-(7-((1-ethylpiperidin-4-yl)methoxy)-6-methoxyquinazolin-4-yl)phenyl)-2-(4-isopropyl-1H-1,2,3-triazole-1-yl)acetamide;
(66) 2-(4-(*tert*-butyl)-1*H*-1,2,3-triazole-1-yl)-*N*-(4-(7-((1-ethylpiperidin-4-yl)methoxy)-6-methoxyquinazolin-4-yl)phenyl)acetamide;
(67) 2-(4-isopropyl-1*H*-1,2,3-triazole-1-yl)-*N*-(4-(6-methoxy-7-(3-(4-methylpiperazin-1-yl)propoxy)quinazolin-4-yl)phenyl)acetamide;
(68) 2-(4-(*tert*-butyl)-1*H*-1,2,3-triazole-1-yl)-*N*-(4-(6-methoxy-7-(3-(4-methylpiperazin-1-yl)propoxy)quinazolin-4-yl)phenyl)acetamide;
(69) 2-(6-chloropyridin-3-yl)-*N*-(4-(6-methoxy-7-(piperidin-4-ylmethoxy)quinazolin-4-yl)phenyl)acetamide;
(70) 2-(6-chloropyridin-3-yl)-*N*-(4-(7-((1-ethylpiperidin-4-yl)methoxy)-6-methoxyquinazolin-4-yl)phenyl)acetamide;
(71) *N*-(4-(6-methoxy-7-(piperidin-4-ylmethoxy)quinazolin-4-yl)phenyl)-3-phenylpropanamide;
(72) *N*-(4-(7-((1-ethylpiperidin-4-yl)methoxy)-6-methoxyquinazolin-4-yl)phenyl)-3-phenylpropanamide;
(73) (*R*)-*N*-(4-(6-methoxy-7-(piperidin-3-ylmethoxy)quinazolin-4-yl)phenyl)-2-(4-(trifluoromethyl)phenyl)acetamide;
(74) (*R*)-*N*-(4-(7-((1-ethylpiperidin-3-yl)methoxy)-6-methoxyquinazolin-4-yl)phenyl)-2-(4-(trifluoromethyl)phenyl)acetamide;
(75) (*S*)-*N*-(4-(6-methoxy-7-(piperidin-3-ylmethoxy)quinazolin-4-yl)phenyl)-2-(4-(trifluoromethyl)phenyl)acetamide;
(76) (*S*)-*N*-(4-(7-((1-ethylpiperidin-3-yl)methoxy)-6-methoxyquinazolin-4-yl)phenyl)-2-(4-(trifluoromethyl)phenyl)acetamide;
(77) 2-(4-chloro-3-(trifluoromethyl)phenyl)-*N*-(4-(6-methoxy-7-(piperidin-4-ylmethoxy)quinazolin-4-yl)phenyl)acetamide;
(78) 2-(4-chloro-3-(trifluoromethyl)phenyl)-*N*-(4-(7-((1-ethylpiperidin-4-yl)methoxy)-6-methoxyquinazolin-4-yl)phenyl)acetamide;
(79) 2-(3-fluoro-4-(trifluoromethyl)phenyl)-*N*-(4-(6-methoxy-7-(piperidin-4-ylmethoxy)quinazolin-4-yl)phenyl)acetamide;
(80) *N*-(4-(7-((1-ethylpiperidin-4-yl)methoxy)-6-methoxyquinazolin-4-yl)phenyl)-2-(3-fluoro-4-(trifluoromethyl)phenyl)acetamide;
(81) *N*-(4-(6-methoxy-7-(3-(4-methylpiperazin-1-yl)propoxy)quinazolin-4-yl)phenyl)-2-(6-methoxypyridin-3-yl)acetamide;
(82) 2-(4-chloro-3-(trifluoromethyl)phenyl)-*N*-(4-(6-methoxy-7-(3-(4-methylpiperazin-1-yl)propoxy)quinazolin-4-yl)phenyl)acetamide;
(83) 2-(4-cyanophenyl)-*N*-(4-(6-methoxy-7-(3-(4-methylpiperazin-1-yl)propoxy)quinazolin-4-yl)phenyl)acetamide;
(84) *N*-(4-(6-methoxy-7-(3-(4-methylpiperazin-1-yl)propoxy)quinazolin-4-yl)phenyl)-2-(thiophene-3-yl)acetamide;
(85) 2-(2,3-dihydrobenzofuran-5-yl)-*N*-(4-(6-methoxy-7-(3-(4-methylpiperazin-1-yl)propoxy)quinazolin-4-yl)phenyl)acetamide;
(86) *N*-(4-(6-methoxy-7-(3-(4-methylpiperazin-1-yl)propoxy)quinazolin-4-yl)phenyl)-2-(piperidin-4-yl)acetamide;
(87) *N*-(4-(6-methoxy-7-(3-(4-methylpiperazin-1-yl)propoxy)quinazolin-4-yl)phenyl)but-3-enamide;
(88) 2-(4-fluorophenyl)-*N*-(4-(6-methoxy-7-(piperidin-4-ylmethoxy)quinazolin-4-yl)phenyl)-2-methylpropanamide;

(89) 2-(4-fluorophenyl)-*N*-(4-(6-methoxy-7-((1-(2-methoxyethyl)piperidin-4-yl)methoxy)quinazolin-4-yl)phenyl)-2-methylpropanamide;

(90) *N*-(4-(7-((1-ethylpiperidin-4-yl)methoxy)-6-methoxyquinazolin-4-yl)phenyl)-2-(4-fluorophenyl)-2-methylpropanamide;

(91) 1-(4-fluorophenyl)-*N*-(4-(6-methoxy-7-(piperidin-4-ylmethoxy)quinazolin-4-yl)phenyl)cyclopropane-1-carboxamide;

(92) 1-(4-fluorophenyl)-*N*-(4-(6-methoxy-7-((1-(2-methoxyethyl)piperidin-4-yl)methoxy)quinazolin-4-yl)phenyl)cyclopropane-1-carboxamide;

(93) *N*-(4-(7-((1-ethylpiperidin-4-yl)methoxy)-6-methoxyquinazolin-4-yl)phenyl)-1-(4-fluorophenyl)cyclopropane-1-carboxamide;

(94) 2-(4-isopropyl-1*H*-1,2,3-triazole-1-yl)-*N*-(4-(6-methoxy-7-((1-(2-methoxyethyl)piperidin-4-yl)methoxy)quinazolin-4-yl)phenyl)acetamide;

(95) 2-(4-(*tert*-butyl)-1*H*-1,2,3-triazole-1-yl)-*N*-(4-(6-methoxy-7-((1-(2-methoxyethyl)piperidin-4-yl)methoxy)quinazolin-4-yl)phenyl)acetamide;

(96) *N*-(4-(6-methoxy-7-((1-(2-methoxyethyl)piperidin-4-yl)methoxy)quinazolin-4-yl)phenyl)-2-(6-(trifluoromethyl)pyridin-3-yl)acetamide;

(97) 2-(benzo[*d*][1,3]dioxol-5-yl)-*N*-(4-(6-methoxy-7-(piperidin-4-ylmethoxy)quinazolin-4-yl)phenyl)acetamide;

(98) 2-(benzo[*d*][1,3]dioxol-5-yl)-*N*-(4-(6-methoxy-7-((1-(2-methoxyethyl)piperidin-4-yl)methoxy)quinazolin-4-yl)phenyl)acetamide;

(99) 2-(benzo[*d*][1,3]dioxol-5-yl)-*N*-(4-(7-((1-ethylpiperidin-4-yl)methoxy)-6-methoxyquinazolin-4-yl)phenyl)acetamide;

(100) *N*-(4-(6-methoxy-7-((1-methylpiperidin-4-yl)methoxy)quinazolin-4-yl)phenyl)-2-(6-methoxypyridin-3-yl)acetamide;

(101) *N*-(4-(6-methoxy-7-((1-methylpiperidin-4-yl)methoxy)quinazolin-4-yl)phenyl)-2-(6-(trifluoromethyl)pyridin-3-yl)acetamide;

(102) *N*-(4-(7-((1-acetylpiperidin-4-yl)methoxy)-6-methoxyquinazolin-4-yl)phenyl)-2-(4-(trifluoromethyl)phenyl)acetamide;

(103) *N*-(4-(7-((1-acetylpiperidin-4-yl)methoxy)-6-methoxyquinazolin-4-yl)phenyl)-2-(6-methoxypyridin-3-yl)acetamide;

(104) *N*-(4-(7-((1-acetylpiperidin-4-yl)methoxy)-6-methoxyquinazolin-4-yl)phenyl)-2-(6-(trifluoromethyl)pyridin-3-yl)acetamide;

(105) 2-(4-isopropyl-1*H*-1,2,3-triazole-1-yl)-N-(4-(6-methoxy-7-((1-methylpiperidin-4-yl)methoxy)quinazolin-4-yl)phenyl)acetamide;

(106) 2-(4-(*tert*-butyl)-1*H*-1,2,3-triazole-1-yl)-*N*-(4-(6-methoxy-7-((1-methylpiperidin-4-yl)methoxy)quinazolin-4-yl)phenyl)acetamide;

(107) *N*-(4-(7-((1-acetylpiperidin-4-yl)methoxy)-6-methoxyquinazolin-4-yl)phenyl)-2-(4-isopropyl-1H-1,2,3-triazole-1-yl)acetamide;

(108) *N*-(4-(7-((1-acetylpiperidin-4-yl)methoxy)-6-methoxyquinazolin-4-yl)phenyl)-2-(4-(*tert*-butyl)-1H-1,2,3-triazole-1-yl)acetamide;

(109) *N*-(4-(7-((1-ethylpiperidin-3-yl)methoxy)-6-methoxyquinazolin-4-yl)phenyl)-2-(6-methoxypyridin-3-yl)acetamide;

(110) *N*-(4-(7-((1-ethylpiperidin-3-yl)methoxy)-6-methoxyquinazolin-4-yl)phenyl)-2-(6-(trifluoromethyl)pyridin-3-yl)acetamide;

(111) *N*-(4-(7-((1-ethylpiperidin-3-yl)methoxy)-6-methoxyquinazolin-4-yl)phenyl)-2-(4-isopropyl-1H-1,2,3-triazole-1-yl)acetamide;

(112) 2-(4-(*tert*-butyl)-1*H*-1,2,3-triazole-1-yl)-*N*-(4-(7-((1-ethylpiperidin-3-yl)methoxy)-6-methoxyquinazolin-4-yl)phenyl)acetamide;

(113) 2-(6-chloropyridin-3-yl)-*N*-(4-(6-methoxy-7-((1-(2-methoxyethyl)piperidin-4-yl)methoxy)quinazolin-4-yl)phenyl)acetamide;

(114) *N*-(4-(6-methoxy-7-((1-(2-methoxyethyl)piperidin-4-yl)methoxy)quinazolin-4-yl)phenyl)-4-phenylbutanamide;

(115) *N*-(4-(7-((1-isopropylpiperidin-4-yl)methoxy)-6-methoxyquinazolin-4-yl)phenyl)-2-(4-(trifluoromethyl)phenyl)acetamide;

(116) *N*-(4-(7-((1-isopropylpiperidin-4-yl)methoxy)-6-methoxyquinazolin-4-yl)phenyl)-2-(6-methoxypyridin-3-yl)acetamide;

(117) *N*-(4-(6-methoxy-7-(2-methoxyethoxy)quinazolin-4-yl)phenyl)-2-(4-(trifluoromethyl)phenyl)acetamide;

(118) *N*-(4-(6-methoxy-7-(2-methoxyethoxy)quinazolin-4-yl)phenyl)-2-(6-(trifluoromethyl)pyridin-3-yl)acetamide;

(119) 2-(4-(*tert*-butyl)-1*H*-1,2,3-triazole-1-yl)-*N*-(4-(7-((1-isopropylpiperidin-4-yl)methoxy)-6-methoxyquinazolin-4-yl)phenyl)acetamide;

(120) 2-(4-isopropyl-1*H*-1,2,3-triazole-1-yl)-*N*-(4-(7-((1-isopropylpiperidin-4-yl)methoxy)-6-methoxyquinazolin-4-yl)phenyl)acetamide;

(121) *N*-(4-(7-((1-isopropylpiperidin-4-yl)methoxy)-6-methoxyquinazolin-4-yl)phenyl)-2-(6-(trifluoromethyl)pyridin-3-yl)acetamide;

(122) *N*-(4-(6-(2-(diethylamino)ethoxy)-7-methoxyquinazolin-4-yl)phenyl)-2-(4-fluorophenyl)acetamide;

(123) *N*-(4-(6-((1-ethylpiperidin-4-yl)oxy)-7-methoxyquinazolin-4-yl)phenyl)-2-(4-(trifluoromethyl)phenyl)acetamide;

(124) 2-(4-fluorophenyl)-*N*-(4-(6-hydroxy-7-methoxyquinazolin-4-yl)phenyl)acetamide;

(125) (*R*)-*N*-(4-(7-methoxy-6-((tetrahydrofuran-3-yl)oxy)quinazolin-4-yl)phenyl)-2-(4-(trifluoromethyl)phenyl)acetamide;

(126) *N*-(4-(7-methoxy-6-(3-(4-methylpiperazin-1-yl)propoxy)quinazolin-4-yl)phenyl)-2-(4-(trifluoromethyl)phenyl)acetamide;

(127) *N*-(4-(6-(3-(4-cyclopropylpiperazin-1-yl)propoxy)-7-methoxyquinazolin-4-yl)phenyl)-2-(4-(trifluoromethyl)phenyl)acetamide;

(128) *N*-(4-(6-(3-(4-acetylpiperazin-1-yl)propoxy)-7-methoxyquinazolin-4-yl)phenyl)-2-(4-(trifluoromethyl)phenyl)acetamide;

(129) *N*-(4-(7-methoxy-6-(3-morpholinopropoxy)quinazolin-4-yl)phenyl)-2-(4-(trifluoromethyl)phenyl)acetamide;

(130) *tert*-butyl 3-(((7-methoxy-4-(4-(2-(4-(trifluoromethyl)phenyl)acetamido)phenyl)quinazolin-6-yl)oxy)methyl)piperidin-1-carboxylate;

(131) *N*-(4-(7-methoxy-6-(2-(4-methylpiperazin-1-yl)ethoxy)quinazolin-4-yl)phenyl)-2-(4-(trifluoromethyl)phenyl)acetamide;

(132) 7-methoxy-4-(4-(2-(4-(trifluoromethyl)phenyl)acetamido)phenyl)quinazolin-6-yl 4-methylpiperazin-1-carboxylate;

(133) *N*-(4-(7-methoxy-6-(piperidin-3-ylmethoxy)quinazolin-4-yl)phenyl)-2-(4-(trifluoromethyl)phenyl)acetamide;

(134) *N*-(4-(6-((1-ethylpiperidin-3-yl)methoxy)-7-methoxyquinazolin-4-yl)phenyl)-2-(4-(trifluoromethyl)phenyl)acetamide;

(135) *tert*-butyl 3-((7-methoxy-4-(4-(2-(4-(trifluoromethyl)phenyl)acetamido)phenyl)quinazolin-6-yl)oxy)azetidin-1-carboxylate;

(136) *tert*-butyl 3-((7-methoxy-4-(4-(2-(4-(trifluoromethyl)phenyl)acetamido)phenyl)quinazolin-6-yl)oxy)pyrrolidin-1-carboxylate;

(137) *tert*-butyl 4-((7-methoxy-4-(4-(2-(4-(trifluoromethyl)phenyl)acetamido)phenyl)quinazolin-6-yl)oxy)piperidin-1-carboxylate;

(138) *N*-(4-(6-(azetidin-3-yloxy)-7-methoxyquinazolin-4-yl)phenyl)-2-(4-(trifluoromethyl)phenyl)acetamide;

(139) *N*-(4-(7-methoxy-6-(pyrrolidin-3-yloxy)quinazolin-4-yl)phenyl)-2-(4-(trifluoromethyl)phenyl)acetamide;

(140) *N*-(4-(7-methoxy-6-(piperidin-4-yloxy)quinazolin-4-yl)phenyl)-2-(4-(trifluoromethyl)phenyl)acetamide;

(141) *N*-(4-(7-(3-(4-acetylpiperazin-1-yl)propoxy)-6-methoxyquinazolin-4-yl)phenyl)-2-(4-(trifluoromethyl)phenyl)acetamide;

(142) *N*-(4-(7-(3-(4-cyclopropylpiperazin-1-yl)propoxy)-6-methoxyquinazolin-4-yl)phenyl)-2-(4-(trifluoromethyl)phenyl)acetamide;

(143) *N*-(4-(6-methoxy-7-(3-(4-methyl-3-oxopiperazin-1-yl)propoxy)quinazolin-4-yl)phenyl)-2-(4-(trifluoromethyl)phenyl)acetamide;

(144) *N*-(4-(6-((1-ethylazetidin-3-yl)oxy)-7-methoxyquinazolin-4-yl)phenyl)-2-(4-(trifluoromethyl)phenyl)acetamide;

(145) *N*-(4-(6-((1-ethylpyrrolidin-3-yl)oxy)-7-methoxyquinazolin-4-yl)phenyl)-2-(4-(trifluoromethyl)phenyl)acetamide;

(146) *N*-(4-(7-methoxy-6-(piperidin-4-ylmethoxy)quinazolin-4-yl)phenyl)-2-(4-(trifluoromethyl)phenyl)acetamide;

(147) *N*-(4-(6-((1-ethylpiperidin-4-yl)methoxy)-7-methoxyquinazolin-4-yl)phenyl)-2-(4-(trifluoromethyl)phenyl)acetamide;

(148) *N*-(4-(6-(3-(2,6-diazaspiro[3.3]heptane-2-yl)propoxy)-7-methoxyquinazolin-4-yl)phenyl)-2-(4-(trifluoromethyl)phenyl)acetamide;

(149) *N*-(4-(6-(3-(6-ethyl-2,6-diazaspiro[3.3]heptane-2-yl)propoxy)-7-methoxyquinazolin-4-yl)phenyl)-2-(4-(trifluoromethyl)phenyl)acetamide;

(150) *N*-(4-(6-(3-(3,3-bis(hydroxymethyl)azetidin-1-yl)propoxy)-7-methoxyquinazolin-4-yl)phenyl)-2-(4-(trifluoromethyl)phenyl)acetamide;

(151) *N*-(4-(6-(3-(7-oxa-2-azaspiro[3.5]nonane-2-yl)propoxy)-7-methoxyquinazolin-4-yl)phenyl)-2-(4-(trifluoromethyl)phenyl)acetamide;

(152) *N*-(4-(6-(3-(3-(dimethylamino)azetidin-1-yl)propoxy)-7-methoxyquinazolin-4-yl)phenyl)-2-(4-(trifluoromethyl)phenyl)acetamide;

(153) *N*-(4-(7-methoxy-6-(oxetan-3-yloxy)quinazolin-4-yl)phenyl)-2-(4-(trifluoromethyl)phenyl)acetamide;

(154) *N*-(4-(6-(3-(cyclopropylamino)propoxy)-7-methoxyquinazolin-4-yl)phenyl)-2-(4-(trifluoromethyl)phenyl)acetamide;

(155) *N*-(4-(7-methoxy-6-(3-((1-methyl-1H-pyrazol-4-yl)amino)propoxy)quinazolin-4-yl)phenyl)-2-(4-(trifluoromethyl)phenyl)acetamide;

(156) (*S*)-N-(4-(7-methoxy-6-((tetrahydrofuran-2-yl)oxy)quinazolin-4-yl)phenyl)-2-(4-(trifluoromethyl)phenyl)acetamide;

(157) *N*-(4-(7-methoxy-6-(pyridin-3-ylmethoxy)quinazolin-4-yl)phenyl)-2-(4-(trifluoromethyl)phenyl)acetamide;

(158) *N*-(4-(7-methoxy-6-(3-((1*S*,4*S*)-5-methyl-2,5-diazabicyclo[2.2.1]heptane-2-yl)propoxy)quinazolin-4-yl)phenyl)-2-(4-(trifluoromethyl)phenyl)acetamide;

(159) *N*-(4-(7-methoxy-6-(3-((1*R*,4*R*)-5-methyl-2,5-diazabicyclo[2.2.1]heptane-2-yl)propoxy)quinazolin-4-yl)phenyl)-2-(4-(trifluoromethyl)phenyl)acetamide;

(160) *N*-(4-(7-methoxy-6-(3-(pyrrolidin-1-yl)propoxy)quinazolin-4-yl)phenyl)-2-(4-(trifluoromethyl)phenyl)acetamide;

(161) *N*-(4-(6-(3-(hexahydropyrrolo[1,2-a]pyrazin-2(1*H*)-yl)propoxy)-7-methoxyquinazolin-4-yl)phenyl)-2-(4-(trifluoromethyl)phenyl)acetamide;

(162) *N*-(4-(6-(3-(2-oxa-6-azaspiro[3.3]heptane-6-yl)propoxy)-7-methoxyquinazolin-4-yl)phenyl)-2-(4-(trifluoromethyl)phenyl)acetamide;

(163) 2-(benzo[*d*][1,3]dioxol-5-yl)-*N*-(2-fluoro-4-(6-methoxy-7-(3-(4-methylpiperazin-1-yl)propoxy)quinazolin-4-yl)phenyl)acetamide;

(164) 2-(benzo[*d*][1,3]dioxol-5-yl)-*N*-(4-(6-methoxy-7-(3-(4-methylpiperazin-1-yl)propoxy)quinazolin-4-yl)-2-methylphenyl)acetamide;

(165) 4-(6-methoxy-7-(3-(4-methylpiperazin-1-yl)propoxy)quinazolin-4-yl)-*N*-(4-(trifluoromethyl)benzyl)benzamide;

(166) *N*-(benzo[*d*][1,3]dioxol-5-ylmethyl)-4-(6-methoxy-7-(3-(4-methylpiperazin-1-yl)propoxy)quinazolin-4-yl)benzamide;

(167) *N*-(3,4-difluorobenzyl)-4-(6-methoxy-7-(3-(4-methylpiperazin-1-yl)propoxy)quinazolin-4-yl)benzamide;

(168) 1-(4-chloro-3-(trifluoromethyl)phenyl)-3-(4-(6-methoxy-7-(3-(4-methylpiperazin-1-yl)propoxy)quinazolin-4-yl)phenyl)urea; and

(169) 1-(4-(6-methoxy-7-(3-(4-methylpiperazin-1-yl)propoxy)quinazolin-4-yl)phenyl)-3-(3-(trifluoromethyl)phenyl)urea.

**8.** A pharmaceutical composition comprising the compound, the stereoisomer thereof, or the pharmaceutically acceptable salt thereof according to any one of claims 1 to 7.

**9.** A pharmaceutical composition for preventing or treating one or more diseases selected from the group consisting of cancer, mast cell-related diseases, fibrosis diseases, neurological diseases, atherosclerosis, and pulmonary hypertension, comprising, as an active ingredient, the compound, the stereoisomer thereof, or the pharmaceutically acceptable salt thereof according to any one of claims 1 to 7.

**10.** The pharmaceutical composition of claim 9, wherein the cancer is any one or more selected from the group consisting of squamous cell carcinoma, small cell lung cancer, non-small cell lung cancer, adenocarcinoma of lung, squamous cell carcinoma of lung, peritoneal cancer, skin cancer, skin or ocular melanoma, rectal cancer, perianal gland cancer, esophageal cancer, small bowel cancer, endocrine tumor, thyroid cancer, parathyroid cancer, adrenal cancer, soft tissue sarcoma, urethral cancer, chronic or acute leukemia, lymphocytic lymphoma, hepatocellular carcinoma, gastrointestinal cancer, stomach cancer, pancreatic cancer, glioblastoma, cervical cancer, ovarian cancer, liver cancer, bladder cancer, liver tumor, breast cancer, colon cancer, colorectal cancer, endometrial cancer or uterine cancer, salivary gland cancer, kidney cancer, prostate cancer, vulvar cancer, head and neck cancer, brain cancer, osteosarcoma, neuroblastoma, germ cell tumor, and gastrointestinal stromal tumor.

**11.** The pharmaceutical composition of claim 9, wherein the fibrosis is any one or more selected from the group consisting of systemic sclerosis, pulmonary fibrosis, hepatic fibrosis, cirrhosis, renal fibrosis, myelofibrosis, myocardial fibrosis,

sarcoidosis, keloid, burn-induced hypertrophic scar, proliferative retinopathy, glaucoma, cataract, posterior capsular opacification, angioplasty, vascular restoneosis after vascular surgery or vascular inhury, cystic fibrosis, and Marfan syndrome.

**12.** A pharmaceutical composition for preventing or treating c-KIT or PDGFR-related diseases comprising, as an active ingredient, the compound, the stereoisomer thereof, or the pharmaceutically acceptable salt thereof according to any one of claims 1 to 7.

**13.** The pharmaceutical composition of claim 12, wherein the composition inhibits any one or more selected from the group consisting of PDGFR-$\alpha$, PDGFR-$\beta$, c-KIT(D816V), c-KIT V560G/D816V, and c-KIT D814Y.

**14.** A method of treating or preventing any one or more diseases selected from the group consisting of cancer, mast cell-related diseases, fibrosis diseases, neurological diseases, atherosclerosis, and pulmonary hypertension, comprising administering to a subject in need thereof a therapeutically effective amount of the compound, the stereoisomer thereof, or the pharmaceutically acceptable salt thereof according to any one of claims 1 to 7.

**15.** A method for treating or preventing c-KIT or PDGFR-related diseases, comprising administering to a subject in need thereof a therapeutically effective amount of the compound, the stereoisomer thereof, or the pharmaceutically acceptable salt thereof according to any one of claims 1 to 7.

**16.** Use of the compound, the stereoisomer thereof, or the pharmaceutically acceptable salt thereof according to any one of claims 1 to 7 for use in preparation of a medicament to treat or prevent any one or more diseases selected from the group consisting of cancer, mast cell-related diseases, fibrosis diseases, neurological diseases, atherosclerosis, and pulmonary hypertension.

**17.** Use of the compound, the stereoisomer thereof, or the pharmaceutically acceptable salt thereof according to any one of claims 1 to 7 for use in the preparation of medicament to treat or prevent c-KIT or PDGFR-related diseases.

## INTERNATIONAL SEARCH REPORT

International application No.
**PCT/KR2022/016766**

**A. CLASSIFICATION OF SUBJECT MATTER**

**C07D 239/74**(2006.01)i; **A61K 31/517**(2006.01)i; **A61P 35/00**(2006.01)i; **A61P 3/04**(2006.01)i; **A61P 25/00**(2006.01)i; **A61P 9/10**(2006.01)i; **A61P 9/12**(2006.01)i; **C07D 401/12**(2006.01)i; **C07D 487/10**(2006.01)i; **C07D 403/12**(2006.01)i

According to International Patent Classification (IPC) or to both national classification and IPC

**B. FIELDS SEARCHED**

Minimum documentation searched (classification system followed by classification symbols)

C07D 239/74(2006.01); A61K 31/495(2006.01); A61K 31/517(2006.01); A61K 31/535(2006.01); C07D 215/44(2006.01); C07D 401/12(2006.01); C07D 487/04(2006.01); C07D 495/04(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, CAplus) & keywords: 퀴나졸린 유도체 (quinazoline derivative), c-KIT, PDGFR

**C. DOCUMENTS CONSIDERED TO BE RELEVANT**

| Category* | Citation of document, with indication, where appropriate, of the relevant passages | Relevant to claim No. |
|---|---|---|
| X | WO 2014-039714 A2 (PLEXXIKON INC.) 13 March 2014 (2014-03-13) See claims 14, 28 and 30-32; paragraphs [0081], [0222], [0249], [0250], [0268] and [0271]; and page 165. | 1-13,16,17 |
| A | WO 2019-046778 A1 (MAVUPHARMA, INC.) 07 March 2019 (2019-03-07) See entire document. | 1-13,16,17 |
| A | WO 2020-140001 A1 (RIBOSCIENCE LLC) 02 July 2020 (2020-07-02) See entire document. | 1-13,16,17 |
| A | WO 2005-040125 A1 (AXXIMA PHARMACEUTICALS AG) 06 May 2005 (2005-05-06) See entire document. | 1-13,16,17 |
| A | US 5480883 A (SPADA, A. P. et al.) 02 January 1996 (1996-01-02) See entire document. | 1-13,16,17 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 February 2023** | **10 February 2023** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

INTERNATIONAL SEARCH REPORT

International application No.
PCT/KR2022/016766

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **14, 15**
because they relate to subject matter not required to be searched by this Authority, namely:

Claims 14 and 15 pertain to a method for treatment of the human body by surgery or therapy (PCT Article 17(2)(a)(i) and PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/KR2022/016766**

| Patent document cited in search report | Publication date (day/month/year) | Patent family member(s) | Publication date (day/month/year) |
|---|---|---|---|
| WO 2014-039714 A2 | 13 March 2014 | AU 2013-312477 A1 | 02 April 2015 |
| | | AU 2013-312477 B2 | 31 May 2018 |
| | | CA 2883894 A1 | 13 March 2014 |
| | | CA 2883894 C | 18 August 2020 |
| | | CN 104981247 A | 14 October 2015 |
| | | EP 2892534 A2 | 15 July 2015 |
| | | EP 2892534 B1 | 04 August 2021 |
| | | EP 2892534 B8 | 15 September 2021 |
| | | ES 2889757 T3 | 13 January 2022 |
| | | JP 2015-529224 A | 05 October 2015 |
| | | JP 6318156 B2 | 25 April 2018 |
| | | MX 2015002887 A | 06 July 2015 |
| | | US 10227357 B2 | 12 March 2019 |
| | | US 2014-0128390 A1 | 08 May 2014 |
| | | WO 2014-039714 A3 | 02 October 2014 |
| WO 2019-046778 A1 | 07 March 2019 | AR 112537 A1 | 06 November 2019 |
| | | AU 2018-325445 A1 | 19 March 2020 |
| | | BR 112020004209 A2 | 01 September 2020 |
| | | CA 3074013 A1 | 07 March 2019 |
| | | CL 2020000501 A1 | 10 July 2020 |
| | | CN 111315723 A | 19 June 2020 |
| | | CO 2020003478 A2 | 13 April 2020 |
| | | CR 20200140 A | 15 May 2020 |
| | | DO P2020000050 A | 15 August 2020 |
| | | EC SP20020410 A | 30 June 2020 |
| | | EP 3676254 A1 | 08 July 2020 |
| | | IL 272910 A | 30 April 2020 |
| | | JP 2020-532526 A | 12 November 2020 |
| | | KR 10-2020-0047627 A | 07 May 2020 |
| | | PE 20210128 A1 | 19 January 2021 |
| | | PH 12020500396 A1 | 04 January 2021 |
| | | RU 2020112090 A | 04 October 2021 |
| | | RU 2020112090 A3 | 28 March 2022 |
| | | SG 11202001664 A | 30 March 2020 |
| | | TW 201920104 A | 01 June 2019 |
| | | US 2020-291024 A1 | 17 September 2020 |
| WO 2020-140001 A1 | 02 July 2020 | EP 3902787 A1 | 03 November 2021 |
| | | US 2022-056052 A1 | 24 February 2022 |
| WO 2005-040125 A1 | 06 May 2005 | EP 1673346 A1 | 28 June 2006 |
| | | US 2007-0123537 A1 | 31 May 2007 |
| US 5480883 A | 02 January 1996 | CA 1323147 C | 19 October 1993 |
| | | CA 1326335 C | 25 January 1994 |
| | | CA 1327434 C | 08 March 1994 |
| | | CA 1334046 C | 24 January 1995 |
| | | CA 2056290 A1 | 09 July 1992 |
| | | CA 2056290 C | 24 October 2000 |
| | | CA 2102780 A1 | 11 November 1992 |
| | | CA 2102780 C | 09 January 2007 |
| | | CA 2223016 A1 | 12 December 1996 |
| | | CA 2223016 C | 20 May 2003 |

Form PCT/ISA/210 (patent family annex) (July 2022)

**INTERNATIONAL SEARCH REPORT**
**Information on patent family members**

International application No.
**PCT/KR2022/016766**

| Patent document cited in search report | Publication date (day/month/year) | Patent family member(s) | Publication date (day/month/year) |
|---|---|---|---|
| | | CA 2296940 A1 | 09 July 1992 |
| | | CA 2296940 C | 06 February 2001 |
| | | CN 1187129 A | 08 July 1998 |
| | | CN 1187129 C | 08 July 1998 |
| | | EP 1488792 A2 | 22 December 2004 |
| | | EP 1488792 A3 | 05 January 2005 |
| | | EP 397703 A1 | 22 November 1990 |
| | | EP 397703 B1 | 16 February 1994 |
| | | EP 476931 A2 | 25 March 1992 |
| | | EP 476931 B1 | 17 January 1996 |
| | | EP 494386 A1 | 15 July 1992 |
| | | EP 494386 B1 | 09 August 2000 |
| | | EP 494386 B2 | 11 August 2010 |
| | | EP 508550 A2 | 14 October 1992 |
| | | EP 508550 B1 | 05 July 1995 |
| | | EP 508551 A2 | 14 October 1992 |
| | | EP 508551 B1 | 19 July 1995 |
| | | EP 509613 A2 | 21 October 1992 |
| | | EP 584222 A1 | 02 March 1994 |
| | | EP 584222 B1 | 08 October 1997 |
| | | EP 831831 A1 | 01 April 1998 |
| | | EP 871448 A1 | 21 October 1998 |
| | | EP 871448 B1 | 02 March 2005 |
| | | EP 990390 A1 | 05 April 2000 |
| | | JP 04-123016 A | 23 April 1992 |
| | | JP 04-123017 A | 23 April 1992 |
| | | JP 04-334303 A | 20 November 1992 |
| | | JP 06-507643 A | 01 September 1994 |
| | | JP 11-507355 A | 29 June 1999 |
| | | JP 2924142 B2 | 26 July 1999 |
| | | JP 3507071 B2 | 15 March 2004 |
| | | KR 10-1999-0022533 A | 25 March 1999 |
| | | US 2004-0014774 A1 | 22 January 2004 |
| | | US 5062820 A | 05 November 1991 |
| | | US 5139457 A | 18 August 1992 |
| | | US 5154340 A | 13 October 1992 |
| | | US 5195923 A | 23 March 1993 |
| | | US 5196044 A | 23 March 1993 |
| | | US 5312292 A | 17 May 1994 |
| | | US 5409930 A | 25 April 1995 |
| | | US 5477554 A | 19 December 1995 |
| | | US 5646153 A | 08 July 1997 |
| | | US 5656643 A | 12 August 1997 |
| | | US 5681792 A | 28 October 1997 |
| | | US 5683959 A | 04 November 1997 |
| | | US 5683961 A | 04 November 1997 |
| | | US 5683962 A | 04 November 1997 |
| | | US 5703011 A | 30 December 1997 |
| | | US 5703012 A | 30 December 1997 |
| | | US 5703013 A | 30 December 1997 |

Form PCT/ISA/210 (patent family annex) (July 2022)

**INTERNATIONAL SEARCH REPORT**
**Information on patent family members**

International application No.
**PCT/KR2022/016766**

| Patent document cited in search report | Publication date (day/month/year) | Patent family member(s) | | | Publication date (day/month/year) |
|---|---|---|---|---|---|
| | | US | 5703014 | A | 30 December 1997 |
| | | US | 5710158 | A | 20 January 1998 |
| | | US | 5714493 | A | 03 February 1998 |
| | | US | 5721237 | A | 24 February 1998 |
| | | US | 5795889 | A | 18 August 1998 |
| | | US | 6034034 | A | 07 March 2000 |
| | | US | 6057320 | A | 02 May 2000 |
| | | US | 6645969 | B1 | 11 November 2003 |
| | | WO | 89-05584 | A1 | 29 June 1989 |
| | | WO | 92-20642 | A1 | 26 November 1992 |
| | | WO | 95-15758 | A1 | 15 June 1995 |
| | | WO | 96-39145 | A1 | 12 December 1996 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **PAPADOPOULOS, NATALIA ; JOHAN LENNARTSSON.** The PDGF/PDGFR pathway as a drug target. *Molecular aspects of medicine,* 2018, vol. 62, 75-88